(19) Europäisches Patentamt
European Patent Office
Office européen des brevets

(11) Publication number: 0 254 032
A2

(12) # EUROPEAN PATENT APPLICATION

(21) Application number: 87108730.0

(22) Date of filing: 17.06.87

(51) Int. Cl.³: A 61 K 37/64

(30) Priority: 20.06.86 US 876610
27.03.87 US 32153

(43) Date of publication of application:
27.01.88 Bulletin 88/4

(84) Designated Contracting States:
AT BE CH DE ES FR GB GR IT LI LU NL SE

(71) Applicant: SCHERING CORPORATION
2000 Galloping Hill Road
Kenilworth, New Jersey 07033(US)

(72) Inventor: Haslanger, Martin F.
53 West Gein Avenue
Ridgewood New Jersey 07450(US)

(72) Inventor: Sybertz, Edmund, Jr.
367 Woodland Place
South Orange New Jersey 07079(US)

(72) Inventor: Neustadt, Bernard R.
24 Brook Place
West Orange New Jersey 07052(US)

(72) Inventor: Smith, Elizabeth M.
166 Grove Avenue
Verona New Jersey 07044(US)

(74) Representative: von Kreisler, Alek, Dipl.-Chem. et al,
Deichmannhaus am Hauptbahnhof
D-5000 Köln 1(DE)

(54) **Neutral metalloendopeptidase inhibitors in the treatment of hypertension.**

(57) The method of treating hypertension with neutral metalloendopeptidase (NMEP) inhibitors, NMEP inhibitors in combination with atrial peptides, and NMEP inhibitors in combination with angiotensin converting enzyme inhibitors, as well as pharmaceutical compositions therefor, are disclosed.

EP 0 254 032 A2

Croydon Printing Company Ltd.

## NEUTRAL METALLOENDOPEPTIDASE INHIBITORS IN THE TREATMENT OF HYPERTENSION

Human hypertension represents a disease of multiple etiologies. Included among these is a sodium and volume dependent low renin form of hypertension. Drugs that act to control one aspect of hypertension will not necessarily be effective in controlling another.

We have surprisingly found that neutral metalloendopeptidase (NMEP) inhibitors lower blood pressure under conditions where angiotensin converting enzyme (ACE) inhibitors are relatively ineffective, for example in salt-dependent hypertension. NMEP inhibitors potentiate the activity of atrial natriuretic factors (ANF).

Enkephalin, a natural opiate receptor agonist, is known to produce analgesia, and it is also known that enkephalin is inactivated by a neutral metallopeptidase (EC 3.4.24.11) frequently referred to as enkephalinase or enkephalinase A. Various NMEP inhibitors have been discovered which, by their inhibition of this enzyme elicit an analgesic effect. To date, no significant cardiovascular activity has been attributed to NMEP inhibitors. The NMEP inhibitor thiorphan was found to inhibit peripheral ACE, but to have little direct cardiovascular activity in its own right: blood pressure and heart rate were not affected. See T. Baum, et al,

"Enkephalinase A Inhibition by Thiorphan: Central and Peripheral Cardiovascular Effects", Eur. J. Pharm., 94(1983), pg 85-91.

Atrial peptides or atrial natriuretic factors, recently discovered peptide hormones secreted by the heart, help to regulate blood pressure, blood volume and the excretion of water, sodium and potassium. ANF were found to produce a short-term reduction in blood pressure and to be useful in the treatment of congestive heart failure. See P. Needleman et al, "Atriopeptin: A Cardiac Hormone Intimately Involved in Fluid, Electrolyte and Blood-Pressure Homeostasis", N. Engl. J. Med., 314, 13 (1986) pp. 828-834, and M. Cantin et al in "The Heart as an Endocrine Gland", Scientific American, 254 (1986) pg. 76-81.

We have also found that NMEP inhibitors may be combined with other drugs used in the treatment of hypertension to enhance the effects of those drugs: combinations of NMEP inhibitors and exogenous atrial peptides provide improved magnitude and duration of the antihypertensive effect when compared to the use of atrial peptides alone, and combinations of NMEP inhibitors and ACE inhibitors provide an antihypertensive effect greater than the activity of either the NMEP inhibitor or the ACE inhibitor alone.

ACE inhibitors, drugs which are known to be effective in treating some types of hypertension, are useful in blocking the rise in blood pressure caused by increases in vascular resistance and fluid volume due to the formation of angiotensin II from angiotensin I. For a review of ACE inhibitors, see M. Wyvratt and A. Patchett, "Recent Developments in the Design of Angiotensin Converting Enzyme Inhibitors" in Med. Res. Rev. Vol. 5, No. 4 (1985) pp. 483-531, incorporated herein by reference.

The invention sought to be patented in its pharmaceutical composition aspect is an NMEP inhibitor in combination with a pharmaceutically acceptable carrier for use in treating hypertension. Also sought to be patented are a pharmaceutical composition comprising both an NMEP inhibitor and an ACE inhibitor in combination with a pharmaceutically acceptable carrier, and a pharmaceutical composition comprising both an NMEP inhibitor and an atrial peptide in combination with a pharmaceutically acceptable carrier.

In another aspect of the invention, pharmaceutical compositions containing components of the combination therapies may be administered separately. Where the compositions are administered separately, any convenient combination of dosage forms may be used, e.g. oral NMEP inhibitor/oral ACE inhibitor, oral NMEP inhibitor/parenteral atrial peptide, parenteral NMEP inhibitor/oral ACE inhibitor, parenteral NMEP inhibitor/parenteral atrial peptide.

Still another aspect of the invention sought to be patented is a combination of separate pharmaceutical compositions in kit form. That is, two kits are contemplated, each combining two separate units: an NMEP inhibitor pharmaceutical composition and an ACE inhibitor pharmaceutical composition in one kit, and an NMEP inhibitor pharmaceutical composition and an atrial peptide pharmaceutical composition in a second kit. The kit form is particularly advantageous when the separate components must be administered in different dosage forms (e.g. oral and parenteral) or are administered at different dosage intervals.

When the components of a combination of an NMEP inhibitor and an atrial peptide are administered separately, it is preferred that the NMEP inhibitor be administered first.

The NMEP inhibitors contemplated for use in this invention are exemplified by, but not limited to:

thiorphan (N-[(R,S)-3-mercapto-2-benzylpropanoyl]-glycine) and related compounds, see B.P. Rogues et al, Eur. J. Biochem., 139 (1984) 267-274;

carboxyalkyl inhibitors such as (S)-HomoPhe-[N]-L-Phe-Gly (See R.A. Mumford, et al, Biochem. Biophys. Res. Commun., 109 (1982) 1303-1307) and N-[(R,S)-2-carboxy-3-phenypropanoyl]-L-leucine (See B.P. Rogues et al, J. Med. Chem., 26 (1983) 60-65);

hydroxamic acid inhibitors such as N-[3(R,S)-(N-formyl-N-hydroxyamino)-1-oxo-2-benzylpropyl]glycine, N-[3(R,S)-[(hydroxyamino)carbonyl]-2-benzyl-1-oxopropyl]-glycine and N-[3(R,S)-[(hydroxyamino)carbonyl]-2-benzyl-1-oxopropyl]-L-alaine (See B.P. Rogues, J. Med. Chem., 28 (1985) 1158-1169);

compounds disclosed in U.S. Patent 4,610,816 having the formula

$$R_1{}^uC^*(HCOR_2{}^u)\text{-}NH\text{-}C^*(HR_3{}^u)\text{-}CONH(CH_2)_p{}^u\text{-}C^*(R_4{}^uR_5{}^u)\text{-}COR_6{}^u$$

and the racemates, enantiomers and diastereoisomers thereof and the pharmaceutically acceptable salts thereof wherein:

$R_1{}^u$ is alkyl having from 1 to 6 carbon atoms, adamantylmethyl, cycloakylmethyl having from 4 to 8 carbon atoms or $A^u\text{-}X^u{}_m{}^u\text{-}C_n{}^uH_{2n}{}^u\text{-}$ wherein $X^u$ is oxygen or sulfur, $A^u$ is phenyl which may be substituted with the group, $Y^u$, wherein $Y^u$ is halogen, hydroxy, trifluoromethyl, alkoxy having from 1 to 6 carbon atoms, alkyl having from 1 to 6 carbon atoms, 2- and 3-furanyl, 2- and 3-thienyl, or phenyl [which may be substituted with halogen, hydroxy, trifluoromethyl, alkoxy having from 1 to 6 carbon atoms or alkyl having from 1 to 6 carbon atoms] benzyl [the phenyl ring of which may be

substituted with the group, $Y^u$, as defined herein], 1- and 2-naphthyl, 2- and 3-furanyl or 2- and 3-thienyl; $m^u$ is 0 or 1 and $n^u$ is 0, 1, 2, 3, or 4;

$R_2{}^u$ and $R_6{}^u$ may be the same or different and are hydroxy, alkoxy having from 2 to 8 carbon atoms, $B^u$-$X^u{}_m{}^u$-$C_n{}^uH_{2n}{}^u$-O- wherein $B^u$ is phenyl [which may be substituted with the group, $Y^u$, as defined herein] or 1-and 2-naphthyl, $X^u$, $m^u$, and $n^u$ are as defined herein provided that when $n^u$=0, $m^u$=0, $-OCH_2OCO$-alkyl having from 3 to 8 carbon atoms, $-OCH_2CO$-phenyl [the phenyl ring of which may be substituted with the group, $Y^u$, as defined herein], 1-glyceryl,

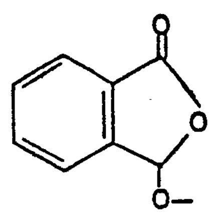 or 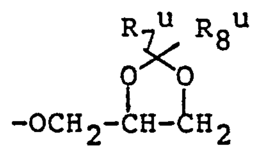

wherein $R_7{}^u$ is hydrogen, alkyl having from 1 to 6 carbon atoms, or phenyl which may be substituted with the group, $Y^u$, as defined herein, and $R_8{}^u$ is hydrogen or alkyl having from 1 to 6 carbon atoms;

$R_2{}^u$ may also be $-NR_7{}^uR_8{}^u$ wherein $R_7{}^u$ and $R_8{}^u$ are as defined herein;

$R_3{}^u$ is alkyl having from 1 to 6 carbon atoms, cycloalkylmethyl having from 4 to 8 carbon atoms, 2- and 3-thienylmethyl, 2- and 3-furanylmethyl, 1- and 2-naphthylmethyl, or benzyl, the phenyl ring of which may be substituted with the group, $Y^u$, as defined herein;

$R_4{}^u$ is $D^u$-$C_n{}^uH_{2n}{}^u$-$O_m{}^u$- wherein $D^u$ is hydrogen, alkyl having from 1 to 4 carbon atoms or phenyl which may be substituted with the group, $Z^u$, wherein $Z^u$ is halogen, hydroxy, trifluoromethyl, alkoxy having from 1 to 6 carbon atoms, or alkyl having from 1 to 6 carbon atoms; $m^u$ and $n^u$ are as defined herein;

$R_4{}^u$ may also be $-NR_5{}^uCOR_7{}^u$ (wherein $R_5{}^u$ and $R_7{}^u$ are defined herein), $NR_5{}^uCO_2R_9{}^u$ (wherein $R_5{}^u$ is defined herein and $R_9{}^u$ is alkyl having from 1 to 6 carbon atoms or phenyl which may be substituted with the group, $Y^u$, defined herein), provided that $p^u$ is 1 or 2;

$R_5{}^u$ is hydrogen or alkyl having from 1 to 6 carbon atoms; and

$p^u$ is 0, 1 or 2.

Preferred compounds having the above structural formula are:

N-[N-[(L-1-carboxy-2-phenylethyl)]-L-phenylalanyl]-β-alanine;

N-[N-[L-1-(2,2-dimethyl-1-oxopropoxy)methoxy]-carbonyl]-2-phenylethyl]-L-phenylalanyl]-β-alanine;

N-[N-[1-(S)-carboxyl-3-phenylpropyl]-(S)-phenylalanyl]-(R)-isoserine; and

N-[N-[1-(S)-[2-(phenoxy)ethoxycarbonyl]-3-phenylpropyl]-(S)-phenylalanyl]-(S)-isoserine.

Compounds disclosed in European Patent Application 117,429 having the formula:

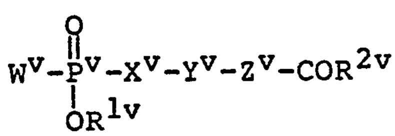

wherein $R^{1v}$ is hydrogen, alkyl having from 1 to 6 carbon atoms,

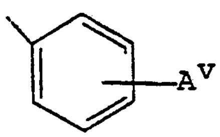 or 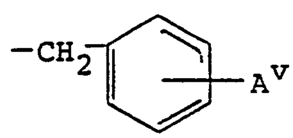

in which $A^v$ is hydrogen, halogen, hydroxy, nitro, trifluoromethyl, alkoxy having from 1 to 6 carbon atoms, alkyl having from 1 to 6 carbon atoms, 2- or 3-thienyl or phenyl which may be substituted with halogen, hydroxy, nitro, trifluoromethyl, alkoxy having from 1 to 6 carbon atoms, or alkyl having from 1 to 6 carbon atoms;

$W^v$ is $R^{1v}$ or $OR^{1v}$ wherein $R^{1v}$ is as defined above;

$X^v$ is $-(CH_2)_{p^v}-CHR^{3v}-$ or $-CHR^{3v}-(CH_2)_{p^v}-$ wherein

$p^v$ is 0 or 1, and

$R^{3v}$ is 2- or 3-thienylmethyl, 3-indolylmethyl, alkyl having from 1 to 6 carbon atoms, cycloalkyl having from 3 to 7 carbon atoms, cycloalkylmethyl having from 4 to 8 carbon atoms,

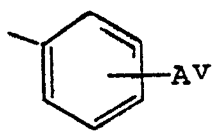 or 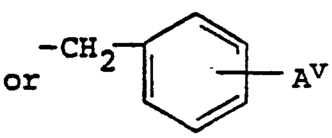 in which $A^v$ is as defined above,

$Y^v$ is $-NH\overset{O}{\overset{\|}{C}}-$, $-\overset{O}{\overset{\|}{C}}NH-$, $-NH\overset{O}{\overset{\|}{C}}NH-$, or $-NH\overset{O}{\overset{\|}{C}}-O-$ .

$Z^v$ is <u>o</u>-, <u>m</u>- or p-phenylene, $-(CHR^{4v})_{r^v}-$ [wherein $r^v$ is 1,2,3, or 4 and $R^{4v}$ is independently chosen from hydrogen, alkyl having from 1 to 6 carbon atoms, hydroxy, alkoxy having from 1 to 6 carbon atoms, cycloalkyl having from 3 to 7 carbon atoms, cycloalkylmethyl having from 4 to 8 carbon atoms, 3-indolylmethyl, hydroxymethyl, 2-(methylthio)ethyl,

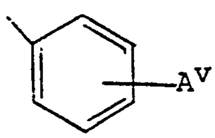 or 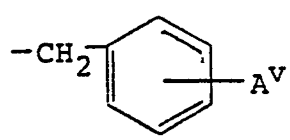 in which $A^v$ is as defined above] or

$-B^v q^v-B'^v-B''^v-$[wherein $q^v$ is 0 or 1, one of the groups, $B^v$, $B'^v$ or $B''^v$ is

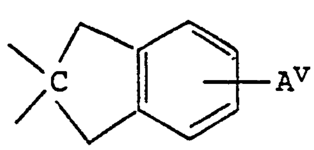 ; 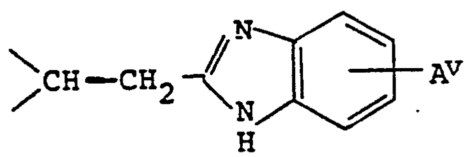 ; or 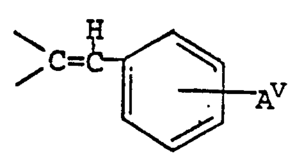

in which $A^v$ is as defined above and the remaining $B^v$, $B'^v$ and/or $B''^v$ is $-CH_2-$],

$R_2^v$ is hydroxy, alkoxy having from 1 to 6 carbon atoms,

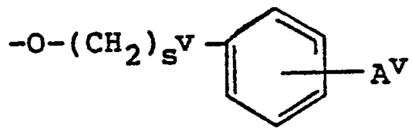

[wherein $s^v$ is 0, 1, 2, or 3], $-O-(CR^{5v}R^{6v})O\overset{O}{\overset{\|}{C}}R_7^v$ [wherein $R^{5v}$ and $R^{6v}$ may be the same or different and are hydrogen or alkyl having from 1 to 6 carbon atoms, and $R_7^v$ is

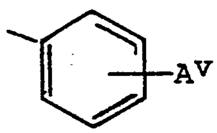 or 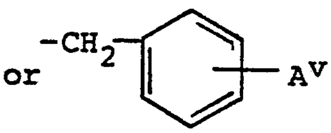

or alkyl having from 1 to 6 carbon atoms],

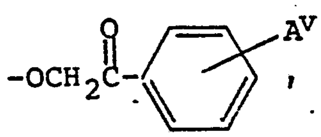 , 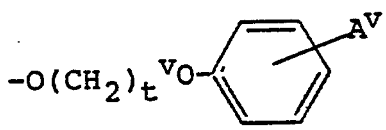 ,

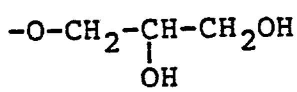 , 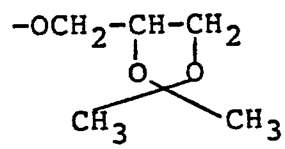

[wherein $t^v$ is 1 or 2], 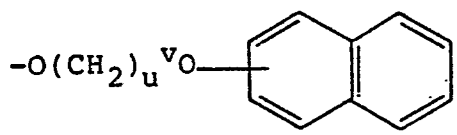 ,

[wherein $u^v$ is 1 or 2], 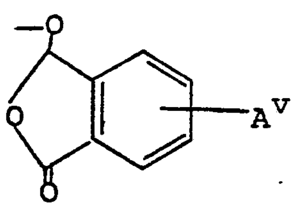 or $NR^{8v}R^{9v}$ wherein

$R^{8v}$ is 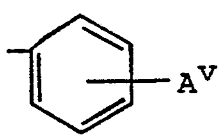 , $R^{9v}$ is hydrogen or alkyl having from 1 to 6 carbon atoms, and wherein $A^v$ in the groups defined for $R_2^v$ is as defined above;

Preferred compounds having the above structural formula are

N-[1-oxo-3-phenyl-2-(phosphonomethyl)propyl]-β-alanine and the ethyl ester thereof;

compounds disclosed in U.S. Serial No. 32,153, filed March 27, 1987, US-Patent 4 513 009, and EPA 38 046, having the formula

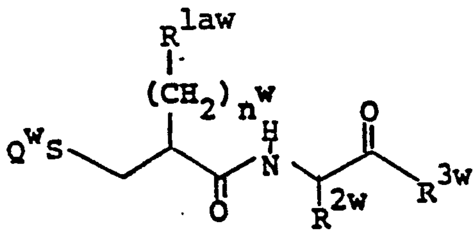

$R^{1aw}$ is $Y^w$-$C_6H_4$-, $Y^w$-$C_6H_4S$-, $Y^w$-$C_6H_4O$-, 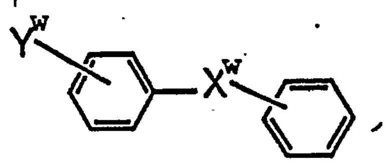,

α-naphthyl, β-naphthyl, furyl, thienyl, benzofuryl, benzothienyl, $H_2N(CH_2)_{m^w}$-, diphenylmethyl,

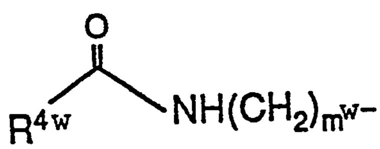 or 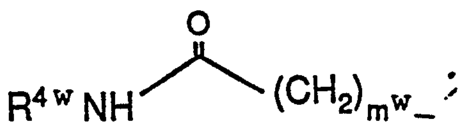;

$R^{2w}$ is alkyl, alkyl-$S(O)_{0-2}(CH_2)_{q^w}$-, $R^{5w}(CH_2)_{k^w}S(O)_{0-2}(CH_2)_{q^w}$-, alkyl-$O(CH_2)_{q^w}$-, $R^{5w}(CH_2)_{k^w}$-$O(CH_2)_{q^w}$-, $R^{5w}(CH_2)_{q^w}$-, $H_2N(CH_2)_{q^w}$-, cycloalkyl$(CH_2)_{k^w}$-, $R^{13w}CONH(CH_2)_{q^w}$-, $R^{13w}NHCO(CH_2)_{q^w}$- or $R^{6w}OCO(CH_2)_{q^w}$-;

$R^{3w}$ is $-OR^{7w}$, 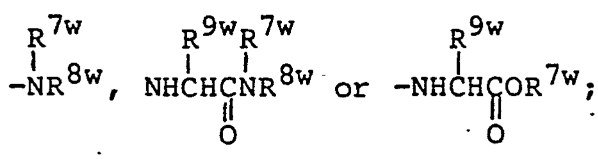;

$R^{4w}$ and $R^{13w}$ are independently hydrogen, alkyl or $Y^{1w}-C_6H_4-$;

$R^{5w}$ is $Y^{2w}-C_6H_4-$, $Y^{2w}-C_6H_4S-$, $Y^{2w}-C_6H_4O-$, α-naphthyl, β-naphthyl, furyl, thienyl, benzofuryl, benzothienyl, indolyl or 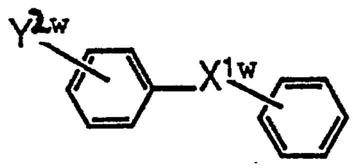

provided that when $R^{5w}$ is $Y^{2w}-C_6H_4S-$ or $Y^{2w}-C_6H_4O-$, $k^w$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

$n^w$ is 0-2;

$m^w$ and $k^w$ are independently 0-3;

$q^w$ is 1-4;

$X^w$ and $X^{1w}$ are independently a bond, -O-, -S-, or $-CH_2-$;

$Q^w$ is hydrogen or $R^{10w}CO-$;

$R^{10w}$ is alkyl, hydroxyalkyl, alkoxyalkyl, dialkylaminoalkyl, $Y^{3w}-C_6H_4$-alkyl, alkoxy, $Y^{3w}-C_6H_4-$, naphthyl, furyl, thienyl or pyridyl;

$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;

and the pharmaceutically acceptable acid addition salts thereof.

A preferred compound is N-[2-(S-acetylthiomethyl)-3-phenylpropionyl]-(S)-methionine amide.

The above general formula encompasses novel compounds which may be made by the processes disclosed in US 4 513 009 and EPA 38 046. Said novel compounds are of three structural formulae, as follows:

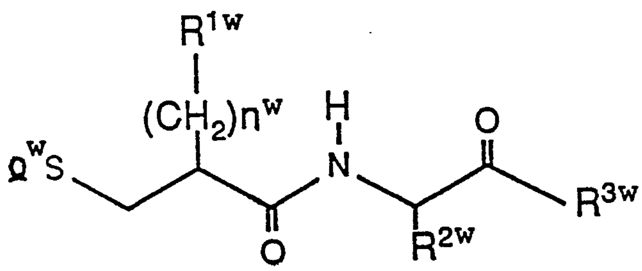

I

wherein $R^{1w}$ is phenyl substituted by one or more substituents independently selected from alkyl, alkoxy, cycloalkyl, cyano and aminomethyl, $Y^w-C_6H_4S-$, $Y^w-C_6H_4O-$,

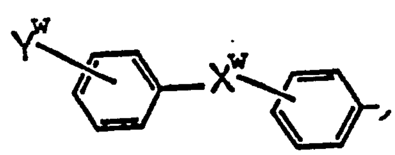

α-naphthyl, β-naphthyl, furyl, benzofuryl, benzothienyl, $H_2N(CH_2)_m{}^w-$, diphenylmethyl,

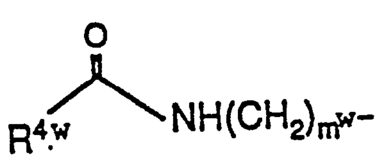

or

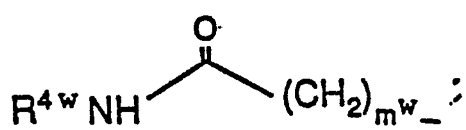

$R^{2w}$ is alkyl, alkyl-$S(O)_{0-2}(CH_2)_q{}^w-$, $R^{5w}(CH_2)_k{}^wS(O)_{0-2}(CH_2)_q{}^w-$, alkyl-$O(CH_2)_q{}^w-$, $R^{5w}(CH_2)_k{}^w-O(CH_2)_q{}^w-$, $R^{5w}(CH_2)_q{}^w-$, $H_2N(CH_2)_q{}^w-$, cycloalkyl$(CH_2)_k{}^w-$, $R^{13w}CONH(CH_2)_q{}^w-$, $R^{13w}NHCO(CH_2)_q{}^w-$ or $R^{6w}OCO(CH_2)_q{}^w-$;

$R^{3w}$ is $-OR^{7w}$, $-NR^{7w}R^{8w}$, 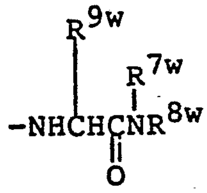 or 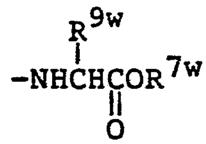;

$R^{4w}$ and $R^{13w}$ are independently hydrogen, alkyl or $Y^{1w}-C_6H_4-$;

$R^{5w}$ is $Y^{2w}-C_6H_4-$, $Y^{2w}-C_6H_4S-$, $Y^{2w}-C_6H_4O-$, α-naphthyl, β-naphthyl, furyl, thienyl, benzofuryl, benzothienyl, indolyl or

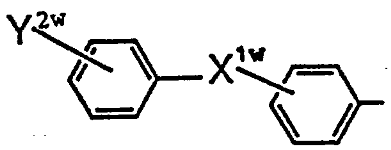 ,

provided that when $R^{5w}$ is $Y^{2w}-C_6H_4S-$ or $Y^{2w}-C_6H_4O-$, $k^w$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl;

$n^w$ is 0-2;

$m^w$ and $k^w$ are independently 0-3;

$q^w$ is 1-4;

$X^w$ and $X^{1w}$ are independently a bond, -O-, -S-, or $-CH_2-$;

$Q^w$ is hydrogen or $R^{10w}CO-$;

$R^{10w}$ is alkyl, hydroxyalkyl, alkoxyalkyl, dialkylaminoalkyl, $Y^{3w}$-$C_6H_4$-alkyl, alkoxy, $Y^{3w}$-$C_6H_4$-, naphthyl, furyl, thienyl or pyridyl;
$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;
and the pharmaceutically acceptable addition salts thereof;

--

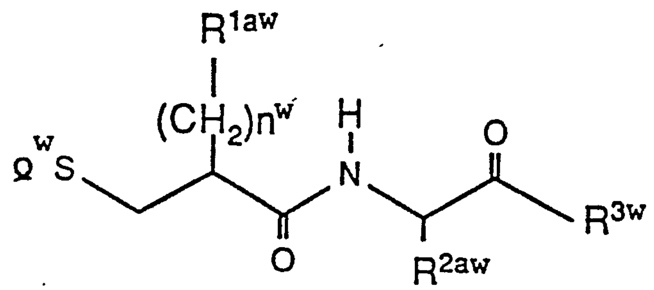

II

wherein $R^{1aw}$ is $Y^w$-$C_6H_4$-, $Y^w$-$C_6H_4S$-, $Y^w$-$C_6H_4O$-,

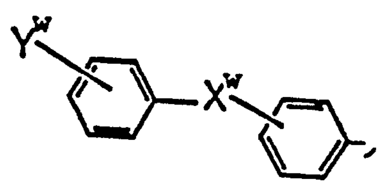

α-naphthyl, β-naphthyl, furyl, thienyl, benzofuryl, benzothienyl, $H_2N(CH_2)_m{}^w$-, diphenylmethyl,

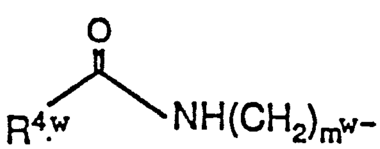 or 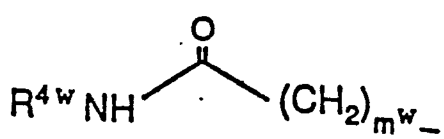;

$R^{2aw}$ is $R^{5aw}(CH_2)_k^wS(O)_{0-2}(CH_2)_q{}^w$-, $R^{5aw}(CH_2)_k{}^w$-$O(CH_2)_q{}^w$-, $R^{5aw}(CH_2)_q{}^w$-, or cycloalkyl-$(CH_2)_k{}^w$, and when $R^{3w}$ is

$-NR^{7w}R^{8w}$,

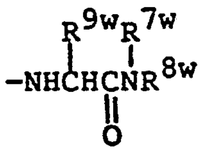 or 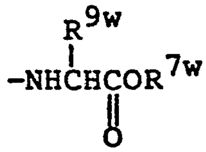, $R^{2aw}$ may also be indolyl-$(CH_2)_q{}^{w}$-,

$R^{13w}CONH(CH_2)_q{}^{w}$-, $R^{13w}NHCO(CH_2)_q{}^{w}$- or $R^{6w}OCO(CH_2)_q{}^{w}$-;

$R^{3w}$ is $-OR^{7w}$, $-NR^{7w}R^{8w}$, 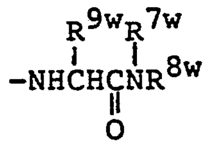 or 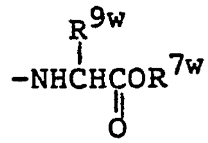;

$R^{4w}$ and $R^{13w}$ are independently hydrogen, alkyl or $Y^{1w}$-$C_6H_4$-;

$R^{5aw}$ is $Y^{2w}$-$C_6H_4$- provided $Y^{2w}$ is not H or OH, $Y^{2w}$-$C_6H_4S$-, $Y^{2w}$-$C_6H_4O$-, α-naphthyl, β-naphthyl, furyl, thienyl, benzofuryl, benzothienyl or

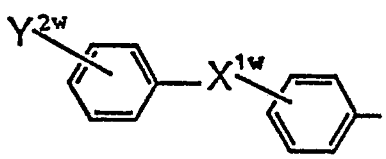 ,

provided that when $R^{5aw}$ is $Y^{2w}$-$C_6H_4S$- or $Y^{2w}C_6H_4O$-, $k^{w}$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl,

alkylthioalkyl, aminoalkyl, hydroxyalkyl, phenylalkyl, hydroxyphenylalkyl, guanidinoalkyl, imidazolylalkyl, indolylalkyl, or carbamoylalkyl;
$n^w$ is 0-2;
$m^w$ and $k^w$ are independently 0-3;
$q^w$ is 1-4;
$X^w$ and $X^{1w}$ are independently a bond, -O-, -S-, or $-CH_2-$;
$Q^w$ is hydrogen or $R^{10w}CO-$;
$R^{10w}$ is alkyl, hydroxyalkyl, alkoxyalkyl, dialkylaminoalkyl, $Y^{3w}-C_6H_4$-alkyl, alkoxy, $Y^{3w}-C_6H_4-$, naphthyl, furyl, thienyl or pyridyl;
$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;
and the pharmaceutically acceptable addition salts thereof; and

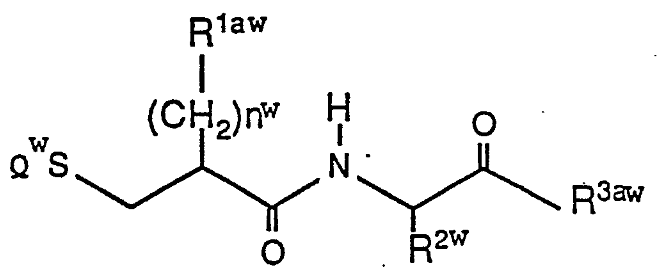

III

wherein $R^{1aw}$ is $Y^w-C_6H_4-$, $Y^w-C_6H_4S-$, $Y^w-C_6H_4O-$,

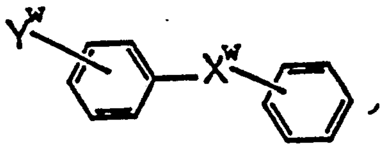,

α-naphthyl, β-naphthyl, furyl, thienyl, benzofuryl, benzothienyl, $H_2N(CH_2)_{m^w}-$, diphenylmethyl,

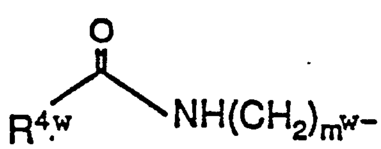 or 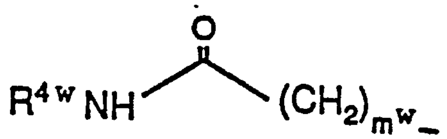;

$R^{2w}$ is alkyl, alkyl-$S(O)_{0-2}(CH_2)_q{}^{w}$-, $R^{5w}(CH_2)_k{}^{w}S(O)_{0-2}(CH_2)_q{}^{w}$-, alkyl-$O(CH_2)_q{}^{w}$-, $R^{5w}(CH_2)_k{}^{w}$-$O(CH_2)_q{}^{w}$-, $R^{5w}(CH_2)_q{}^{w}$-, $H_2N(CH_2)_q{}^{w}$-, cycloalkyl$(CH_2)_k{}^{w}$-, $R^{13w}CONH(CH_2)_q{}^{w}$-, $R^{13w}NHCO(CH_2)_q{}^{w}$- or $R^{6w}OCO(CH_2)_q{}^{w}$-;

$R^{3aw}$ is 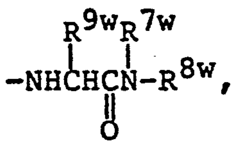, 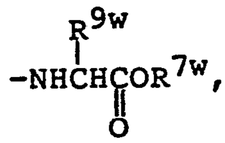, $-OR^{11w}$ or 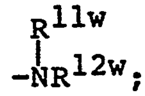;

$R^{4w}$ and $R^{13w}$ are independently hydrogen, alkyl or $Y^{1w}$-$C_6H_4$-;

$R^{5w}$ is $Y^{2w}$-$C_6H_4$-, $Y^{2w}$-$C_6H_4S$-, $Y^{2w}$-$C_6H_4O$-, α-naphthyl, β-naphthyl, furyl, thienyl, benzofuryl, benzothienyl, indolyl or

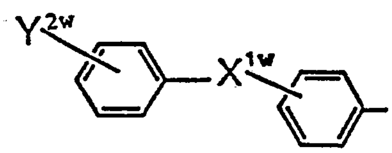,

provided that when $R^{5w}$ is $Y^{2w}$-$C_6H_4S$- or $Y^{2w}$-$C_6H_4O$-, $k^w$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl;

$n^w$ is 0-2;

$m^w$ and $k^w$ are independently 0-3;
$q^w$ is 1-4;
$X^w$ and $X^{1w}$ are independently a bond, -O-, -S-, or $-CH_2-$;
$Q^w$ is hydrogen or $R^{10w}CO-$;
$R^{10w}$ is alkyl, hydroxyalkyl, alkoxyalkyl, dialkylaminoalkyl, $Y^{3w}-C_6H_4$-alkyl, alkoxy, $Y^{3w}-C_6H_4-$, naphthyl, furyl, thienyl or pyridyl;
$R^{11w}$ is hydroxyalkyl or substituted phenylalkyl wherein the phenyl group is substituted by one or more groups selected from alkyl, alkoxy, cycloalkyl and cyano; $R^{12w}$ is H or selected from the same group as $R^{11w}$; or $R^{11w}$ and $R^{12w}$ together with the nitrogen to which they are attached complete a 5-7 membered ring wherein one of the 4-6 ring members comprising $R^{11w}$ and $R^{12w}$ may be a nitrogen atom, an alkyl-substituted nitrogen atom or an oxygen atom, and wherein the ring may be substituted on the ring carbon atoms with substituents chosen from alkyl and hydroxy groups;
$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;
and the pharmaceutically acceptable addition salts thereof.

As used herein the term "alkyl" means straight or branched alkyl chains of 1 to 6 carbon atoms, and "alkoxy" similarly refers to alkoxy groups having 1 to 6 carbon atoms. "Cycloalkyl" means cyclic alkyl groups of 3-6 carbon atoms.

"Aryl" means mono-cyclic or fused ring bicyclic aromatic groups having 5 to 10 ring members wherein 0-2 ring members may independently be nitrogen, oxygen or sulfer and wherein the ring members may be substituted by one to three substituents chosen from group Y defined above. Examples of aryl groups are phenyl, α-naphthyl, β-naphthyl, furyl, thienyl, benzofuryl, benzothienyl, indolyl and pyridyl.

"Halo" refers to fluorine, chlorine, bromine or iodine radicals. The term "poly", when used to describe substitution in a phenyl, alkylphenyl or alkoxyphenyl group, means 2 to 5 substituents.

Groups $R^{3w}$ and $R^{3aw}$ comprising the partial structure $-NH\overset{R^{9w}}{\overset{|}{C}}H-\overset{O}{\overset{\|}{C}}-$

are derived from amino acids of formula $H_2N\overset{R^{9w}}{\overset{/}{C}}HCOOH$. Examples of such amino acids are alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine.

Preferred embodiments of compounds of formula I are compounds wherein $R^{2w}$ is alkyl, alkyl-$S(O)_{0-2}(CH_2)_q{}^w$-, $R^{5w}(CH_2)_k{}^wS(O)_{0-2}(CH_2)_q{}^w$- or $R^{5w}(CH_2)_q{}^w$-, wherein $R^{5w}$, $q^w$ and $k^w$ are as defined above. Also preferred are compounds of formula I wherein $R^{1w}$ is naphthyl, furyl, benzofuryl, benzothienyl, diphenylmethyl, aminoalkyl, $Y^w$-$C_6H_4$-$X^w$-$C_6H_4$-, $R^{4w}CONH(CH_2)_m{}^w$- or $R^{4w}NHCO(CH_2)_m{}^w$-, wherein $Y^w$, $X^w$, $R^{4w}$ and $m^w$ are as defined above. A third group of preferred compounds in that wherein $R^{1w}$ is substituted phenyl. Still another group of preferred compounds of formula I is that wherein $R^{3w}$ is $-OR^{7w}$ or $-NR^{7w}R^{8w}$, wherein $R^{7w}$ and $R^{8w}$ are as defined above.

Preferred compounds of formula II are those wherein $R^{2aw}$ is $R^{5w}(CH_2)_k{}^wS(O)_{0-2}(CH_2)_q{}^w$- wherein $R^{5w}$, $q^w$ and $k^w$ are as defined above. Also preferred are compounds of formula II wherein $R^{1aw}$ is naphthyl, furyl, thienyl, benzofuryl, benzothienyl, diphenylmethyl, aminoalkyl, $Y^w$-$C_6H_4$-$X^w$-$C_6H_4$-, $R^{4w}CONH(CH_2)_m{}^w$- or $R^{4w}NHCO(CH_2)_m{}^w$- wherein $Y^w$, $X^w$, $R^{4w}$ and $m^w$ are as defined above. A third group of preferred compounds is that wherein $R^{1aw}$ is $Y^w$-$C_6H_4$. Still another group of preferred compounds of formula II is that wherein $R^{3w}$ is $-OR^{7w}$ or $-NR^{7w}R^{8w}$, wherein $R^{7w}$ and $R^{8w}$ are as defined above.

Preferred compounds of formula III are those wherein $R^{3aw}$ is $-NHCH_2CONH_2$, arylalkoxy or arylalkylamino. Also preferred are compounds of formula III wherein $R^{1aw}$ is naphthyl, furyl, thienyl, benzofuryl, benzothienyl, diphenylmethyl, aminoalkyl, $Y^w$-$C_6H_4$-$X^w$-$C_6H_4$-, $R^{4w}CONH(CH_2)_m{}^w$- or $R^{4w}NHCO(CH_2)_m{}^w$- wherein $Y^w$, $X^w$, $R^{4w}$ and $m^w$ are as defined above. A third group of preferred compounds is that wherein $R^{1aw}$ is substituted phenyl.

Preferred compounds of formulae I-III are those wherein $Q^w$ is hydrogen or $R^{10w}CO$- wherein $R^{10w}$ is alkyl.

Preferred compounds of formulae I-III are those wherein $Q^W$ is hydrogen or $R^{10W}CO-$ wherein $R^{10W}$ is alkyl.

Compounds of this invention may, depending on the nature of functional groups, form addition salts with various inorganic and organic acids and bases. Such salts include salts prepared with organic and inorganic acids, e.g. HCl, HBr, $H_2SO_4$, $H_3PO_4$, methanesulfonic acid, toluenesulfonic acid, maleic acid, furmaric acid and camphorsulfonic acid. Salts prepared with bases include ammonium salts, alkali metal salts, e.g. sodium and potassium salts, and alkaline earth salts, e.g. calcium and magnesium salts.

The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Compounds of formulae I-III have two or more asymmetrical carbon atoms and therefore include various stereoisomers. All stereoisomers are included within the scope of the present invention.

Compounds of the present invention may be prepared by using coupling reactions well known in the peptide art to join a 3-acetylthio-2-(substituted)-propionic acid of formula 1 with an amino acid ester of formula 2. The following reaction Scheme 1 is an example:

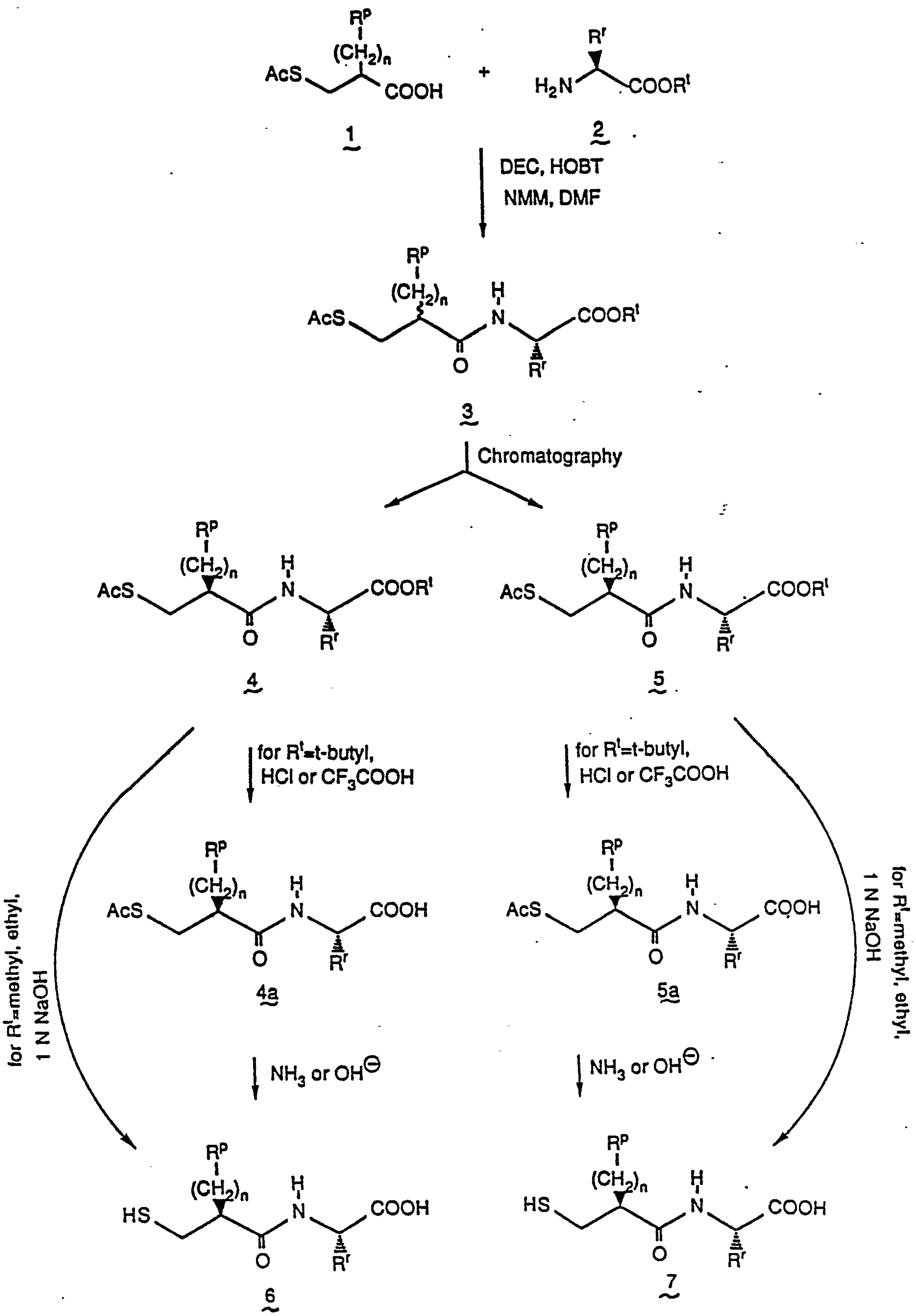


1 + 2 → 3 → 4, 5 → 4a, 5a → 6, 7

In the above scheme, $R^p=R^{1w}$ and $R^{1aw}$; $R^r=R^{2w}$ and $R^{2aw}$; $R^t$ is methyl, ethyl, t-butyl or aralkyl (e.g. benzyl); Ac is acetyl; n is 0-2; DEC is 1-(3-dimethylaminopropyl)-3-ethylcarbodiimide hydrochloride; HOBT is 1-hydroxybenzotriazole hydrate; NMM is N-methylmorpholine; and DMF is dimethylformamide.

As Scheme 1 shows, an amino acid ester of formula 2 and a 3-acetylthio propionic acid of formula 1 are reacted at room temperature in an inert solvent such as DMF in the presence of coupling agents such as DEC and HOBT in the presence of a base such as NMM. The resultant isomers are separated by chromatography and the isomers are deprotected at the acid and mercapto positions.

Alternatively, a propionic acid of formula 1 may be reacted with thionyl chloride to prepare the corresponding propionyl chloride, which may then be reacted with an amino acid ester of formula 2 or with the corresponding free acid 2a in an inert solvent such as acetonitrile in the presence of a base such as triethylamine to give isomers of formula 3, which may be separated as in Scheme 1. The following Scheme 2 is an example:

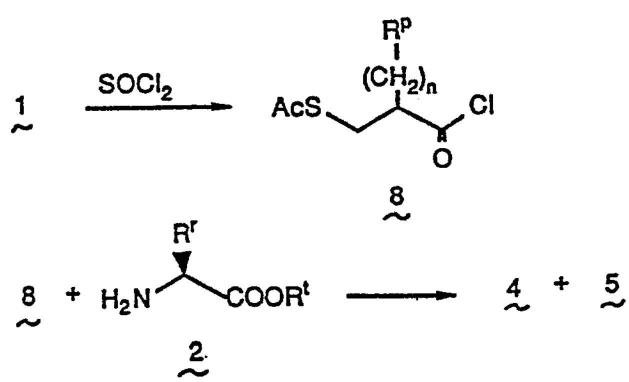


wherein n, Ac, $R^p$, $R^r$ and $R^t$ are as defined above, and wherein $R^t$ may also be hydrogen.

Compounds of formulae I-III wherein $R^{3w}$ or $R^{3aw}$ is $-NR^{7w}R^{8w}$ are prepared by coupling reactions as described above in Schemes 1 and 2 by replacing the amino acid ester 2 with an amide or substituted amide as shown in Scheme 3:

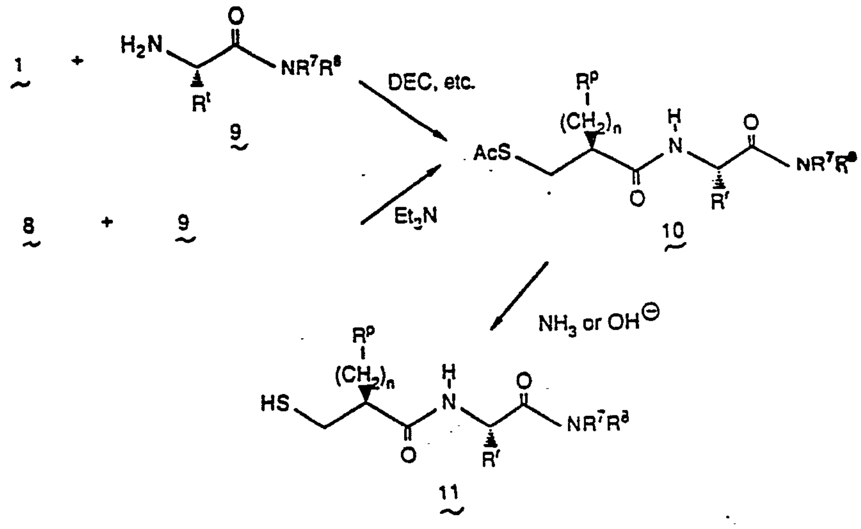


Alternatively, compounds of formulae I-III wherein $R^{3w}$ or $R^{3aw}$ is $-NR^{7w}R^{8w}$ may be prepared by coupling a propionyl chloride of formula 8 with an amino acid of formula 2a in the presence of a base and then coupling the desired $-NR^{7w}R^{8w}$ group to the carboxylic group using a typical peptide-coupling reaction. Scheme 4 shows an example of such a procedure:

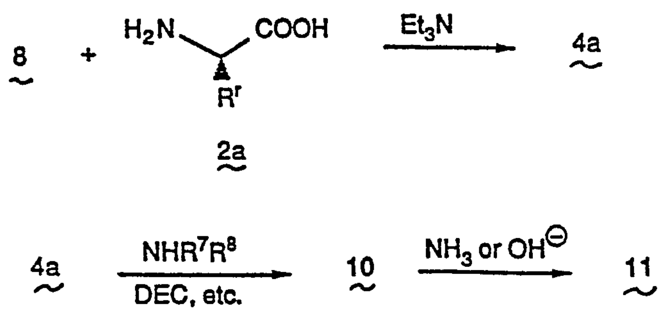


A third method for preparing compounds of formulae I-III wherein $R^{3w}$ or $R^{3aw}$ is $-NR^{7w}R^{8w}$ comprises reacting a propionic acid of formula 1 with an amino acid t-butyl ester of formula 2, removing the t-butyl ester

and coupling the $-NR^{7w}R^{8w}$ group to the carboxylic acid group as above.

Compounds wherein $R^{3w}$ and $R^{3aw}$ are 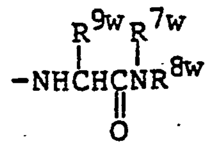 and 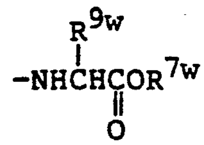

are prepared analogously to those wherein $R^{3w}$ and $R^{3aw}$ are $-NR^{7w}R^{8w}$.

Compounds wherein $Q^{w}$ is $R^{10w}CO-$ may be prepared by known methods, for example by adding a mercaptoacid of formula $R^{10w}COSH$ to an acrylic acid to obtain a thio-substituted propionic acid analogous to compounds of formula 1.

Starting materials of formulae 1 and 2 are known in the art or may be prepared by methods well known to those skilled in the art.

Following are descriptions of preparations of typical starting materials and examples of procedures for preparing compounds of formulae I-III. Temperature designations, i.e. reaction conditions and melting points, are in °C.

## PREPARATION 1

## L-CYSTEINE ESTERS

### Method 1:

#### S-(4-Methylbenzyl)-L-Cysteine, Methyl Ester, Hydrochloride:

At room temperature, add thionyl chloride (2.8 ml, 2.2 equiv.) dropwise to N-t-butyloxycarbonyl-S-(4-methylbenzyl)-L-cysteine (5.0g) in methanol (500 ml) and heat the resulting mixture under reflux for 90 minutes. Cool the reaction mixture to room temperature and concentrate *in vacuo* to give the title compound, a white solid (4.31g), m.p. 158-160°, $[\alpha]_D^{26}$= -22.9° (MeOH).

By the same method, other amino acid esters are prepared:

S-Benzyl-D-cysteine ethyl ester hydrochloride, a while solid, m.p. 149-151°, $[\alpha]_D^{26}$= +15.5° ($H_2O$);

S-(4-Methoxybenzyl)-L-cysteine methyl ester hydrochloride, a white solid, m.p. 145-6°, $[\alpha]_D^{26}$= -23.2° (MeOH);

S-(3,4-Dimethylbenzyl)-L-cysteine ethyl ester hydrochloride, white solid, m.p. 161-167°, $[\alpha]_D^{26}$= -26.6° (MeOH); and

S-t-Butyl-L-cysteine methyl ester, an oil.

### Method 2:

#### S-Phenethyl-L-Cysteine Ethyl Ester Hydrochloride:

Add thionyl chloride (2.0 ml) to absolute ethanol (25 ml) at 0-5°. To this solution add S-phenethyl-L-cysteine (2.5g, 1.11 mmole). Warm the resulting mixture to room temperature, and then heat at 60° for 5 hours. Concentrate the reaction mixture *in vacuo*, dissolve the resultant residue in dichloromethane ($CH_2Cl_2$), and concentrate the solution *in vacuo*.

Dissolve the residue in absolute ethanol, treat with DARCO, filter and concentrate in vacuo to give the title compound, a white solid (2.90g) m.p. 155-156°, $[\alpha]_D^{26}$= -1.5° (MeOH).

By the same method, other amino acid esters are prepared:

O-Benzyl-L-tyrosine methyl ester hydrochloride, a white solid, m.p. 189-190, $[\alpha]_D^{26}$= +6.9° (MeOH);

S-Methyl-L-cysteine ethyl ester, an oil, $[\alpha]_D^{26}$= +25.1° (MeOH);

S-Ethyl-L-cysteine ethyl ester hydrochloride, a white solid, m.p. 130-3° $[\alpha]_D^{26}$= -11.0° (MeOH); and

Ethionine ethyl ester, hydrochloride, clear oil, $[\alpha]_D^{26}$= +15.5° (MeOH).

## PREPARATION 2

## 3-ACETYLTHIO-2-(ARYLMETHYL)PROPIONIC ACIDS

### 3-Acetylthio-2-(4-phenylbenzyl)propionic acid

Step 1: 4-Biphenylmethylenemalonic acid: Heat 4-biphenyl carboxaldehyde (18.0g, 9.89 mmole) and malonic acid (10.8g, 10.4 mmole) in glacial acetic acid (6 ml) at approximately 100° for 2 hours. Cool the reaction mixture, dilute with dichloromethane and filter to give a white solid (8.76g). Concentrate the filtrate, add malonic acid (1.5g) and heat the resulting mixture at 100° for 2 hours. Cool the reaction mixture, dilute with $CH_2Cl_2$ and filter to give a white solid (3.44g). Suspend the combined solids (12.20g) in water and filter to give the title compound, a pale yellow solid (9.56g), m.p. 208-9°↑.

By this method other aryl methylene malonic acids are prepared, for example:

β-Naphthylmethylene malonic acid, a white solid, m.p. 204-205°.

Step 2: (4-Phenylbenzyl)malonic acid: Hydrogenate the product of Step 1 (9.50g, 3.5 mmole) in ethyl acetate (200 ml) in the presence of 10% palladium-on-charcoal (0.80g) at 50 psi for 3 hours. Filter and concentrate the reaction mixture *in vacuo* to give the title compound, a white solid (8.16g) m.p. 180-181°.

By this method other arylmethyl malonic acids are prepared, for example:

β-Naphthylmethyl malonic acid, a white solid, m.p. 150-152°.

Step 3: 2-(4-Phenylbenzyl)acrylic acid: Treat a portion of the product of Step 2 (4.05g, 1.50 mmole) in water (20 ml) at 0-5° with 40% dimethylamine in water to pH 7-8. Add the remaining product of Step 2 (4.05g, 1.50 mmole). After 15 minutes, add aqueous formaldehyde (10.0 ml, 38%). Slowly warm the resulting mixture to room temperature and stir for 18 hours. Filter the reaction mixture, wash the white solid with water, and suspend the solid in water (150 ml). Heat this suspension at 100° for 3 hours until the solution is almost clear. Cool the solution and add concentrated hydrochloric acid to pH 2 to give a white precipitate. Filter the mixture and dry the white solid. Dissolve this white solid in hot methanol, filter and concentrate *in vacuo* to give the title compound, a white solid (6.68g) m.p. 168-170°.

By this method other 2-(aryl)acrylic acids are prepared, for example:

2-(β-naphthylmethyl)acrylic acid, a white solid, m.p. 83-84°.

Step 4: 3-Acetylthio-2-(4-phenylbenzyl)propionic acid: Add thioacetic acid (8.0 ml) to the product of Step 3 (6.0g, 2.77 mmole) in $CH_2Cl_2$ (30 ml) and ethyl acetate (100 ml) and stir the resulting mixture at room temperature for 72 hours. Concentrate the reaction mixture *in vacuo*. Dissolve the residue in toluene (100 ml), and concentrate *in vacuo* (3 times) to give a yellow oil (6.0g). Chromatograph the oil on a column of silica gel (1.6L), eluting with $CH_2Cl_2$ (4L) $CH_2Cl_2$:methanol 1000:1 (3L), and 1000:5 (15L) to give the title compound, a white solid (3.44g), m.p. 101-103°.

By this method, other 3-acetylthio-2-(aryl methyl)propionic acids are prepared:

3-Acetylthio-2-(4-chlorobenzyl)propionic acid, an oil.

3-Acetylthio-2-(α-naphthylmethyl)propionic acid, a white solid, m.p. 94-7°; and

3-Acetylthio-2-(β-naphthylmethyl)propionic acid, a white solid, m.p. 103-106°.

## PREPARATION 3

## 1-(α-NAPHTHYLMETHYL)ACRYLIC ACID

Step 1: Diethyl α-naphthylmethyl malonate: Add sodium metal (11.0g, 0.478 mole) to absolute ethanol (650 ml) with cooling and stirring until the sodium is dissolved. Add diethyl malonate (75.6g, 0.473 mole) over 15 minutes at room temperature. After 30 min., add α-bromomethylnaphthalene (100g, 0.452 mole) in absolute ethanol (400 ml). Heat the resulting mixture under reflux for 5 hours. Keep at room temperature for 20 hours, and concentrate *in vacuo*. Partition the residue between water (500 ml) and diethyl ether (700 ml). Extract the diethyl ether solution with water (200 ml),

and brine (200 ml), then dry the organic layer ($MgSO_4$) and concentrate in vacuo to give the title compound, an oil (133.7g).

Step 2: 3-(1-Naphthyl)-2-ethoxycarbonylpropionic acid: To the product of Step 1 (133.7g, 0.446 mole) in absolute ethanol (400 ml) add a solution of potassium hydroxide (24.9g, 0.445 mole) in absolute ethanol (400 ml) and stir the resulting mixture at room temperature for 20 hours. Concentrate the reaction mixture in vacuo and partition the residue between ice water (1L) and diethyl ether (500 ml). Cool the aqueous solution to 0-5°C and acidify to approximately pH 2 with 2N hydrochloric acid, keeping the temperature at 0-5°. Extract the mixture with diethyl ether, dry the organic layer ($MgSO_4$) and concentrate in vacuo to give the title compound, an oil (100g).

Step 3: Ethyl 1-(α-naphthylmethyl)acrylate: Add to the product of Step 2 (100g, 0.367 mole) and diethylamine (39 ml) a 37% aqueous solution of formaldehyde (38 ml) over 30 min. at 0-5°C with vigorous stirring. Stir the mixture at room temperature for 7 hours and then extract with diethyl ether (3x500 ml). Extract the organic layer with 2N hydrochloric acid (2x500 ml), saturated aqueous sodium bicarbonate solution (500 ml) and brine (500 ml). Dry the organic layer ($MgSO_4$) and concentrate in vacuo to give the title compound, an oil (58.6g).

Step 4: 1-(α-Naphthylmethyl)acrylic acid: Treat the product of Step 3 (12.0g, 50 mmole) in dioxane (50 ml) with 1N sodium hydroxide (60 ml) and stir the resulting mixture at room temperature for 18 hours. Concentrate the reaction mixture under nitrogen, dilute with water and extract with ethyl acetate. Cool the aqueous solution to 0-5° and add concentrated hydrochloric acid

slowly to pH 3-4 to give a white solid. Filter the reaction mixture, wash the resultant white solid with water and dry to give the title compound, a white solid (9.62g), m.p. 115-117°.

## PREPARATION 4

## L-BISHOMOPHENYLALANINE t-BUTYL ESTER [(2S)-AMINO-5-PHENYLPENTANOIC ACID, t-BUTYL ESTER]

Step 1: 4-Benzoyl-2(S)-trifluoroacetamidobutyric acid: Heat at reflux for 3 hours a mixture of N-trifluoroacetyl-L-glutamic anhydride (18.0g, 80 mmol) and $AlCl_3$ (23.5g, 177 mmol) in dry benzene (400 ml). Allow to cool, treat with ice (400 ml), conc. HCl (100 ml), and ethylacetate (EtOAc) (400 ml). Dry the organic layer and concentrate to obtain the title compound as a brown crystalline solid (25g).

Step 2: 5-Phenyl-2(S)-trifluoroacetamidopentanoic acid: Reduce the product of Step 1 (9.7g) with Pearlman's catalyst (3.5g) in EtOAc (75 ml) and ethanol (25 ml) at 50 psi $H_2$ for 3 hours. Filter, concentrate and wash the resultant residue with 3:1 hexane:diethyl ether to give the title compound (7.8g).

Step 3: N-Trifluoroacetyl-L-bishomophenylalanine, t-butyl ester: Treat the product of Step 2 (11.3g) with isobutylene (25 ml) and conc. $H_2SO_4$ (1.0 ml) in $CH_2Cl_2$ (100 ml) for 16 hours. Partition between diethyl ether and 1N $NaHCO_3$, dry, concentrate, and chromatograph the resultant residue on silica gel, eluting with 2:1 hexane:diethyl ether to obtain 11.3g of the title compound as a colorless oil.

Step 4: L-Bishomophenylalanine, t-butyl ester, hydrochloride: To the product to Step 3 in EtOH (120 ml) add $NaBH_4$ (5.2g) portionwise over 30 min. Stir another 1.5 hours, concentrate, and partition between diethyl ether and $H_2O$. Dry and concentrate to obtain an oil (8.1g). Treat with HCl:diethyl ether to give the hydrochloride salt of the title compound, white crystals (4.0g), m.p. 161-2°, $[\alpha]_D^{26}$= +15.6° (MeOH, c=0.5).

PREPARATION 5

S-(4-METHYLBENZYL)-L-CYSTEINE AMIDE

Step 1: N-t-Butyloxycarbonyl-S-(4-methylbenzyl)-L-cysteine amide: React N-t-butyloxycarbonyl-S-(4-methylbenzyl)-L-cysteine (6.50g) with triethylamine (4.44g, 6.16 ml) in tetrahydrofuran (THF). Cool the mixture to 0-5°. Add ethyl chloroformate (4.77g, 3.41 ml) in THF (5 ml) dropwise over 5 min. and stir the reaction mixture for 15 min. Add ammonium hydroxide (28%, 2.0 ml) in THF (5 ml) dropwise. Allow the reaction mixture to warm to room temperature and stir for 18 hr. Filter the reaction mixture and concentrate the filtrate in vacuo to give a pale yellow solid. Dissolve this solid in $CH_2Cl_2$ and extract with $H_2O$. Concentrate the dried ($MgSO_4$) $CH_2Cl_2$ solution in vacuo to give a pale yellow solid (6.41g). Recrystallize this solid from EtOAc to give the title compound, a white solid (2.82g), m.p. 140-141°, $[\alpha]_D^{26}$= -7.5° (MeOH).

Step 2: S-(4-Methylbenzyl)-L-cysteine amide: Treat the product of Step 1 (2.79g) in $CH_2Cl_2$ (40 ml) with trifluoroacetic acid (10 ml) at room temperature for 18 hr. Concentrate the reaction mixture in vacuo. Dissolve the residue in $CH_2Cl_2$ and concentrate in vacuo (twice).

Dissolve the white solid in EtOAc and extract with 10% sodium bicarbonate solution. Dry ($MgSO_4$) the EtOAc and concentrate <u>in vacuo</u> to give the title compound, a white solid (1.53g) m.p. 94-95°, $[\alpha]_D^{26}$= -1.3° (MeOH).

## PREPARATION 6

## S-(4-METHYLBENZYL)-L-CYSTEINE t-BUTYL ESTER

To a cold solution of isobutylene (50 ml) in dioxane (80 ml), add S-(4-methylbenzyl)-L-cysteine (5.0g) and concentrated $H_2SO_4$ (10 ml). Seal the vessel, allow to warm to room temperature, and stir for 18 hr. Pour into 5% NaOH (500 ml), extract with $Et_2O$ (3x400 ml), dry ($MgSO_4$) and concentrate the $Et_2O$ <u>in vacuo</u> to give an oil. Treat the oil with HCl in $Et_2O$ to give the title compound, white needles (2.76g) m.p. 218° (dec).

## EXAMPLE 1

## N-(2-BENZYL-3-MERCAPTOPROPIONYL)-S-(4-METHYLBENZYL)-L-CYSTEINE (ISOMERS A AND B)

<u>Step 1:</u> <u>N-(3-Acetylthio-2-Benzylpropionyl)-S-(4-Methylbenzyl)-L-Cysteine Methyl Ester (Isomers A & B)</u>: Add S-(4-methylbenzyl)-L-cysteine methyl ester, hydrochloride (1.85g, 0.77 mmole) to 3-acetylthio-2-benzyl propionic acid (1.93g, 0.81 mmole), DEC (1.46g, 0.76 mmole), HOBT (1.18g, 0.76 mmole) and NMM (2.30g, 2.5 ml, 2.27 mmole) in DMF (20 ml) and stir the resulting mixture at room temperature for 20 hours. Concentrate the reaction mixture <u>in vacuo</u> and partition the residue between EtOAc and water. Concentrate the dried ($MgSO_4$) ethyl acetate solution <u>in vacuo</u> to give an amber oil (3.85g). Chromatograph the oil on silica gel (Baker, 60-200 mesh) (1.5L) using EtOAc:hexane 4:21 as eluent to give Isomer A, white solid (0.64g), m.p. 101-104°, $[\alpha]_D^{26}$= -76.2° (MeOH); overlap Isomer A and Isomer B (0.40g); and Isomer B, white solid (0.67g), m.p. 52-55° $[\alpha]_D^{26}$= -4.9° (MeOH).

Step 2: N-(2-Benzyl-3-Mercaptopropionyl)-(S)-(4-Methylbenzyl)-L-Cysteine (Isomer B): Dissolve Isomer B (0.66g, 1.6 mmole) in methanol (20 ml) under a nitrogen atmosphere, cool to 0-5°, add 1N sodium hydroxide (4.8 ml), stir the mixture at 0-5° for 6 hours and then keep at that temperature for 18 hours. Concentrate the reaction mixture under nitrogen, dilute the resultant oil with water (200 ml) and ethyl acetate (200 ml) and acidify to pH 2-4 with 1N hydrochloric acid. Dry ($MgSO_4$) the ethyl acetate solution and concentrate *in vacuo* to give the title compound (Isomer B), a viscous oil (0.45g), $[\alpha]_D^{26}$= -56.3° (MeOH).

Step 3: N-(2-Benzyl-3-Mercaptopropionyl)-(S)-4-Methylbenzyl)-L-Cysteine (Isomer A): By a procedure similar to that of Step 2 react Isomer A (0.63g) and 1N sodium hydroxide (4.5 ml) to give the title compound (Isomer A), a viscous clear oil (0.165g), $[\alpha]_D^{26}$= -8.8° (MeOH).

In a similar manner, according to Example 1, Step 1, using the appropriate propionic acid, prepare:

N-[3-Acetylthio-2-(α-naphthylmethyl)propionyl]-S-(4-methylbenzyl)-L-cysteine ethyl ester, Isomer A, m.p. 71-74°, $[\alpha]_D^{26}$= -40.6° (MeOH);

N-[3-Acetylthio-2-(α-naphthylmethyl)propionyl]-S-(4-methylbenzyl)-L-cysteine ethyl ester, Isomer B, m.p. 88-90°, $[\alpha]_D^{26}$= -58.8° (MeOH);

N-[3-Acetylthio-2(β-naphthylmethyl)propionyl]-S-(4-methylbenzyl)-L-cysteine ethyl ester, Isomer A, m.p. 74-77°, $[\alpha]_D^{26}$= -61.0° (MeOH);

N-[3-Acetylthio-2(β-naphthylmethyl)propionyl]-S-(4-methylbenzyl)-L-cysteine ethyl ester, Isomer B, m.p. 86-88°, $[\alpha]_D^{26}$= -20.7° (MeOH);

N-[3-Acetylthio-2-(4-chlorobenzyl)propionyl]-S-benzyl-L-cysteine ethyl ester (Isomer A), m.p. 89-90°;

N-[3-Acetylthio-2-(4-chlorobenzyl)propionyl]-S-benzyl-L-cysteine ethyl ester (Isomer B), m.p. 103-4°; and

N-[3-Acetylthio-2-(4-chlorobenzyl)propionyl]-L-tryptophan methyl ester (Isomers A and B).

Using the procedure of Example 1, Step 2, treat the above 3-acetylthio compounds to obtain the following 3-mercaptopropionyl compounds:

N-[2-(α-Naphthylmethyl)-3-mercaptopropionyl]-S-(4-methylbenzyl)-L-cysteine Isomer A, m.p. 70-75°, $[\alpha]_D^{26}$= +18.3° (MeOH);

N-[2-(α-Naphthylmethyl)-3-mercaptopropionyl]-S-(4-methylbenzyl)-L-cysteine Isomer B, m.p. 48-55°, $[\alpha]_D^{26}$= -102.3° (MeOH);

N-[2-(β-Naphthylmethyl)-3-mercaptopropionyl]-S-(4-methylbenzyl)-L-cysteine, Isomer A, a white foam, $[\alpha]_D^{26}$= +9.9° (MeOH);

N-[2-(β-Naphthylmethyl)-3-mercaptopropionyl]-S-(4-methylbenzyl)-L-cysteine, Isomer B, a white foam, $[\alpha]_D^{26}$= -50.1° (MeOH);

N-[2-(4-chlorobenzyl)-3-mercaptopropionyl]-S-benzyl-L-cysteine (Isomer A), $[\alpha]_D^{26}$= -3.0° (EtOH, c=1);

N-[2-(4-chlorobenzyl)-3-mercaptopropionyl]-S-benzyl-L-cysteine (Isomer B), $[\alpha]_D^{26}$= -48.7° (EtOH, c=1);

N-[2-(4-chlorobenzyl)-3-mercaptopropionyl]-L-tryptophan (Isomer A), $[\alpha]_D^{26}$= +18.1° (EtOH, c=0.5); and

N-[2-(4-chlorobenzyl)-3-mercaptopropionyl]-L-tryptophan (Isomer B), $[\alpha]_D^{26}$= -22.5° (EtOH, c=0.5).

## EXAMPLE 2

### N-(2-BENZYL-3-MERCAPTOPROPIONYL)-S-BENZYL-L-CYSTEINE (ISOMERS A & B)

Step 1: N-(3-Acetylthio-2-Benzylpropionyl)-S-Benzyl-L-Cysteine, Ethyl Ester (Isomers A & B): React S-benzyl-L-cysteine ethyl ester hydrochloride (1.38g) and 3-acetylthio-2-benzyl propionic acid (1.19g) in a procedure similar to that described in Example 1, Step 1 to give a yellow oil. Chromatograph the oil on silica gel (1.5L, 60-200 mesh) using $CH_2Cl_2$:ethyl acetate 98:2 as eluant to give Isomer A, white solid (0.49g), m.p. 83-5°, $[\alpha]_D^{26}$= -73.5° (MeOH); overlap Isomer A and B (0.66g); and Isomer B, white solid, m.p. 72-4°, $[\alpha]_D^{26}$= -9.4° (MeOH).

Step 2: Using the procedure described in Example 1, Step 2, separately treat the Isomers of Step 1 above to obtain Isomers A and B of the title compound: Isomer A, a colorless oil, $[\alpha]_D^{26}$= -2.1° (MeOH), and Isomer B, a colorless oil, $[\alpha]_D^{26}$= -46.7° (MeOH).

## EXAMPLE 3

### N-(2-BENZYL-3-MERCAPTOPROPIONYL)-S-BENZYL-D-CYSTEINE (ISOMERS A AND B)

Step 1: N-(3-Acetylthio-2-Benzylpropionyl)-S-Benzyl-D-Cysteine, Ethyl Ester (Isomers A and B): React S-benzyl-D-cysteine ethyl ester hydrochloride (2.05g) and 3-acetylthio-2-benzylpropionic acid (1.77g) in a manner similar to that described in Example 1, Step 1 to give a light amber oil. Place the oil on a column of silica gel (2L, 60-200 mesh) and elute with $CH_2Cl_2$:ethyl acetate 98:2 to give Isomer A, white solid (0.70g), m.p. 84-85°;

$[\alpha]_D^{26}$= +75.9° (MeOH); overlap Isomer A and Isomer B (0.85g); and Isomer B, white solid (0.33g), $[\alpha]_D^{26}$= +15.6° (MeOH).

Step 2: Using the procedure described in Example 1, Step 2, separately treat the Isomers of Step 1 above to obtain Isomers A and B of the title compound: Isomer A, a colorless oil, $[\alpha]_D^{26}$= +13.5° (MeOH); and Isomer B, a colorless oil, $[\alpha]_D^{26}$= +38.2° (MeOH).

## EXAMPLE 4

## N-(2-BENZYL-3-MERCAPTOPROPIONYL)-S-(4-METHOXYBENZYL)-L-CYSTEINE (ISOMERS A AND B)

Step 1: N-(3-Acetylthio-2-Benzylpropionyl)-S-(4-Methoxybenzyl)-L-Cysteine Methyl Ester (Isomers A and B) React S-(4-methoxybenzyl)-L-cysteine methyl ester hydrochloride (1.85g) and 3-acetylthio-2-benzylpropionic acid (1.95g) in the manner described in Example 1, Step 1 to give an amber oil. Chromatograph this oil on a column of silica gel (2L, 60-200 mesh) and elute with ethyl acetate:hexane 5:20 to give Isomer A, a clear oil (0.63g), $[\alpha]_D^{26}$= -66.5° (MeOH), overlap Isomer A and Isomer B (0.28g); and Isomer B, a clear oil (0.67g), $[\alpha]_D^{26}$= +3.0° (MeOH).

Step 2: Using the procedure described in Example 1, Step 2 separately treat the isomers of Step 1 above to obtain Isomers A and B of the title compound: Isomer A, a viscous oil, $[\alpha]_D^{26}$= -19.3° (MeOH); and Isomer B, a viscous oil, $[\alpha]_D^{26}$= -44.2° (MeOH).

EXAMPLE 5

N-(2-BENZYL-3-MERCAPTOPROPIONYL)-S-(3,4-DIMETHYLBENZYL)-L-CYSTEINE (ISOMERS A AND B)

Step 1: N-(3-Acetylthio-2-Benzylpropionyl)-S-(3,4-Dimethylbenzyl)-L-Cysteine Ethyl Ester (Isomers A and B): React S-(3,4-dimethylbenzyl)-L-cysteine ethyl ester hydrochloride (2.20g) and 3-acetylthio-2-benzylpropionic acid (1.74g) in the manner described in Example 1, Step 1 to give an amber oil. Place the oil on a column of silica gel (1L) and elute with ethyl acetate:hexane 25:170 (4L) and then methanol:hexane 25:170 to give a light orange oily solid. Repeat the chromatography to give Isomer A, a white solid (0.52g), m.p. 89.5-92.5°, $[\alpha]_D^{26}$= -71.1° (MeOH) and Isomer B, a white solid (0.60g), m.p. 51-55°, $[\alpha]_D^{26}$= -8.4° (MeOH).

Step 2: Using the procedure described in Example 1, Step 2, separately treat the Isomers of Step 1 above, to obtain Isomers A and B of the title compound: Isomer A, a clear viscous oil, $[\alpha]_D^{26}$= -18.0° (MeOH); and Isomer B, a clear viscous oil, $[\alpha]_D^{26}$= -56.5° (MeOH).

EXAMPLE 6

N-(2-BENZYL-3-MERCAPTOPROPIONYL)-S-PHENETHYL-L-CYSTEINE (ISOMERS A AND B)

Step 1: N-(3-Acetylthio-2-Benzylpropionyl)-S-Phenethyl-L-Cysteine Ethyl Ester, (Isomers A and B): React S-phenethyl-L-cysteine ethyl ester hydrochloride (2.85g) and 3-acetylthio-2-benzylpropionic acid, (2.38g) in a manner similar to that described in Example 1, Step 1 to give an amber oil. Chromatograph this oil on Prep 500 (2

silica gel cartridges) and elute with $CH_2Cl_2$ (4L) and then $CH_2Cl_2$:ethyl acetate 100:2 to give Isomer A, a white solid (1.32g), m.p. 63-64°, $[\alpha]_D^{26}$= -51.2° (MeOH); overlap Isomer A and Isomer B (0.63g); and Isomer B, white solid (1.14g), m.p. 84-86°, $[\alpha]_D^{26}$= +5.3° (MeOH).

Step 2: Using the procedure described in Example 1, Step 2, separately treat Isomers A and B of Step 1 above to obtain Isomers A and B of the title compound. Isomer A, a colorless oil, $[\alpha]_D^{26}$= +4.8° (MeOH); and Isomer B, a colorless oil, $[\alpha]_D^{26}$= -39.7° (MeOH).

## EXAMPLE 7

## N-(2-BENZYL-3-MERCAPTOPROPIONYL)-S-(t-BUTYL)-L-CYSTEINE (Isomers A and B)

Step 1: N-(3-Acetylthio-2-Benzylpropionyl)-S-(t-Butyl)-L-Cysteine Methyl Ester (Isomers A and B): React S-(t-butyl)-L-cysteine methyl ester (2.32g) and 3-acetylthio-2-benzylpropionic acid (3.22g) in the manner described in Example 1, Step 1 to give an orange solid. Chromatograph this solid on a column of silica gel (2L, 60-200 mesh) and elute with ethyl acetate:hexane 3:17 to give Isomer A, a clear oil (1.09g), $[\alpha]_D^{26}$= -44.9° (MeOH); overlap Isomer A and Isomer B (0.52g); and Isomer B, a clear oil (0.75g), $[\alpha]_D^{26}$= +8.3° (MeOH).

Step 2: Using the procedure described in Example 1, Step 2, separately treat the Isomers above to obtain Isomers A and B of the title compound; Isomer A, a clear viscous oil, $[\alpha]_D^{26}$= +0.4° (MeOH), and Isomer B, a white solid, m.p. 68-75°, $[\alpha]_D^{26}$= -32.3° (MeOH).

## EXAMPLE 8

## N-(2-BENZYL-3-MERCAPTOPROPIONYL)-L-ETHIONINE (Isomers A and B)

Step 1: N-(3-Acetylthio-2-Benzylpropionyl)-L-Ethionine Ethyl Ester (Isomers A and B): React L-Ethionine ethyl ester (3.51g) and 3-acetylthio-2-benzylpropionic acid (4.37g) in a manner similar to that described in Example 1, Step 1 to give a yellow residue. Chromatograph the yellow residue on the Waters Prep 500 (2 silica gel cartridges) and elute with ethyl acetate:hexane 2:18 (16L), then ethyl acetate:hexane 3:17. Repeat chromatography of the fractions using ethyl acetate:hexane as eluant to give Isomer A, a white solid (0.89g), m.p. 84-90°, $[\alpha]_D^{26}$= -60.6° (MeOH) and Isomer B (0.82g), m.p. 79-84°, $[\alpha]_D^{26}$= -0.3° (MeOH).

Step 2: Using the procedure described in Example 1, Step 2, separately treat the Isomers of Step 1 above to obtain Isomers A and B of the title compound; Isomer A, a milky viscous oil, $[\alpha]_D^{26}$= -41.8° (MeOH); and Isomer B, a milky viscous oil, $[\alpha]_D^{26}$= -66.0° (MeOH).

## EXAMPLE 9

## N-(2-BENZYL-3-MERCAPTOPROPIONYL)-O-BENZYL-L-TYROSINE (Isomers A and B)

Step 1: N-(3-Acetylthio-2-benzylpropionyl)-O-benzyl-L-tyrosine Methyl Ester (Isomers A and B): React O-benzyl-L-tryosine methyl ester hydrochloride (2.77g) and 3-acetylthio-2-benzylpropionic acid (2.05g) in a manner similar to that described in Example 1, Step 1 to give a yellow-orange oil. Chromatograph this oil on a column of

silica gel (2.5L) and elute with $CH_2Cl_2$:ethyl acetate 98:2 to give Isomer A, a white solid (0.84g) m.p. 108-109°; $[\alpha]_D^{26}$= -39.8° (MeOH); overlap Isomer A and Isomer B (0.80g); and Isomer B, white solid (0.45g), m.p. 92-93°, $[\alpha]_D^{26}$= +19.2° (MeOH).

Step 2: Using the procedure described in Example 1, Step 2, separately treat the Isomers of Step 1 above to obtain Isomers A and B of the title compound: Isomer A, an off-white solid, $[\alpha]_D^{26}$= +4.8° (MeOH); and Isomer B, a viscous colorless oil $[\alpha]_D^{26}$= +2.4° (MeOH).

## EXAMPLE 10

### N-(2-BENZYL-3-MERCAPTOPROPIONYL)-(S)-BISHOMOPHENYL ALANINE (Isomers A and B)

Step 1: N-(3-Acetylthio-2-Benzylpropionyl)-(S)-Bishomophenylalanine t-Butyl Ester (Isomers A and B): React (S)-bishomophenylalanine t-butyl ester hydrochloride (2.49g) and 3-acetylthio-2-benzylpropionic acid (2.39g) in the manner described in Example 1, Step 1 to give a yellow oil. Chromatograph this oil on Waters Prep 500 (2 silica gel cartridges) and elute with $CH_2Cl_2$ (4L) and then $CH_2Cl_2$:ethyl acetate 100:2 to give Isomer A, a colorless oil (0.99g), $[\alpha]_D^{26}$= -54.7° (MeOH), overlap Isomer A and Isomer B (0.62g); and Isomer B, a colorless oil (0.79g), $[\alpha]_D^{26}$= +5.1° (MeOH).

Step 2: N-(3-Acetylthio-2-Benzylpropionyl)-(S)-Bishomophenylalanine (Isomer A and B): To Isomer A of the product of Step 1 (0.97g, 0.21 mmole) in $CH_2Cl_2$ (10 ml) at 0-5°, add dropwise trifluoroacetic acid (10 ml). Warm the resulting mixture to room temperature, stir for 18 hours, and concentrate in vacuo. Dissolve the residue

in $CH_2Cl_2$ (10 ml) and concentrate in vacuo. Treat the residue with diethyl ether (10 ml) and concentrate in vacuo to give Isomer A of the title compound, a light amber oil (0.87g), $[\alpha]_D^{26}$= -43.0° (MeOH).

By this same method, convert Isomer B of Step 1 to N-(3-acetylthio-2-benzylpropionyl)-(S)-bishomophenylalanine Isomer B, a light amber oil, $[\alpha]_D^{26}$= +19.6° (MeOH).

Step 3: N-(2-Benzyl-3-Mercaptopropionyl)-(S)-Bishomophenylalanine (Isomers A and B): Dissolve Isomer A of Step 2 in methanol (15 ml) at 0-5° under a nitrogen atmosphere and treat with 1N sodium hydroxide (6.3 ml). Treat the resulting mixture as described in Example 1, Step 2, to give Isomer A of the title compound, a pale yellow viscous oil (0.69g), $[\alpha]_D^{26}$= -25.4° (MeOH).

By this same method, convert Isomer B of Step 2 to N-(2-benzyl-3-mercaptopropionyl)-(S)-bishomophenylalanine Isomer B, a pale yellow viscous oil, $[\alpha]_D^{26}$= -50.0° (MeOH).

In a similar manner, substitute (S)-(4-methylbenzyl)-L-cysteine t-butyl ester (Preparation 6) for (S)-bishomophenylalanine in Example 10, Step 1 to obtain

N-(3-acetylthio-2-benzylpropionyl)-(S)-(4-methylbenzyl)-L-cysteine t-butyl ester, Isomer A, $[\alpha]_D^{26}$= -82.8° (MeOH); and

N-(3-acetylthio-2-benzylpropionyl)-(S)-(4-methylbenzyl)-L-cysteine t-butyl ester, Isomer B, $[\alpha]_D^{26}$= -23.5° (MeOH).

Treat the above esters in a manner similar to that described in Example 10, Step 2 to obtain

N-(3-acetylthio-2-benzylpropionyl)-(S)-(4-methylbenzyl)-L-cysteine, Isomer A, $[\alpha]_D^{26}$= -58.8° (MeOH); and

N-(3-acetylthio-2-benzylpropionyl)-(S)-(4-methylbenzyl)-L-cysteine, Isomer B, $[\alpha]_D^{26}$= -6.6° (MeOH).

EXAMPLE 11

N-[3-MERCAPTO-2(R,S)-BENZYLPROPIONYL]-L-METHIONINE

Step 1: N-[3-Acetylthio-2(R,S)-Benzylpropionyl]-L-Methionine Methyl Ester: Add methionine methyl ester (2.00g, 1.23 mmole) to 3-acetylthio-2-benzylpropionic acid (3.01g, 1.26 mmole), DEC (2.34g, 1.22 mmole), HOBT (1.88g, 1.23 mmole) and NMM (2.34g, 2.31 mmole) in DMF (12 ml), and treat the resulting mixture as described in Example 1, Step 1 to give an amber oil (4.36g). Chromatograph the oil on a column of silica gel (1L 60-200 mesh) elute with $CH_2Cl_2$ (1L) and then $CH_2Cl_2$:ethyl acetate 99:1 to give the title compound, a clear oil (2.61g), $[\alpha]_D^{26}$= -38.9° (MeOH).

Step 2: N-[3-Mercapto-2(R,S)-Benzylpropionyl]-L-Methionine: Dissolve the product of Step 1 in methanol (20 ml) and treat with 1N NaOH (20.4 ml) as described in Example 1, Step 2 to give the title compound, a white solid, m.p. 132-5°, $[\alpha]_D^{26}$= -34.6° (MeOH).

Using the method of Example 11, Step 1, other N-[3-(R,S)-Acetylthio-2-benzylpropionyl]amino acid esters are prepared:

N-[3-Acetylthio-2(R,S)-benzylpropionyl]-S-methyl-L-cysteine ethyl ester, a clear oil, $[\alpha]_D^{26}$= -25.9° (MeOH);

N-[3-Acetylthio-2(R,S)-benzylpropionyl]-S-trityl-L-cysteine methyl ester, an amber oil, $[\alpha]_D^{26}$= +5.9° (MeOH); and

N-[3-Acetylthio-2(R,S)-benzylpropionyl]-(S)-tryptophan methyl ester, a colorless oil, $[\alpha]_D^{26}$= -14.7° (MeOH).

Using the procedure of Example 11, Step 2, convert the above 3-acetylthio compounds to the following 3-mercaptopropionyl compounds:

N-[2(R,S)-Benzyl-3-mercaptopropionyl]-S-methyl-L-cysteine, a clear viscous oil, $[\alpha]_D^{26}$= -31.1° (MeOH);

N-[2(R,S)-Benzyl-3-mercaptopropionyl]-S-trityl-L-cysteine, a white solid, $[\alpha]_D^{26}$= +10.5° (MeOH); and

N-[2(R,S)-Benzyl-3-mercaptopropionyl]-(S)-tryptophan, a white foam, m.p. 68-69°, $[\alpha]_D^{26}$= -0.5° (MeOH).

## EXAMPLE 12

## N-[2-(4-PHENYLBENZYL)-3-MERCAPTOPROPIONYL]-S-(4-METHYL BENZYL)-L-CYSTEINE (Isomers A and B)

Step 1: 3-Acetylthio-2-(4-Phenylbenzyl)Propionyl Chloride: To 3-acetylthio-2-(4-phenylbenzyl)propionic acid (3.39g, 10.8 mmole) in toluene (25 ml) add 1% DMF in toluene (2 drops) and thionyl chloride (1.2 ml, 1.65g, 13.8 mmoles) and stir the resulting solution at room temperature for 18 hours. Concentrate the reaction mixture in vacuo, dissolve the residue in toluene (100 ml) and concentrate the solution in vacuo to give the title compound, a light brown oil (3.37g).

Step 2: N-[3-Acetylthio-2-(4-Phenylbenzyl)Propionyl]-S-(4-Methylbenzyl)-L-Cysteine (Isomers A and B): Add the acid chloride (3.37g) from Step 1 in acetonitrile (25 ml) dropwise to S-(4-methylbenzyl)-L-cysteine hydrochloride (2.62g, 10 mmol) in acetonitrile (30 ml), water (15 ml) and triethylamine (2.8 ml), and stir the resulting mixture at room temperature for 4 hours. Concentrate the reaction mixture in vacuo and partition the residue between ethyl acetate (700 ml) and water (2x200 ml) and

then saturated sodium chloride solution (100 ml). Dry the ethyl acetate solution ($MgSO_4$) and concentrate in vacuo to give a brown solid. Chromatograph this solid on a column of silica gel (2L, 60-200 mesh) and elute with $CH_2Cl_2$:methanol:glacial acetic acid (97.5:2.5:0.25) to give a white foam (2.45g). Chromatograph this white foam on a column of silica gel (1.2L, 60-200 mesh) and elute with $CH_2Cl_2$:methanol:glacial acetic acid (97.5:2.5:0.25) to give the title compound, a white solid (1.04g), m.p. 123-125°, $[\alpha]_D^{26}$= -45.5° (MeOH); overlap Isomers A and B (0.19g); and Isomer B of the title compound, a white solid (0.86g), m.p. 131-135°, $[\alpha]_D^{26}$= -7.1° (MeOH).

Step 3: N-[2-(4-Phenylbenzyl)-3-Mercaptopropionyl]-S-(4-Methylbenzyl)-L-Cysteine (Isomers A and B): Dissolve Isomer A of Step 2 in methanol saturated with ammonia (50 ml) at 0-5° under a nitrogen atmosphere. After 35 minutes, bubble nitrogen through the reaction mixture. Dilute the reaction mixture with water and acidify to pH 2-4 with 1N hydrochloric acid. Extract the acidic solution with ethyl acetate, dry the organic layer ($MgSO_4$) and concentrate in vacuo to give Isomer A of the title compound, a white solid (0.73g). Purify Isomer A by flash chromatography on silica gel (Baker flash silica gel, 40 μM) (25g) eluting with $CHCl_2$:MeOH:gl.AcOH, 97.5:2.5:0.25, to obtain a white solid (0.549g), $[\alpha]_D^{26}$= -2.2° (MeOH).

In a similar fashion, prepare Isomer B of the title compound, a white solid, $[\alpha]_D^{26}$= -62.2° (MeOH).

## EXAMPLE 13

## N-[2-BENZYL-3-MERCAPTOPROPIONYL]-L-METHIONINE AMIDE

Step 1: N-(3-Acetylthio-2-Benzylpropionyl)-L-Methionine Amide: In similar fashion to that described in Example

12, Step 2, convert L-methionine amide to N-(3-acetylthio-2-benzylpropionyl)-L-methionine amide. Recrystallize from hexane:$CH_2Cl_2$ to obtain a solid, m.p. 101-3°. Chromatograph on silica gel with 4% methanol/$CH_2Cl_2$ to obtain Isomer A, m.p. 149-51°, and Isomer B, m.p. 119-21°.

Step 2: N-(2-Benzyl-3-Mercaptopropionyl)-L-Methionine Amide: Treat the 3-acetylthio compound (mixture of Isomer A and B) with $NH_3$/MeOH for 4 hours as in Example 12, Step 3, to give the title compound, $[\alpha]_D^{26}$= -53.5° (EtOH, c=1), a mixture of diastereomers.

In a similar manner to that described in Example 13, Step 1, substitute the appropriate acetylthio compounds and amides and separate by chromatography to obtain:

N-(3-Acetylthio-2-benzylpropionyl)-S-(4-methylbenzyl)-L-cysteine amide, Isomer A, $[\alpha]_D^{26}$= -38.2° (MeOH);

N-(3-Acetylthio-2-benzylpropionyl)-S-(4-methylbenzyl)-L-cysteine amide, Isomer B, $[\alpha]_D^{26}$= -1.6° (MeOH);

N-[3-Acetylthio-2-(4-chlorobenzylpropionyl)]-L-methionine amide, Isomer A;

N-[3-Acetylthio-2-(4-chlorobenzylpropionyl)]-L-methionine amide, Isomer B, m.p. 166-9°;

Treat the amides obtained above in a manner similar to that described in Example 13, Step 2 to obtain:

N-[2-(4-Chlorobenzyl)-3-mercaptopropionyl]-L-methionine amide, Isomer A, m.p. 194°, $[\alpha]_D^{26}$= +1.2° (MeOH);

N-[2-(4-Chlorobenzyl)-3-mercaptopropionyl]-L-methionine amide, Isomer B, $[\alpha]_D^{26}$= -65.2° (MeOH);

N-(2-Benzyl-3-mercaptopropionyl)-S-(4-methylbenzyl)-L-cysteine amide, Isomer A, m.p. 130-2°, $[\alpha]_D^{26} = -4.2°$ (MeOH); and

N-(2-Benzyl-3-mercaptopropionyl)-S-(4-methylbenzyl)-L-cysteine amide, Isomer B, $[\alpha]_D^{26} = -29.0°$ (MeOH).

EXAMPLE 14

N-(3-BENZOYLTHIO-2-BENZYLPROPIONYL)-L-METHIONINE AMIDE

Prepare the R and S enantiomers of 3-benzoylthio-2-benzylpropionic acid according to the procedure described in U.S. 4,329,495, herein incorporated by reference.

In a manner similar to that described in Example 1, Step 1, condense each acid separately with L-methionine amide to obtain

N-(3(S)-benzoylthio-2-benzylpropionyl)-L-methionine amide, m.p. 178-180°, $[\alpha]_D^{26} = -97.6°$ (MeOH); and

N-(3(R)-benzoylthio-2-benzylpropionyl)-L-methionine amide, m.p. 145-9°, $[\alpha]_D^{26} = +32.9°$ ($CHCl_3$).

EXAMPLE 15

N-[2-BENZYL-3-MERCAPTOPROPIONYL]-L-ASPARTIC ACID β-BENZYL ESTER

In similar fashion to that described in Example 12, Step 2, convert L-aspartic acid β-benzyl ester to N-(3-acetylthio-2-benzylpropionyl)-L-aspartic acid, β-benzyl ester.

Treat with $NH_3$/MeOH as in Example 12, Step 3 to give the title compound, $[\alpha]_D^{26} = -5.7°$ (EtOH, c=0.5).

## EXAMPLE 16

### N-[N-(2-BENZYL-3-MERCAPTOPROPIONYL)-L-PHENYLALANYL]-L-ALANINE

Using the procedure of Example 1, convert L-phenylalanyl-L-alanine benzyl ester hydrochloride to N-(N-(3-acetylthio-2-benzylpropionyl)-L-phenylalanyl]-L-alanine, benzyl ester.

Treat with NaOH as in Example 1, Step 2 to give the crude title compound. React the product (0.62g) with zinc powder (0.5g) and 5N HCl (10ml) in 20ml MeOH for 1 hour. Concentrate the mixture, extract with $CH_2Cl_2$, dry, and remove the solvent to give the title compound, $[\alpha]_D^{26}$= -23.1° (EtOH, c=0.5).

Use the same procedure to prepare N-(N-(2-benzyl-3-mercaptopropionyl)-L-phenylalanyl-L-leucine, $[\alpha]_D^{26}$= -20.8° (EtOH, c=0.5).

## EXAMPLE 17

### N-[N-(2-BENZYL-3-MERCAPTOPROPIONYL)-L-ALANYL]-L-PROLINE

Step 1: N-[N-(3-Acetylthio-2-Benzylpropionyl)-L-Alanyl]-L-Proline: Using the procedure of Example 12, Step 2, convert L-alanyl-L- proline to N-[N-(3-acetylthio-2-benzylpropionyl)-L-alanyl]-L-proline, a white foam, $[\alpha]_D^{26}$ = -81.8° (MeOH).

Step 2: N-[N-(2-Benzyl-3-Mercaptopropionyl)-L-Alanyl]-L-Proline: Treat the product from Step 1 with methanol saturated with ammonia as described in Example 12, Step 3 (before chromatography). Treat the resultant residue with zinc powder as described in Example 17.

Chromatograph the product on flash grade silica gel using $CH_2Cl_2$:MeOH:$NH_4OH$ (97.5:2.5:0.25) to give the title compound, $[\alpha]_D^{26} = -118.2°$ (MeOH).

Examples of ACE inhibitors are those disclosed in the article by Wyvratt et al., cited above, and in the following patents and published patent applications:

(a) U.S. Patent No. 4,105,776 to Ondetti et al. discloses proline derivatives which are angiotensin converting enzyme (ACE) inhibitors and have the general formula

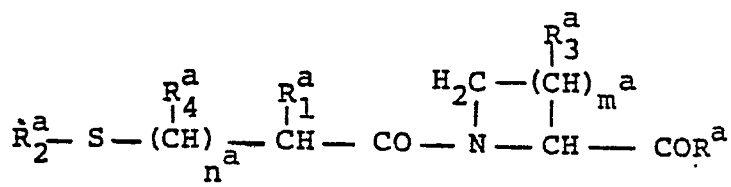

wherein $R^a$ is hydroxy, $NH_2$ or lower alkoxy;
$R_1{}^a$ and $R_4{}^a$ each is hydrogen, lower alkyl, phenyl or phenyl-lower alkyl;
$R_2{}^a$ is hydrogen, lower alkyl, phenyl, substituted phenyl wherein the phenyl substituent is halo, lower alkyl or lower alkoxy, phenyl-lower alkyl, diphenyl-lower alkyl, triphenyl-lower alkyl, lower alkylthiomethyl, phenyl-lower alkylthiomethyl, lower alkanoyl-amidomethyl,

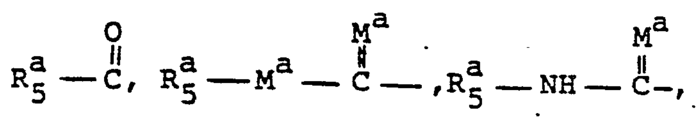

$R_6^a$-S-, or $R_7^a$;

$R_3^a$ is hydrogen, hydroxy or lower alkyl;

$R_5^a$ is lower alkyl, phenyl or phenyl-lower alkyl;

$R_6^a$ is lower alkyl, phenyl, substituted phenyl (wherein the phenyl substituent is halo, lower alkyl or lower alkoxy), hydroxy-lower alkyl or amino (carboxy)-lower alkyl;

$R_7^a$ is 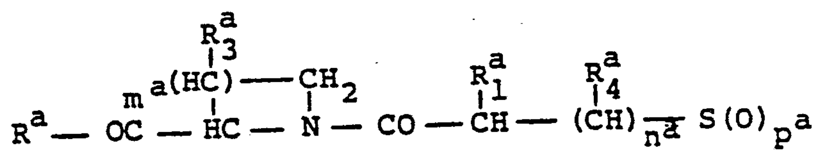

$M^a$ is O or S;

$m^a$ is 1 to 3;

$n^a$ and $p^a$ each is 0 to 2.

(b) European Patent Application 97050 published December 28, 1983 discloses phosphinylalkanoyl substituted prolines which have the formula

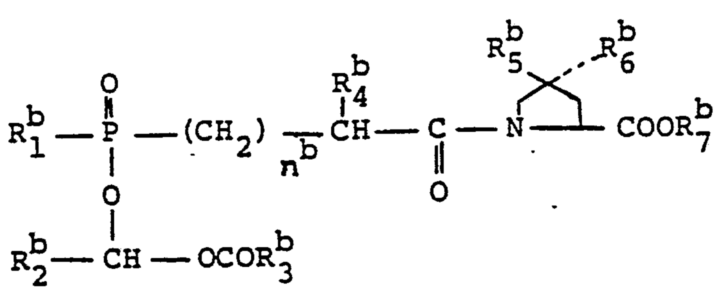

$R_1^b$ is alkyl, aryl, aralkyl, cycloalkyl or cycloalkylalkyl;
$R_2^b$ is cycloalkyl, 3-cyclohexenyl or 2-alkyl-3-cyclohexenyl;
$R_3^b$ is alkyl, cycloalkyl or phenyl;
$R_4^b$ is H or alkyl;
one of $R_5^b$ and $R_6^b$ is H and the other is alkyl-$X^b$-, phenyl-$X^b$, alkoxy, phenoxy, phenyl, cycloalkyl, alkyl or phenylalkyl; or
$R_5^b$ and $R_6^b$ together form $-X^bCH_2CH_2X^b-$, $X^b$ is S, SO or $SO_2$;
$R_7^b$ is H or $CH(R_2^b)OCOR_3^b$;
$n^b$ is O or 1;
'aryl' is phenyl opt. substituted by halo, alkyl, alkoxy, alkylthio, OH, alkanoyl, $NO_2$, amino, dialkylamino and/or $CF_3$;
alkyl has 1-10C, cycloalkyl 3-7C, alkoxy 1-8C and alkanoyl 2-9C.

(c) European Patent Application 83172 published July 6, 1983 discloses N-substituted acetyl-L-proline derivatives which are said to be angiotensin converting enzyme inhibitors and have the formula

$$R_1^cCO-NH-CHR_2^c-CH_2-\underset{\displaystyle OR_3^c}{\overset{\displaystyle O}{\overset{\|}{P}}}-A^cCO-X^c$$

$R_1^c$ and $R_2^c$ are H, 1-7C alkyl, 1-7C haloalkyl, $(CH_2)_{m^c}-D^c$, 1-7C aminoalkyl or a group of formula:

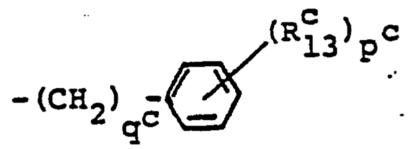

$D^c$ is cycloalkyl, furyl, thienyl or pyridinyl;
$A^c$ is $(CH_2)_n$-$CHR_{21}{}^c$, $NR_{22}{}^c$-$CHR_{23}{}^c$ or O-$CHR_{23}{}^c$;
$n^c$ is 0 or 1;
$q^c$ is 0-7;
$R_{21}{}^c$ is H, 1-7C alkyl, 1-7C haloalkyl, benzyl or phenethyl;
$R_{22}{}^c$ is H or 1-7C alkyl;
$R_{23}{}^c$ is H, 1-7C alkyl, 1-7C haloalkyl or $(CH_2)_r{}^cD_1{}^c$;
$D_1{}^c$ is phenyl, 4-hydroxyphenyl, 3,4-dihydroxyphenyl, 3-indolyl, 4-imidazolyl, $NH_2$, SH, 1-7C alkylthio, guanidino or $CONH_2$;
$X^c$ is a group of formula $(II)^c$-$(V)^c$, or $NR_4{}^c$-$CHR_5{}^c$-$COOR_6{}^c$;

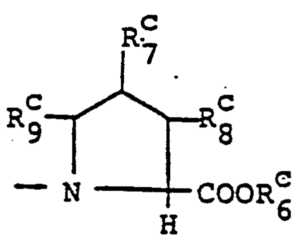

$(II)^c$

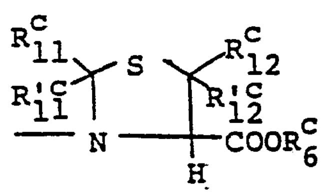

$(III)^c$

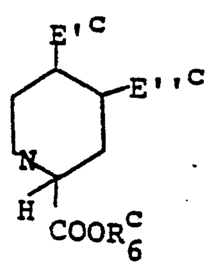

$(IV)^c$

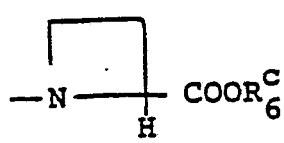

$(V)^c$

where

$R_9{}^c$ and $R_8{}^c$ are H and $R_7{}^c$ is as defined below;
$R_9{}^c$ and $R_7{}^c$ are H and $R_8{}^c$ is as defined below;
$R_7{}^c$ and $R_8{}^c$ are H and $R_9{}^c$ is as defined below;
$R_9{}^c$ and $R_8{}^c$ are H and $-CHR_7{}^c-$ is replaced by $-CR_{10}{}^cR_{10}{}^c$;
$R_9{}^c$ is H and $R_7{}^c + R_8{}^c$ form a double bond;
$R_9{}^c$ is H and $R_7{}^c + R_8{}^c$ complete a fused benzene ring; or
$R_8{}^c$ is H and $R_9{}^c + R_7{}^c$ complete a fused benzene ring;
$E'^c$ and $E''^c$ are H or $E'^c + E''^c$ complete a fused benzene ring;
$R_7{}^c$ is H, 1-7C alkyl, halogen, keto, OH, 2-8C alkanoyl-amino, $N_3$, $NH_2$, $NR_{19}{}^cR_{20}{}^c$, $(CH_2)_m{}^c-D^c$, $O-CONR_{15}{}^cR_{15}{}^c$, 1-7C alkoxy, 1-7C alkylthio, or a group of formula $(VI)^c$, $(VII)^c$ or $(VIII)^c$:

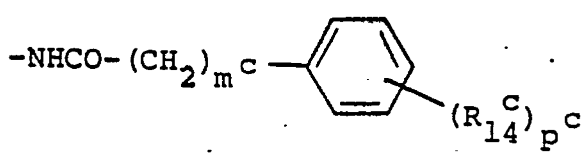

$(VI)^c$

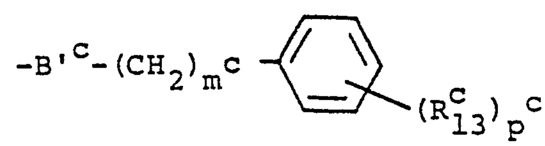

$(VII)^c$

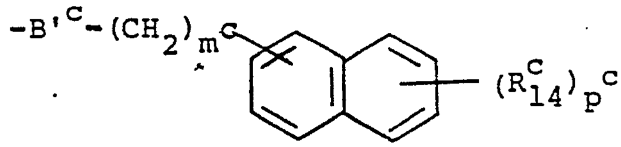

$(VIII)^c$

$B'^c$ is a bond or it is O or S;$R_8{}^c$ is keto, halogen, $O-CONR_{15}{}^cR_{15}{}^c$, 1-7C alkoxy, 1-7C alkylthio, or a group $(VII)^c$ or $(VIII)^c$ in which $B'^c$ is O or S;
$R_9{}^c$ is '.eto or a group $(VII)^c$ in which $B'^c$ is a bond;
$R_{10}{}^c$ is halogen or $Y^cR_{16}{}^c$;
$R_{11}{}^c$, $R'_{11}{}^c$, $R_{12}{}^c$ and $R'_{12}$ are H or 1-7C alkyl, or $R_{11}{}^c$ is a group of formula $(IX)^c$ and the other 3 are H;

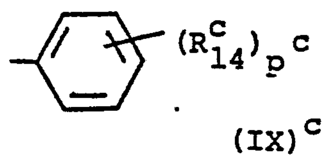

$(IX)^c$

$R_{13}{}^c$ is H, 1-4C alkyl, 1-4C alkoxy, 1-4C alkylthio, Cl, Br, F, $CF_3$, OH, Ph, PhO, PhS or $PhCH_2$;

$R_{14}{}^c$ is H, 1-4C alkyl, 1-4C alkoxy, 1-4C alkylthio, Cl, Br, F, $CF_3$ or OH;

$m^c$ is 0-3;

$p^c$ is 1-3 but it is 2 or 3 only when $R_{13}{}^c$ or $R_{14}{}^c$ is H, Me, MeO, Cl or F;

$R_{15}{}^c$ is H or 1-4C alkyl;

$Y^c$ is O or S;

$R_{16}{}^c$ is 1-4C alkyl or group. $(VII)^c$ in which $B'^c$ is a bond; or the two $R_{16}{}^c$ groups complete a 5- or 6-membered ring in which the C atoms may each bear 1 or 2 alkyls having 1-4C;

$R_4{}^c$ is H, 1-7C alkyl, cycloalkyl or $(CH_2)_r{}^cPh$;

$R_5{}^c$ is H, 1-7C alkyl or $(CH_2)_r{}^c-D_1{}^c$;

$r^c$ is 1-4;

$R_3{}^c$ and $R_6{}^c$ are H, 1-7C alkyl, $PhCH_2$, PhCH or $CHR_{17}{}^c-O-COR_{18}{}^c$;

$R_{17}{}^c$ is H, 1-7C alkyl or Ph;

$R_{18}{}^c$ is H, 1-7C alkyl, 1-7C alkoxy or Ph; or $R_{17}{}^c + R_{18}{}^c$ form $(CH_2)_2$, $(CH_2)_3$, -CH=CH or o-phenylene;

$R_{19}{}^c$ is 1-7C alkyl, benzyl or phenethyl; and

$R_{20}{}^c$ is H or chosen as for $R_{19}{}^c$.

(d) European Patent Application 0 012 401 published June 25, 1980 discloses carboxyalkyl dipeptide derivatives which are said to be angiotensin converting enzyme inhibitors and have the formula

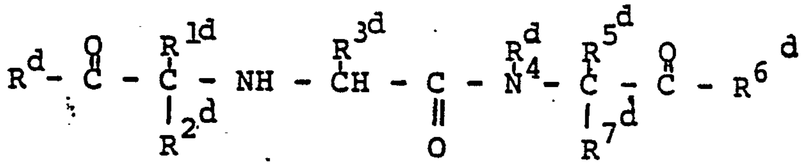

wherein

$R^d$ and $R^{6d}$ are the same or different and are hydroxy, lower alkoxy, lower alkenoxy, dilower alkylamino lower alkoxy (dimethylaminoethoxy), acylamino lower alkoxy (acetylaminoethoxy), acyloxy lower alkoxy (pivaloyloxymethoxy), aryloxy, such as phenoxy, arloweralkoxy, such as benzyloxy, substituted aryloxy or substituted arloweralkoxy wherein the substituent is methyl, halo or methoxy, amino, loweralkylamino, diloweralkylamino, hydroxyamino, arloweralkylamino such as benzylamino;

$R^{1d}$ is hydrogen, alkyl of from 1 to 20 carbon atoms which include branched and cyclic and unsatruated (such as allyl) alkyl groups, substituted loweralkyl wherein the substituent can be halo, hydroxy, lower alkoxy, aryloxy such as phenoxy, amino, diloweralkylamino, acyamino, such as acetamido and benzamido, arylamino, guanidino, imidazolyl, indolyl, mercapto, loweralkylthio, arylthio such as phenylthio, carboxy or carboxamido, carboloweralkoxy, aryl such as phenyl or naphthyl, substituted aryl such as phenyl wherein the substituent is lower alkyl, lower alkoxy or halo, arloweralkyl, arloweralkenyl, heteroarlower alkyl or heteroarlower alkenyl such as benzyl, styryl or indolyl ethyl, substituted arloweralkyl, substituted arloweralkenyl, substituted heteroarlower alkyl, or substituted heteroarlower alkenyl, wherein the

substituent(s) is halo, dihalo, lower alkyl, hydroxy, lower alkoxy, amino, aminomethyl, acylamino (acetylamino or benzoylamino) diloweralkylamino, loweralkylamino, carboxyl, haloloweralkyl, cyano or sulfonamido; arloweralkyl or heteroarloweralkyl substituted on the alkyl portion by amino or acylamino (acetylamino or benzoylamino);

$R^{2d}$ and $R^{7d}$ are the same or different and are hydrogen or lower alkyl;

$R^{3d}$ is hydrogen, lower alkyl, phenyl lower alkyl, aminomethyl phenyl lower alkyl, hydroxy phenyl lower alkyl, hydroxy lower alkyl, acylamino lower alkyl (such as benzoylamino lower alkyl, acetylamino lower alkyl), amino lower alkyl, dimethylamino lower alkyl, halo lower alkyl, guanidino lower alkyl, imidazolyl lower alkyl, indolyl lower alkyl, mercapto lower alkyl, lower alkyl thio lower alkyl;

$R^{4d}$ is hydrogen or lower alkyl;

$R^{5d}$ is hydrogen, lower alkyl, phenyl, phenyl lower alkyl, hydroxy phenyl lower alky, hydroxy lower alkyl, amino lower alkyl, guanidino lower alkyl, imidazolyl lower alkyl, indolyl lower alkyl, mercapto lower alkyl or lower alkylthio lower alkyl;

$R^{4d}$ and $R^{5d}$ may be connected together to form an alkylene bridge of from 2 to 4 carbon atoms, an alkylene bridge of from 2 to 3 carbon atoms and one sulfur atom, an alkylene bridge of from 3 to 4 carbon atoms containing a double bond or an alkylene bridge as above substituted with hydroxy, loweralkoxy, lower alkyl or dilower alkyl;

and the pharmaceutically acceptable salts thereof.

(d) U.S. Patent No. 4,462,943 to Petrillo et al. discloses carboxyalkyl amino acid derivatives of substituted prolines which are angiotensin converting enzyme inhibitors and have the formula

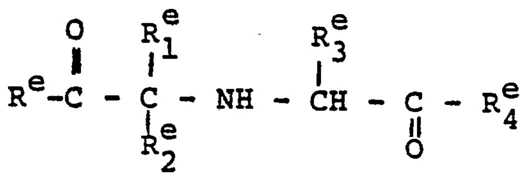

$R_4^e$ is a substituted proline of the formula

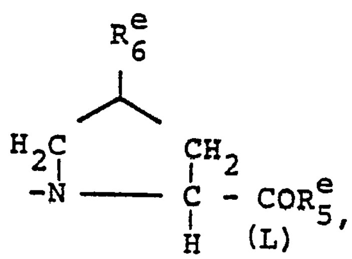

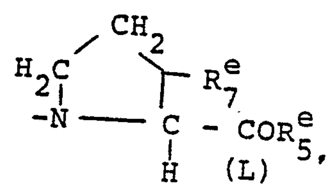

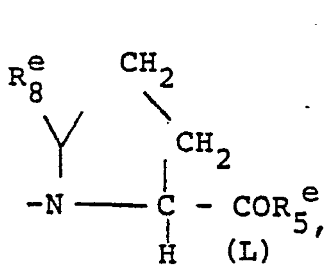

or

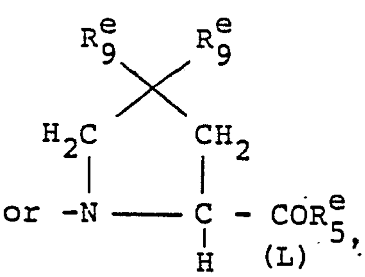

$R_6^e$ is halogen, keto, azido,

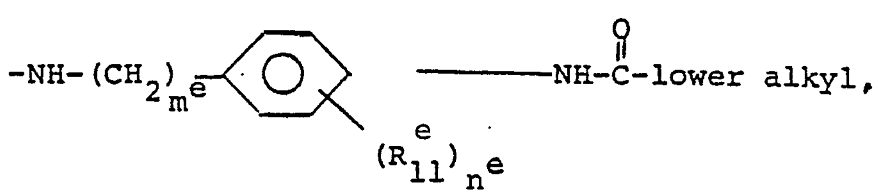

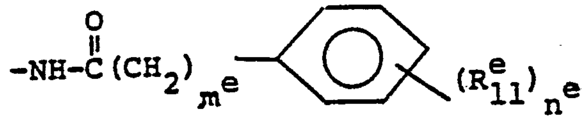

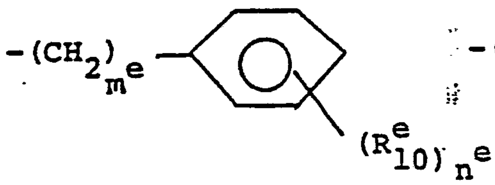

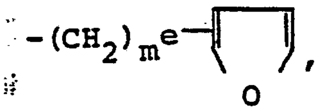,

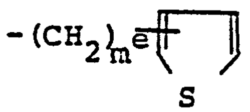,

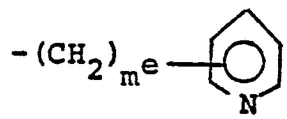,

a 1- or 2-naphthyl of the formula

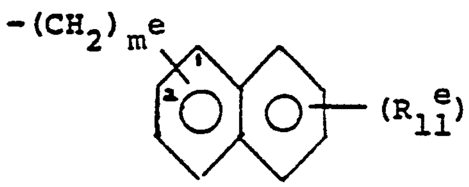

$-(CH_2)_{m^e}$-cycloalkyl,

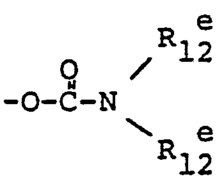

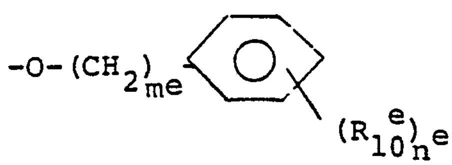

a 1- or 2-naphthyloxy of the formula

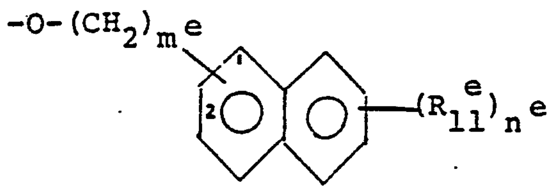

-S-lower alkyl,

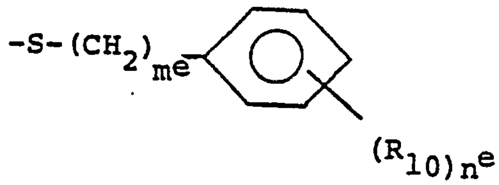

or a 1- or 2-naphthylthio of the formula

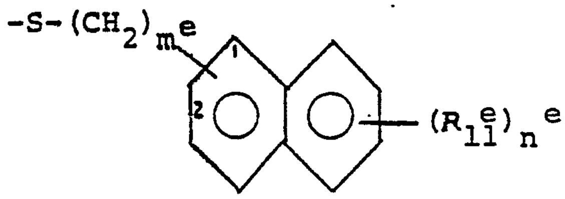

$R_7^e$ is keto, halogen,

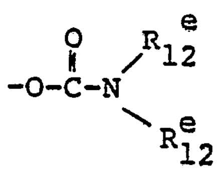

,

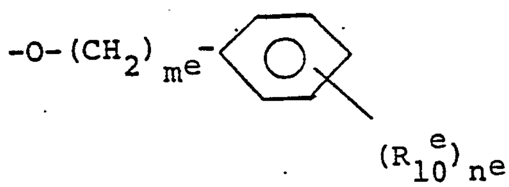

or 1- or 2-naphthloxy of the formula

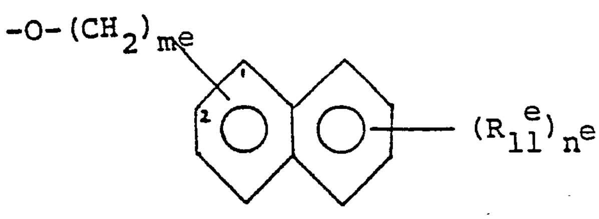

-S-lower alkyl,

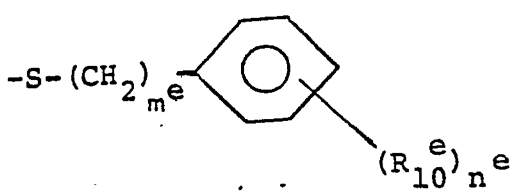

or a 1- or 2-naphthylthio of the formula

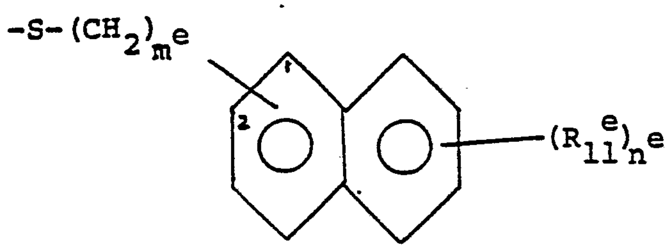

$R_8^e$ is keto or

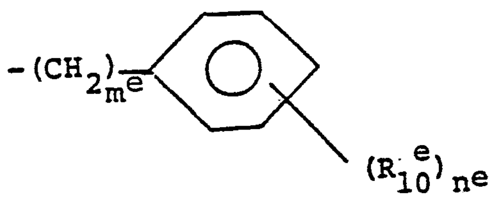

$R_9^e$ is halogen or $-Y^e-R_{13}^e$.
$m^e$ is zero, one, two, or three.
$R_{10}^e$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl,
$R_{11}^e$ is hydrogen, lower alkyl of 1 to 4 carbons, lower alkoxy of 1 to 4 carbons, lower alkylthio of 1 to 4 carbons, bromo, fluoro, trifluoromethyl, or hydroxy.
$n^e$ is one, two or three provided that $n^e$ is more than one only if $R_{10}^e$ or $R_{11}^e$ is hydrogen, methyl, methoxy, chloro, or fluoro.
$R_{12}^e$ is hydrogen or lower alkyl of 1 to 4 carbons,
$Y^e$ is oxygen or sulfur,
$R_{13}^e$ is lower alkyl of 1 to 4 carbons.

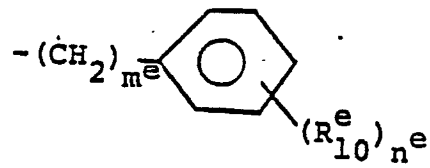

or the $R_{13}^{e}$ group join to complete an unsubstituted 5- or 6-membered ring or said ring in which one or more of the carbons has a lower alkyl of 1 to 4 carbons, or a di(lower alkyl of 1 to 4 carbons) substituent,
$R^{e}$ and $R_{5}^{e}$ are independently selected from hydroxy, lower alkoxy, di(lower alkyl)-amino-lower alkoxy, such as dimethylaminoethoxy, lower alkyl-carbonyl-amino-lower alkoxy, such as acetylaminoethoxy, lower alkyl-carbonyloxy-lower alkoxy, such as pivaloyloxymethoxy,

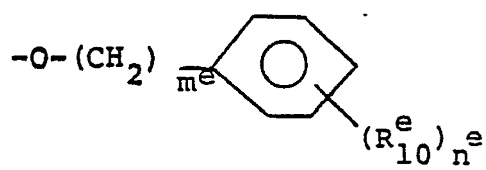

wherein $m^{e}$, $n^{e}$ and $R_{10}^{e}$ are as defined above, amino, lower alkyl-amino, di(lower alkyl)-amino, hydroxyamino, benzylamino, or phenethylamino,
$R_{1}^{e}$ is hydrogen, lower alkyl,

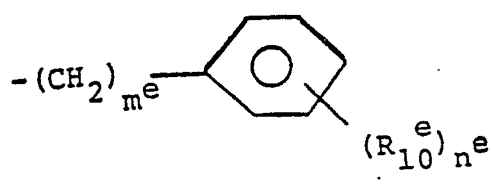

halo substituted lower alkyl, hydroxy substituted lower alky,
$-(CH_2)_{8}^{e}$-cycloalkyl, $-(CH_2)_{q}^{e}$ carboxy, $-(CH_2)_{q}^{e}$-S-lower alkyl,

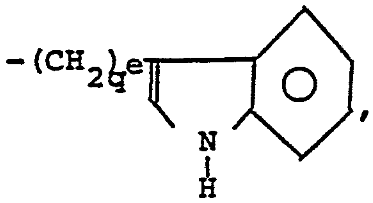, 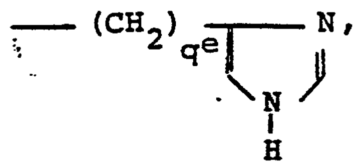,

$-(CH_2)_q{}^e$-guanidinyl, $-(CH_2)_q-NH_2$,

$-(CH_2)_q{}^e-N$(lower alkyl)$_2$,

$-(CH_2)_q{}^e-NH-\overset{O}{\overset{\|}{C}}-$lower alkyl,

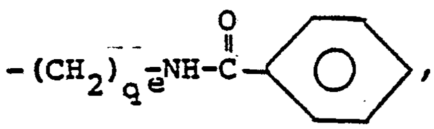, $-(CH_2)_{q^e}-SH$,

$-(CH_2)_{q^e}-$lower alkoxy, 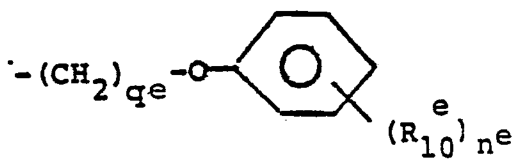

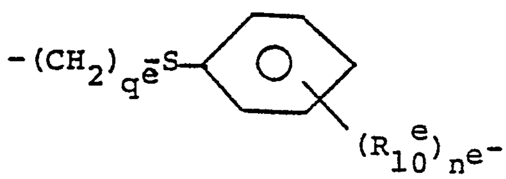 $-(CH_2)_{q^e}-\overset{O}{\overset{\|}{C}}-NH_2$

$-(CH_2)_{q^e}-\overset{O}{\overset{\|}{C}}-$lower alkoxy, or

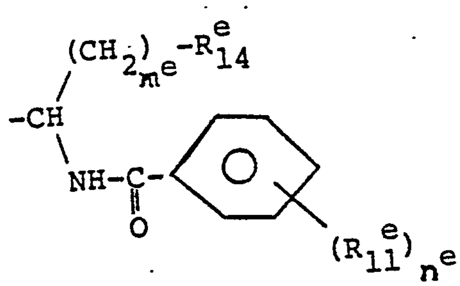

wherein $m^e$, $n^e$, $R_{10}^e$ and $R_{11}^e$ are as defined above, $R_{14}^e$ is lower alkyl, cycloalkyl, or

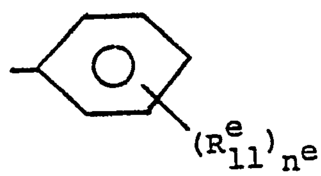

and $q^e$ is an integer from 1 to 4,
$R_2^e$ is hydrogen or lower alkyl,
$R_3^e$ is hydrogen, lower alkyl, halo substituted lower alkyl, hydroxy substituted lower alkyl, $-(CH_2)q^e-NH_2$, $-(CH_2)q^e-N-(\text{lower alkyl})_2$, $-(CH_2)q^e$ guanidinyl, $-(CH_2)q^e-SH$, $-(CH_2)q^e-S-$

$-(CH_2)_{q^e}-\overset{O}{\overset{\|}{C}}-NH_2$, 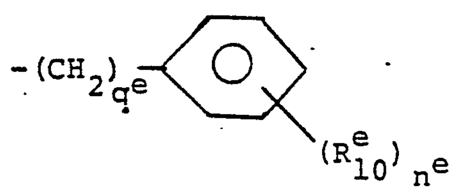,

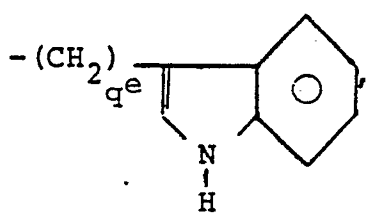, 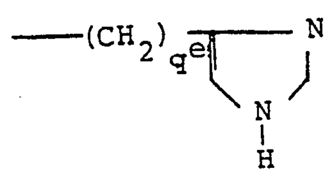,

$-(CH_2)_{q^e}-NH-\overset{O}{\overset{\|}{C}}-$lower alkyl, or

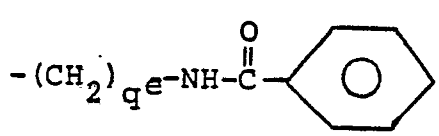

wherein $R_{10}^e$, $n^e$ and $q^e$ are as defined above.

(f) U.S. Patent No. 4,470,973 to Natarajan et al. discloses substituted peptides which are angiotensin converting enzyme inhibitors and have the formula

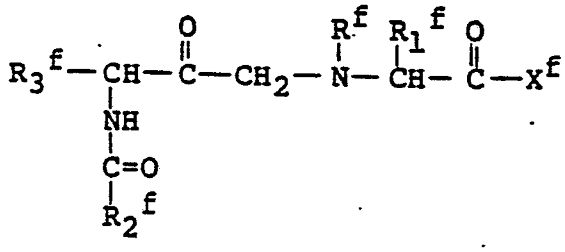

$X^f$ is an amino or imino acid of the formula

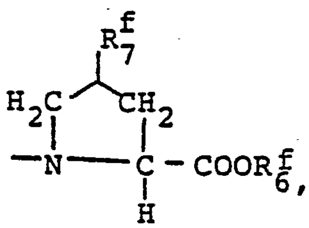

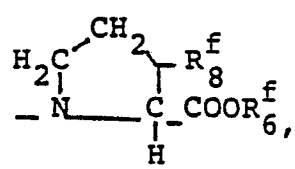

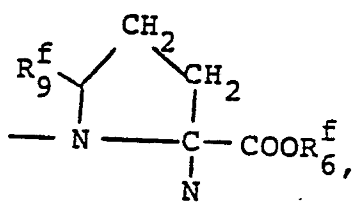

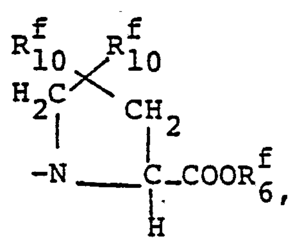

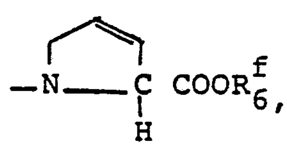

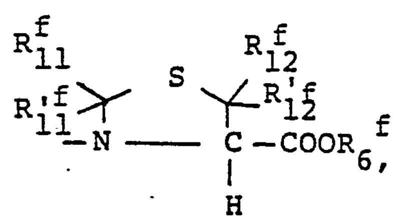

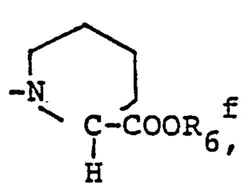

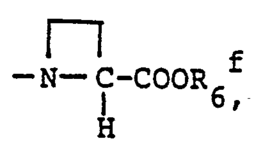

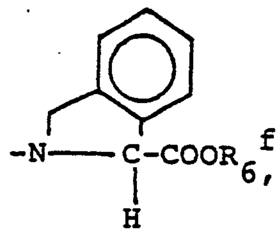

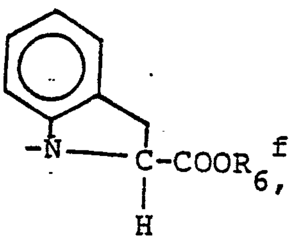

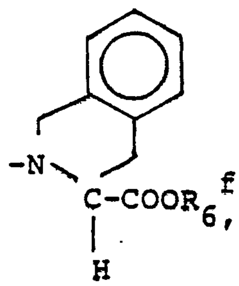

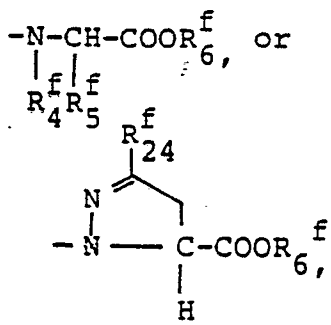
or

$R_7^f$ is hydrogen, lower alkyl, halogen, keto, hydroxy

$$-NH-\overset{O}{\overset{\|}{C}}-\text{lower alkyl}$$

azido, amino,

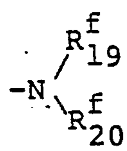
,

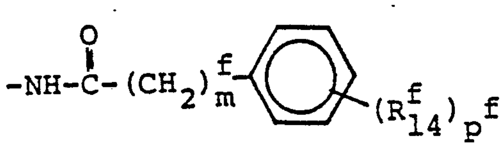

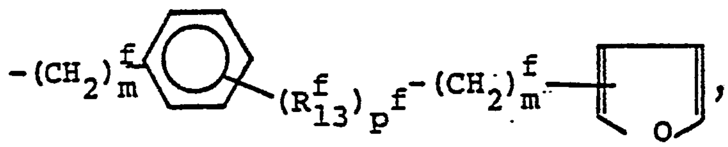

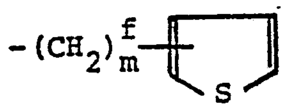

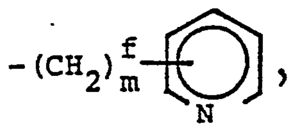

a 1- or 2-naphthyl of the formula

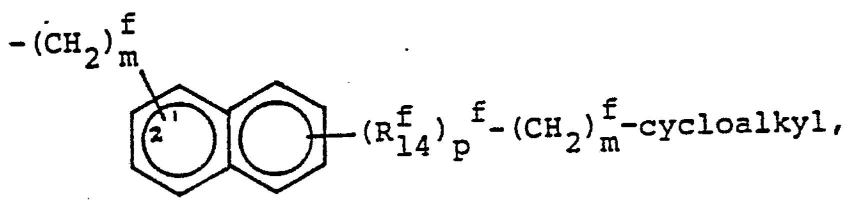

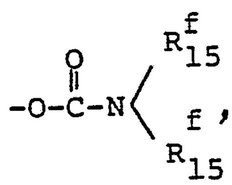, -O-lower alkyl,

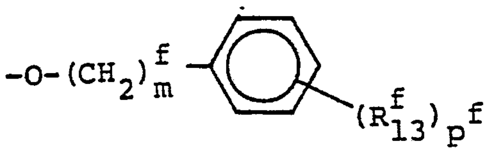

a 1- or 2-naphthyloxy of the formula

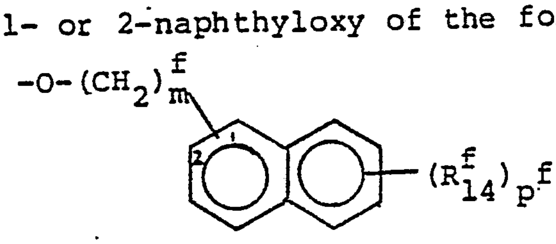

-S-lower alkyl,

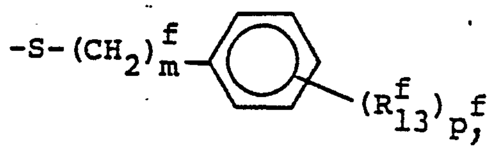

or a 1- or 2-naphthylthio of the formula

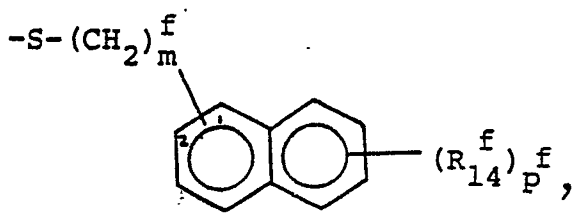

$R_8^f$ is keto, halogen.

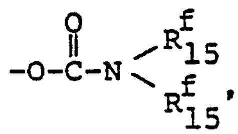

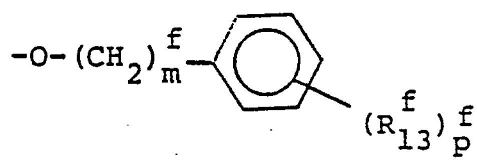

-O-lower alkyl, a 1- or 2-naphthyloxy of the formula

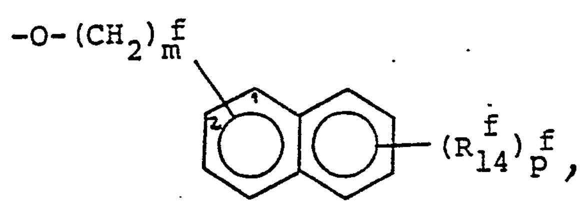

-S-lower alkyl

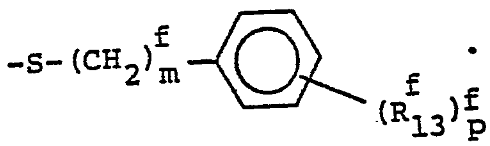

or a 1- or 2-naphthylthio of the formula

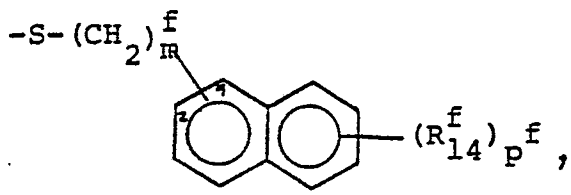

$R_9^f$ is keto or

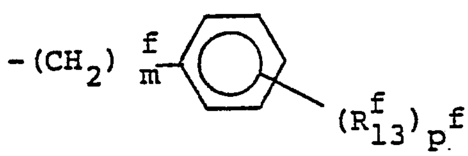

$R_{10}^f$ is halogen or $-Y^f-R_{16}^f$.
$R_{11}^f$, $R_{11'}^f$, $R_{12}^f$ and $R_{12'}^f$ are independently selected from hydrogen and lower alkyl or $R_{11'}^f$, $R_{12}^f$ and $R_{12'}^f$ are hydrogen and $R_{11}^f$ is

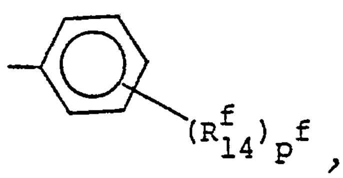

$R_{13}^f$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl.

$R_{14}^{f}$ is hydrogen, lower alkyl of 1 to 4 carbons, lower alkoxy of 1 to 4 carbons, lower alkylthio of 1 to 4 carbons, chloro, bromo, fluoro, trifluoromethyl, or hydroxy.

$m^f$ is zero, one, two, three, or four.

$p^f$ is one, two or three provided that p is more than one only if $R_{13}^{f}$ or $R_{14}^{f}$ is hydrogen, methyl, methoxy, chloro, or fluoro.

$R_{15}^{f}$ is hydrogen or lower alkyl of 1 to 4 carbons.

$Y^f$ is oxygen or sulfur.

$R_{16}^{f}$ is lower alkyl of 1 to 4 carbons.

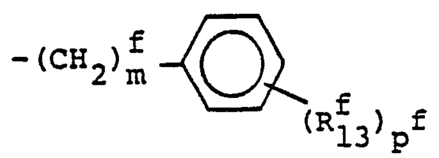,

or the $R_{16}^{f}$ groups join to complete an unsubstituted 5- or 6-membered ring or said ring in which one or more of the carbons has a lower alkyl of 1 to 4 carbons or a di(lower alkyl of 1 to 4 carbons) substituent.

$R_4^f$ is hydrogen, lower alkyl.

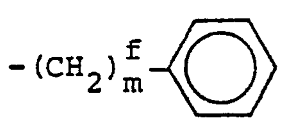, $-(CH_2)_m^f$-cycloalkyl,

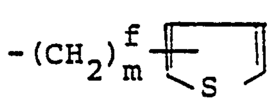, 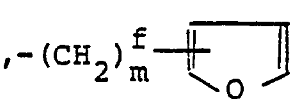,

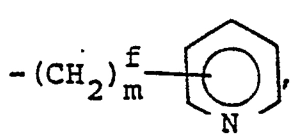, or 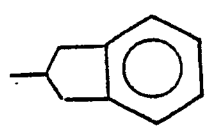,

$R_5^f$ is hydrogen, lower alkyl,

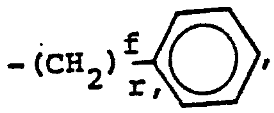 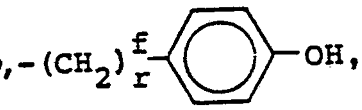

$-(CH_2)_r-OH$, 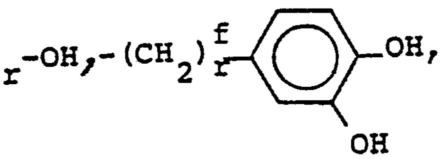

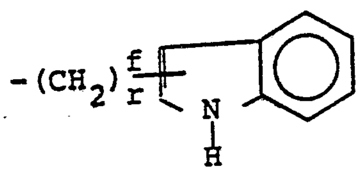, 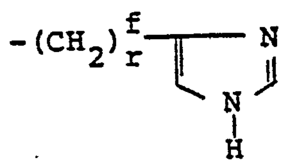,

$-(CH_2)_r^f-NH_2 - (CH_2)_r^f-SH, -(CH_2)_r^f-S$-lower alkyl,

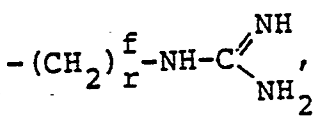, or 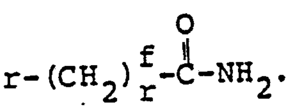.

$r^f$ is an integer from 1 to 4.
$R_{19}^f$ is lower alkyl, benzyl, or phenethyl.
$R_{20}^f$ is hydrogen, lower alkyl, benzyl or phenethyl.
$R^f$ is hydrogen, lower alkyl, cycloalkyl,

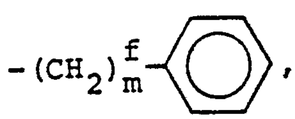,

$-(CH_2)_2-NH_2$, $-(CH_2)_3-NH_2$, $-(CH_2)_4-NH_2$,
$-(CH_2)_2-OH$, $-(CH_2)_3-OH$, $-(CH_2)_4-OH$,
$-(CH_2)_2-SH$, $-(CH_2)_3-SH$, or $-(CH_2)_4-SH$.

$R_1^f$ is hydrogen, lower alkyl, halo substituted lower alkyl,

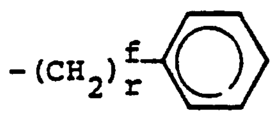, 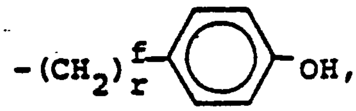

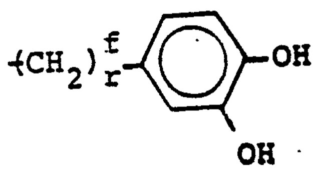, 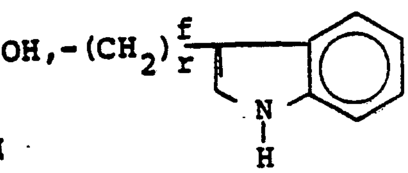

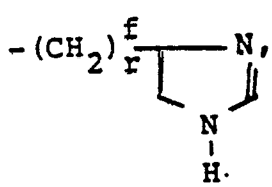, $-(CH_2)_r^f-NH_2$, $-(CH_2)_r^f-SH$,

$-(CH_2)_r^f-OH$, $-(CH_2)_r^f-S-$lower alkyl,

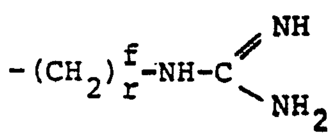 ,or 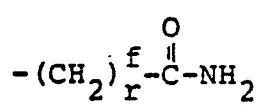

provided that $R_1^f$ is hydrogen only if $R^L$ is other than hydrogen.

$R_2^f$ is

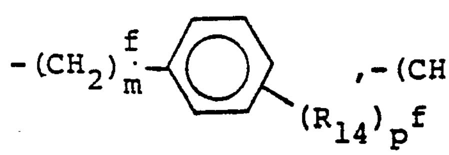, 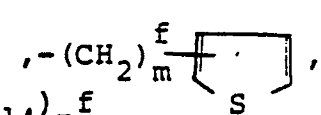,

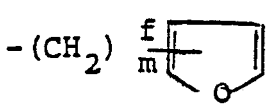,or 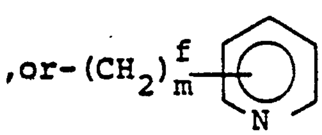

$R_3^f$ is hydrogen, lower alkyl

$-(CH_2)_m^f-$ 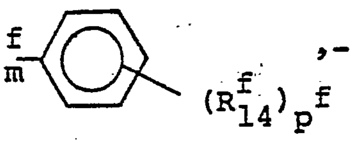 , $-(CH_2)_m^f-$ 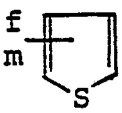 ,

$-(CH_2)_m^f-$ 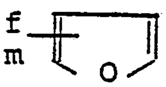 , $-(CH_2)_m^f-$ 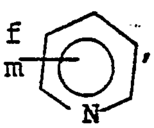 ,halo substituted

lower alkyl , $-(CH_2)_m^f-$cycloalkyl , $-(CH_2)_r^f-$ 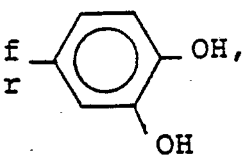

$-(CH_2)_r^f-$ 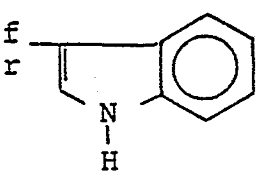 , $-(CH_2)_r^f-OH$,

$-(CH_2)_r^f-$ 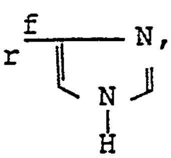 , $-(CH_2)_r^f-NH_2$, $-(CH_2)_r^f-SH$,

$-(CH_2)_r^f-S-$lower alkyl, $-(CH_2)_r^f-NH-$ 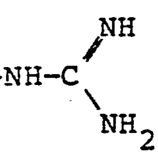 ,or $-(CH_2)_r^f-$ 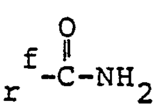

wherein $m^f$, $R_{14}^f$, $p^f$ and $r^f$ are as defined above.

$R_6^f$ is hydrogen, lower alkyl, benzyl, benzhydryl,

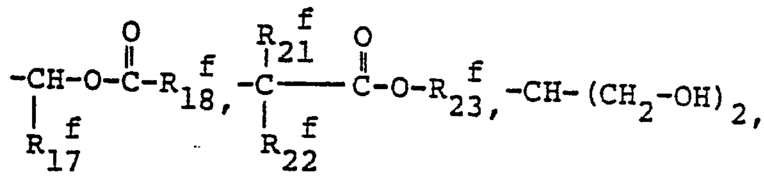

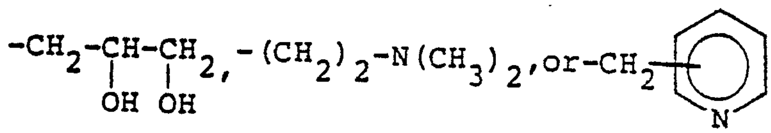

$R_{17}{}^f$ is hydrogen, lower alkyl, cycloalkyl, or phenyl.

$R_{18}{}^f$ is hydrogen, lower alkyl, lower alkoxy, or phenyl or $R_{17}{}^f$ and $R_{18}{}^f$ taken together are $-(CH_2)_2-$, $-(CH_2)_3-$, $-CH=CH-$, or

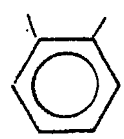 ,

$R_{21}{}^f$ and $R_{22}{}^f$ are independently selected from hydrogen and lower alkyl.

$R_{23}{}^f$ is lower alkyl.

$R_{24}{}^f$ is hydrogen, lower alkyl,

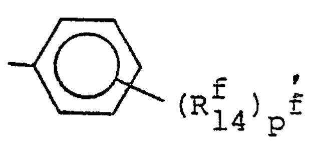

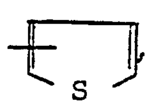 , 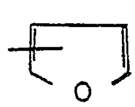 , or 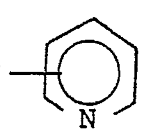

British Specification No. 2095682 published October 6, 1982 discloses N-substituted-N-carboxyalkyl aminocarbonyl alkyl glycine derivatives which are said to

be angiotensin converting enzyme inhibitors and have the formula

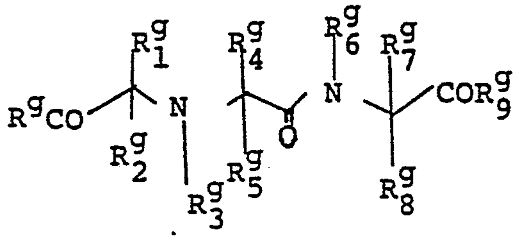

(I)

either

(A) $R^g$ and $R_9{}^g$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1{}^g$-$R_5{}^g$, $R_7{}^g$ and $R_8{}^g$ are 1-20C alkyl, 2-20C alkenyl, 2-20C alkynyl, aryl, aryl-(1-6C) alkyl having 7-12C or heterocyclyl-(1-6C)alkyl having 7-12C;

$R_6{}^g$ is cycloalkyl, polycycloalkyl, partly saturated cycloalkyl or polycycloalkyl, cycloalkyl-(1-6C)alkyl having 3-20C, 6-10C aryl, aryl-(1-6C)alkyl, aryl-(2-6C)alkenyl or aryl-(2-6C) alkynyl; or

$R_2{}^g$ and $R_3{}^g$ together with the C and N atoms to which they are attached or $R_3{}^g$ and $R_5{}^g$ together with the N and C atoms to which they are attached form an N-heterocycle containing 3-5C or 2-4C and a S atom;

all alkyl, alkenyl and alkynyl are optionally substituted by OH, 1-6C alkoxy, thio(sic), 1-6C alkylthio, $NH_2$, mono- or di(1-6C alkyl)amino, halogen or $NO_2$;

all 'cycloalkyl' groups (including poly and partially unsaturated) are optionally substituted by halogen, 1-6C hydroxyalkyl, 1-6C alkoxy, amino-(1-6C alkyl)amino, di-(1-6C alkyl)amino, SH, 1-6C alkylthio, $NO_2$ or $CF_3$; and aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^g$ and $R_9^g$ are H or 1-6C alkoxy;

$R_1^g$ and $R_2^g$ are H, 1-6C alkyl, aryl-(1-6C) alkyl having 7-12C or heterocyclyl-(1-6C) alkyl having 6-12C;

$R_3^g$-$R_5^g$, $R_7^g$ and $R_8^g$ are H or 1-6C alkyl;

$R_6^g$ is cycloalkyl, polycycloalkyl, partly saturated cycloalkyl or polycycloalkyl, cycloalkyl-(1-6C) alkyl having 3-20C, aryl or aryl-(1-6C) alkyl; and

aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino.

U.S. Patent No. 4,470,972 to Gold et al discloses 7-carboxyalkyl-aminoacyl-1,4-dithia-7-azaspiro[4.4]nonane-8-carboxylic acids which are angiotensin converting enzyme inhibitors and have the formula

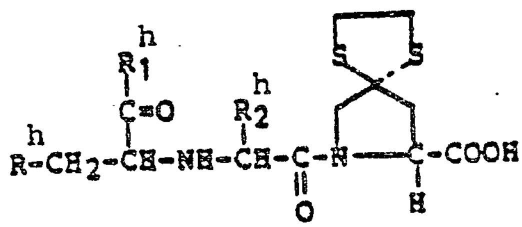

wherein:

$R^h$ is lower alkyl, benzyl, benzylthio, benzyloxy, phenylthio or phenoxy;

$R_1^h$ is hydroxy or lower alkoxy;

$R_2^h$ is hydrogen, lower alkyl or amino lower alkyl; and the pharmaceutcally acceptable salts thereof.

(i) European Patent Application 0 050 800 published May 5, 1982 discloses carboxyalkyl dipeptides derivatives which are said to be angiotensin converting enzyme inhibitors and have the formula

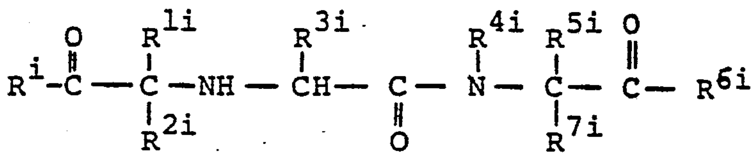

or a pharmaceutically acceptable salt thereof, wherein $R^i$ and $R^{6i}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substitutent is methyl, halo or methoxy; $R^{1i}$ is hydrogen, alkyl of from 1 to 10 carbon atoms, substituted lower alkyl wherein the substitutent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, quanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group

is substituted with a group selected from halo, lower alkyl, hydroxy, lower alkoxy, amino, aminomethyl, carboxyl, cyano, or sulfamoyl; $R^{2i}$ and $R^{7i}$ are the same or different and are hydrogen or lower alkyl; $R^{3i}$ is hydrogen, lower alkyl, phenyl lower alkyl, aminoethylphenyl lower alkyl, hydroxyphenyl lower alkyl, hydroxy lower alkyl, acylamino lower alkyl, amino lower alkyl, dimethylamino lower alkyl, guanidino lower alkyl, imidazolyl lower alkyl, indolyl lower alkyl, or lower alkyl thio lower alkyl; $R^{4i}$ and $R^{5i}$ are the same or different and are hydrogen, lower alkyl or $Z^i$, or $R^{4i}$ and $R^{5i}$ taken together form a group represented by $Q^i$, $U^i$, $V^i$, $Y^i$, $D^i$ or $E^i$, wherein;

$Z^i$ is

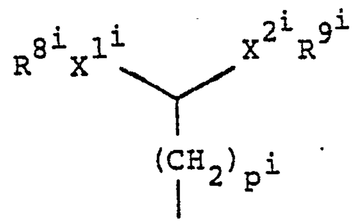

wherein $X^{1i}$ and $X^{2i}$ independent of each other are O, S or $CH_2$, $R^{8i}$ and $R^{9i}$ independent of each other are lower alkyl, lower alkenyl, lower alkynyl, cycloalkyl having 3 to 8 carbon atoms, hydroxy lower alkyl, or $-(CH_2)_{n^i}Ar^i$, wherein $n^i$ is 0, 1, 2 or 3 and $Ar^i$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8i}$ and $R^{9i}$ taken together form a bridge $W^i$, wherein $W^i$ is a single bond or a methylene bridge or a substituted methylene bridge when at least one of $X^{1i}$ and $X^{2i}$ is methylene, or $W^i$ is an alkylene or substituted alkylene bridge having 2 or 3 carbon atoms, said substituted methylene bridge or said substituted alkylene bridge having one or two

substituents selected from lower alkyl, aryl and aryl lower alkyl groups, and $p^i$ is 0, 1 or 2; with the proviso that at least one of $R^{4i}$ and $R^{5i}$ is $Z^i$, with the proviso that if $R^{4i}$ is $Z^i$ and $p^i$ is 0 then $X^{1i}$ and $X^{2i}$ must both be methylene, and with the proviso that if $X^{1i}$ and $X^{2i}$ are both methylene then $R^{8i}$ and $R^{9i}$ must form an alkylene bridge $W^i$;

$Q^i$ is

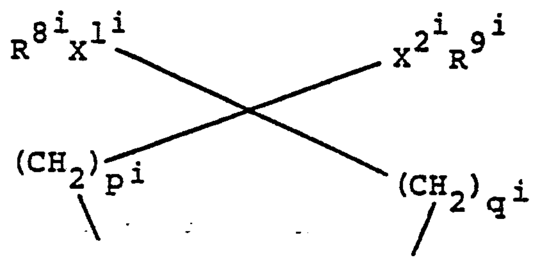

wherein $R^{8i}$, $R^{9i}$, $X^{1i}$ and $X^{2i}$ are as defined above, $p^i$ is 0, 1 or 2, $q^i$ is 0, 1 or 2, with the proviso that the sum of $p^i$ and $q^i$ must be 1, 2 or 3, with the proviso that if $p^i$ is 0 then $X^{1i}$ and $X^{2i}$ must be methylene, and with the proviso that if $X^{1i}$ and $X^{2i}$ are methylene then $R^{8i}$ and $R^{9i}$ taken together form a bridge $W^i$, wherein $W^i$ is as defined above;

$V^i$ is

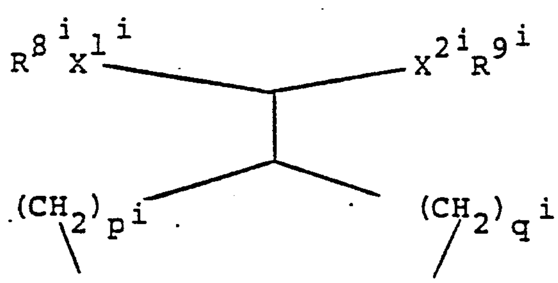

wherein $R^{8i}$, $R^{9i}$, $X^{1i}$ and $X^{2i}$ are as defined above, $p^i$ is 0, 1 or 2 and $q^i$ is 0, 1 or 2, with the proviso that the sum of $p^i$ and $q^i$ is 1, 2 or 3, with the proviso that if $X^{1i}$ and $X^{2i}$ are $CH_2$ then $R^{8i}$ and $R^{9i}$ taken together form a bridge $W^i$, wherein $W^i$ is as defined above;

$U^i$ is

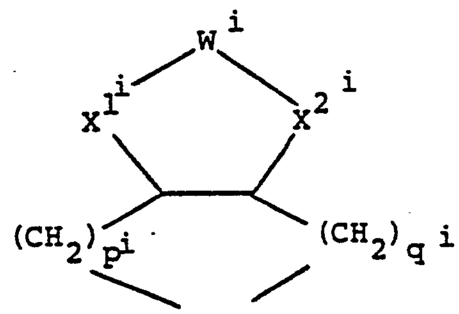

wherein $W^i$ is as defined above (except that $W^i$ may also be a methylene bridge when $X^{1i}$ and $X^{2i}$ are oxygen or sulfur), $X^{1i}$ and $X^{2i}$ are as defined above, $p^i$ is 0, 1 or 2, $q^i$ is 0, 1 or 2, with the proviso that the sum of $p^i$ and $q^i$ is 1 or 2, and with the proviso that if $p^i$ is 0, $X^{1i}$ must be $CH_2$;

$Y^i$ is

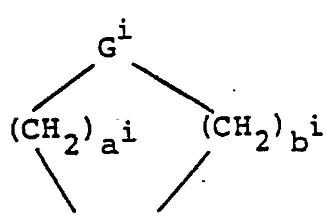

wherein $G^i$ is oxygen, sulfur or $CH_2$, $a^i$ is 2, 3 or 4 and b is 1, 2, 3, 4 or 5, with the proviso that the sum of $a^i$ and $b^i$ is 5, 6 or 7 or

$G^i$ is $CH_2$, $a^i$ is 0, 1, 2 or 3, $b^i$ is 0, 1, 2 or 3 with the proviso that the sum of $a^i$ and $b^i$ is 1, 2 or 3, with the proviso that the sum of $a^i$ and $b^i$ may be 1, 2 or 3 only if $R^{1i}$ is lower alkyl substituted with aralkylthio or aralkyloxy;

$D^i$ is

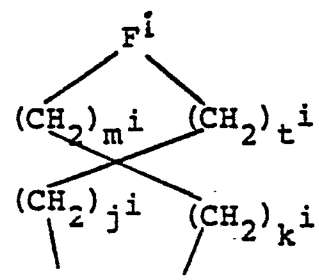

wherein $F^i$ is O or S, $j^i$ is 0, 1 or 2 and $k^i$ is 0, 1 or 2, with the proviso that the sum of $j^i$ and $k^i$ must be 1, 2 or 3, and $m^i$ is 1, 2 or 3 and $t^i$ is 1, 2 or 3, with the proviso that the sum of $m^i$ and $t^i$ must be 2, 3 or 4;
$E^i$ is

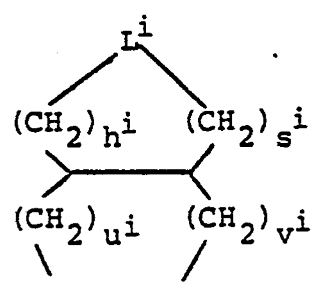

wherein $L^i$ is O or S, $u^i$ is 0, 1 or 2 and $v^i$ is 0, 1 or 2, with the proviso that the sum of $u^i$ and $v^i$ must be 1 or 2, and $h^i$ is 1 or 2 and $s^i$ is 1 or 2, with the proviso that the sum of $h^i$ and $s^i$ must be 2 or 3.

(j) European Patent Application 0 037 231 published October 7, 1981 discloses acyl derivatives of octahydro-1H-indole-2-carboxylic acids which are said to be angiotensin converting enzyme inhibitors and have the formula

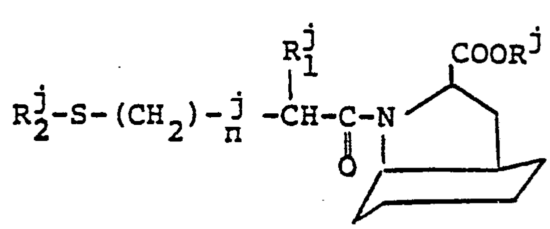

wherein $R^j$ is hydrogen or lower alkyl; $R_1^j$ is hydrogen, lower alkyl, or benzyl; $R_2^j$ is hydrogen or $R_3^j-\overset{O}{\overset{\|}{C}}-$wherein $R_3^j$ is lower alkyl, heteroaryl containing 4 to 9 carbon atoms and one or two nitrogen, oxygen or sulfur atoms; phenyl, substituted phenyl having 1 or 2 substituents selected from the group consisting of fluorine, chlorine, bromine, lower alkyl or alkoxy; and n is 0 or 1; wherein lower alkyl and lower alkoxy include straight or branched groups containing 1 to 4 carbon atoms, and pharmaceutically acceptable salts of the compounds when $R^j$ is hydrogen and when $R_3^j$ is heteroaryl containing 1 or 2 nitrogen atoms, are disclosed. Also disclosed are substituted acyl compounds of octahydro-1H-indole-2-carboxylic acid having the formula

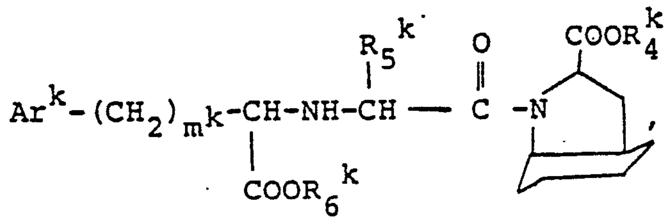

wherein $R_4^k$ is hydrogen or lower alkyl; $R_5^k$ is hydrogen, lower alkyl or benzyl; $R_6^k$ is hydrogen or lower alkyl; $Ar^k$ is phenyl or phenyl substituted with 1 or 2 substituents selected from the group consisting of fluorine, chlorine, bromine, lower alkyl, lower alkoxy, hydroxy or amino; and m is 0 to 3; wherein lower alkyl and lower alkoxy contain 1 to 4 straight or branched carbon atoms; and the pharmaceutically acceptable salts thereof.

(L) European Patent Application 0 079 522 published May 25, 1983 discloses N-carboxymethyl(amino) lysl-proline compounds which are said to be angiotensin converting enzyme inhibitors and have the formula where

wherein $R^j$ is hydrogen or lower alkyl; $R_1^j$ is hydrogen, lower alkyl, or benzyl; $R_2^j$ is hydrogen or $R_3^j-\overset{O}{\overset{\|}{C}}-$wherein $R_3^j$ is lower alkyl, heteroaryl containing 4 to 9 carbon atoms and one or two nitrogen, oxygen or sulfur atoms; phenyl, substituted phenyl having 1 or 2 substituents selected from the group consisting of fluorine, chlorine, bromine, lower alkyl or alkoxy; and n is 0 or 1; wherein lower alkyl and lower alkoxy include straight or branched groups containing 1 to 4 carbon atoms, and pharmaceutically acceptable salts of the compounds when $R^j$ is hydrogen and when $R_3^j$ is heteroaryl containing 1 or 2 nitrogen atoms, are disclosed. Also disclosed are substituted acyl compounds of octahydro-1H-indole-2-carboxylic acid having the formula

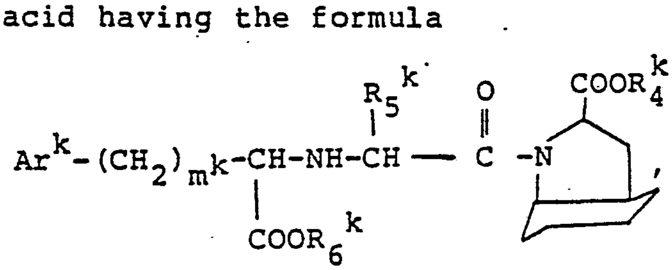

wherein $R_4^k$ is hydrogen or lower alkyl; $R_5^k$ is hydrogen, lower alkyl or benzyl; $R_6^k$ is hydrogen or lower alkyl; $Ar^k$ is phenyl or phenyl substituted with 1 or 2 substituents selected from the group consisting of fluorine, chlorine, bromine, lower alkyl, lower alkoxy, hydroxy or amino; and m is 0 to 3; wherein lower alkyl and lower alkoxy contain 1 to 4 straight or branched carbon atoms; and the pharmaceutically acceptable salts thereof.

(L) European Patent Application 0 079 522 published May 25, 1983 discloses N-carboxymethyl(amino) lysl-proline compounds which are said to be angiotensin converting enzyme inhibitors and have the formula where

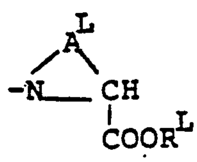 is 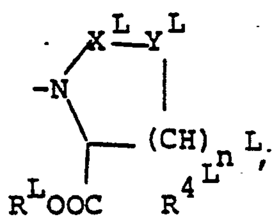, 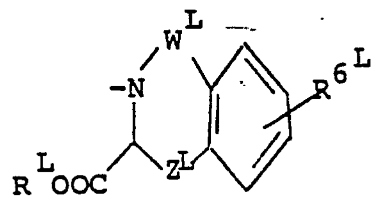

or 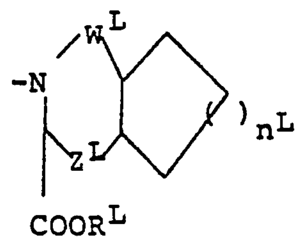

where:

$X^L$ and $Y^L$ taken together are $-CH_2-CH_2-$;

$-CH(R^{5L})-S-$; $-C(=O)-CH_2-$; $-CH_2-C(=O)-$; $-C(=O)-O-$; $-C(=O)-S-$;

$-CH_2-CH(OR^{4L})-$; $-C(=O)-N(R^{4L})-$; or $-CH_2-C(R^{4L})-R^{5L}$;

$R^{4L}$ is hydrogen; loweralkyl; aryl; substituted aryl;

$R^{5L}$ is hydrogen; loweralkyl; aryl or substituted aryl;

$n^L$ is 1 to 3;

$W^L$ is absent; $-CH_2-$; or $-C(=O)-$;

$Z^L$ is $-(CH_2)_m{}^L$, where m is 0 to 2, provided that $m^L$ may not be 0 and $W^L$ may not be absent at the same time; and

$R^{6L}$ is hydrogen; loweralkyl; halo; or $OR^{4L}$;

$R^{2L}$ is $-(CH_2)_{r^L}-B^L-(CH_2)_{s^L}-NR^{7L}R^{15L}$

where

$r^L$ and $s^L$ are independently 0 to 3;

$B^L$ is absent; -O-; -S-; or $-NR^{8L}$;

where $R^{8L}$ is hydrogen; loweralkyl; alkanoyl; or aroyl; and

$R^{7L}$ is 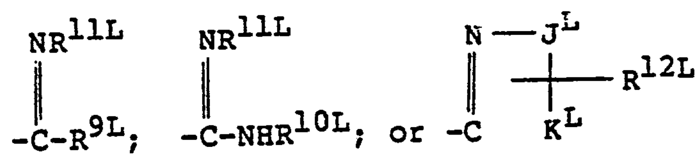

where

$R^{9L}$ is loweralkyl; aralkyl; aryl; heteroaryl; or heteroarloweralkyl and these groups substituted by hydroxy, lower alkoxy or halo; carboxyl; carboxamido; nitromethenyl.

$R^{10L}$ is hydrogen; loweralkyl; aryl; or amidino;

$R^{11L}$ hydrogen; loweralkyl; cyano; amidino; aryl; aroyl; loweralkanoyl; 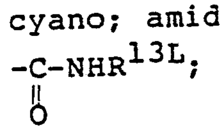;

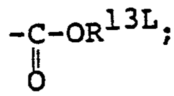; $-NO_2$; $-SO_2NH_2$;

or

$SO_2R^{13L}$;

$R^{12L}$ is hydrogen; loweralkyl; halo; aralkyl; amino; cyano; mono- or diloweralkylamino; or $OR^{4L}$;

$R^{13L}$ is hydrogen; loweralkyl; or aryl;

$R^{15L}$ is hydrogen; lower alkyl; aralkyl; or aryl.

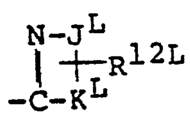

constitute a basic heterocycle of 5 or 6 atoms or benzofused analogs thereof and optionally containing 1-3 N atoms, an oxygen, a sulfur, an

S=O, or an $SO_2$ group optionally substituted by amino, lower alkyl amino, diloweralkyl amino, lower alkoxy, or aralkyl groups;

$R^{3L}$ is $C_{3-8}$ cycloalkyl and benzofused $C_{3-8}$ cycloalkyl; perhydrobenzofused $C_{3-8}$ cycloalkyl; aryl; substituted aryl; heteraryl; substituted heteroaryl;

$R^{14L}$ is hydrogen or loweralkyl; and, a pharmaceutically acceptable salt thereof.

(m) U.S. Patent No. 4,256,761 to Suh et al discloses amides which are angiotensin converting enzyme inhibitors and have the general formula

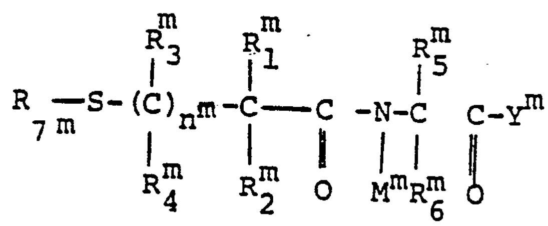

wherein

$R_1^m$, $R_2^m$, $R_3^m$, $R_4^m$, $R_5^m$ and $R_6^m$ are hydrogen, alkyl, alkenyl, alkynyl, phenyl-alkyl, and cycloalkyl, and may be the same or different,

$n^m$ is an integer from 0 to 4 inclusive,

$M^m$ is alkenyl, alkynyl, cycloalkyl, cycloalkyl-alkyl, polycycloalkyl, polycycloalkyl-alkyl, aryl, aralkyl, heteroaryl, heteroaryl-alkyl, hetero-cycloalkyl, hetero-cycloalkyl-alkyl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylamino-alkyl, or dialkylaminoalkyl,

$Y^m$ is hydroxy, alkoxy, amino, or substituted amino, aminoalkanoyl, aryloxy, aminoalkoxy, or hydroxyalkoxy, and

$R_7^m$ is hydrogen, alkanoyl, carboxyalkanoyl, hydroxyalkanoyl, aminoalkanoyl, cyano, amidino, carbalkoxy, $Z^mS$ or

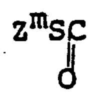

wherein $Z^m$ is hydrogen, alkyl, hydroxyalkyl, or the radical

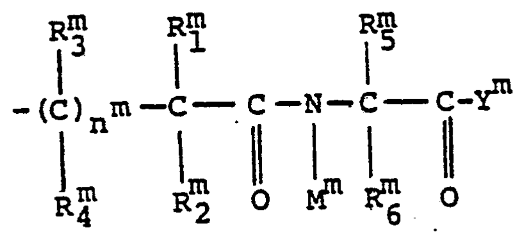

wherein $R_1^m$, $R_2^m$, $R_3^m$, $R_4^m$, $R_5^m$, $R_6^m$ $n^m$, $M^m$ and $Y^m$ are as described above; and where Y is hydroxy, their non-toxic, pharmaceutically acceptable alkali metal, alkaline earth metal, and amine salts.

(n) U.S. Patent No. 4,344,949 to Hoefle et al discloses acyl derivatives of 1,2,3,4-tetrahydroisoquinoline-3-carboxylic acid compounds which are angiotensin converting enzyme inhibitors and have the formula

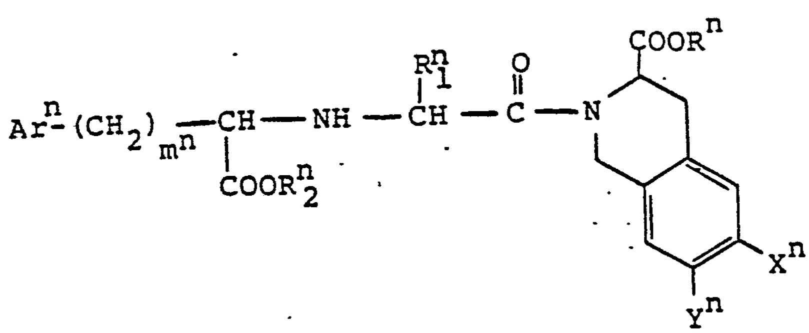

wherein $R^n$ is hydrogen, lower alkyl or aralkyl; $R_1{}^n$ is hydrogen, lower alkyl, or benzyl; $R_2{}^n$ is hydrogen or lower alkyl, and $Ar^n$ is phenyl or phenyl substituted with 1 or 2 substituents selected from the group consisting of fluorine, chlo-ine, bromine, lower alkyl, lower alkoxy, hydroxy or amino; $X^n$ and $Y^n$ are independently hydrogen, lower alkyl, lower alkoxy, lower alkylthio, lower alkylsulfinyl, lower alkylsulfonyl, hydroxy, or $X^n$ and $Y^n$ together are methylenedioxy; $m^n$ is 0 to 3; and the pharmaceutically acceptable acid salts thereof.

(o) European Patent 79022 published May 18, 1983 discloses N-amino acyl-azabicyclooctane carboxylic acid derivatives which have the formula

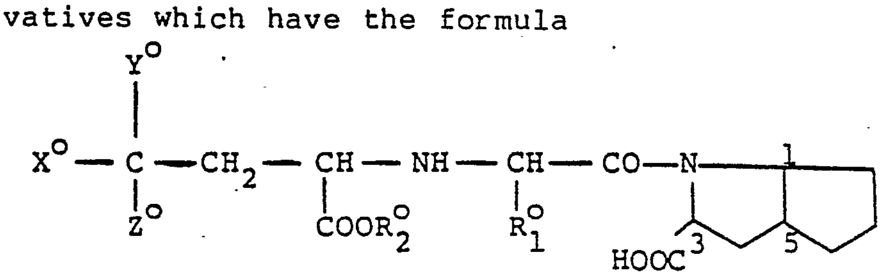

hydrogen atoms at ring positions 1 and 5 are cis to each other and the 3-carboxy group has the endo orientation;

$R_1{}^o$ is H, allyl, vinyl or the side chain of an optionally protected naturally occurring α-amino acid;

$R_2{}^o$ is H, 1-6C alkyl, 2-6C alkenyl or aryl(1-4C alkyl);

$Y^o$ is H or OH and $Z^o$ is H, or $Y^o$ and $Z^o$ together oxygen;

$X^o$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C

(p) European Patent 46953 published March 10, 1982 discloses N-amino acyl-indoline and tetrahydro isoquinoline carboxylic acids which are angiotensin coverting enzyme inhibitors and have the formula

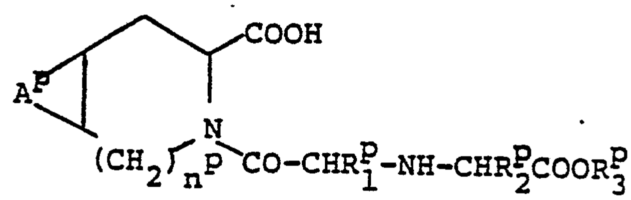

$n^p$ is 0 or 1;

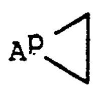 is a benzene or cyclohexane ring:

$R_1{}^p$ and $R_2{}^p$ are each 1-6C alkyl, 2-6C alkenyl, 5-7C cycloalkyl, 5-7C cycloalkenyl, 7-12C cycloalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^p$ and $R_2{}^p$ groups are optionally substituted,
$R_3{}^p$ is H, 1-6C alkyl, 2-6C alkenyl or 7-14C aralkyl.

(q) U.S. Patent No. 4,508,729 to Vincent et àl. discloses substituted imino-acids which are angiotensin converting enzyme inhibitors and have the formula

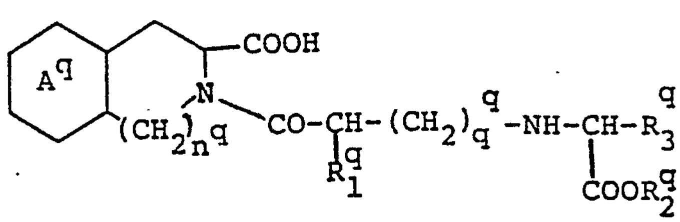

wherein:

the ring $A^q$ is saturated and $n^q$=0 or 1, or the ring $A^q$ is a benzene ring and $n^q$=1,

$R_1{}^q$ represents a lower alkyl group having from 1 to 4 carbon atoms which can carry an amino group,

$R_2{}^q$ represents a hydrogen atom or a alkyl group having from 1 to 4 carbon atoms,

$R_3{}^q$ represents a straight or branched alkyl group, a mono- or dicycloalkylalkyl or phenylalkyl group having no more than a total of 9 carbon atoms, or a substituted alkyl group of the formula:

$$-(CH_2)_p{}^q-Y^q-\underset{R_4{}^q}{CH}-R_5{}^q$$

with $R_4{}^q$=H, a lower alkyl ($C_1$ to $C_4$) or a cycloalkyl ($C_3$ to $C_6$ group,

$R_5{}^q$=H, a lower alkyl ($C_1$ to $C_4$) a cycloalkyl ($C_3$ to $C_6$) or an alkoxycarbonyl group,

$Y^q$=S or >N-$Q^q$ where $Q^q$=H, or an acetyl or benzyloxycarbonyl group, and

$p^q$=1 or 2, and

$q^q$=0 or 1, and the pharmaceutically acceptable salts thereof.

(r) U.S. Patent 4,512,924 to Attwood et al. discloses bicyclic compounds which are angiotensin converting enzyme inhibitors and have the formula

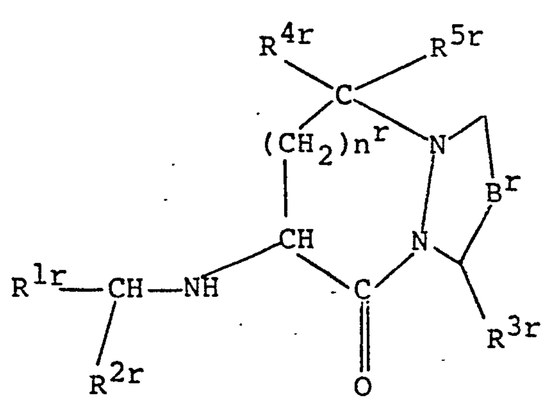

wherein $B^r$ represents a methylene ($-CH_2-$), ethylene ($-CH_2-CH_2-$) or vinylene ($-CH=CH-$) group, $R^{1r}$ represents a hydrogen atom or an alkyl, aralkyl, amino-alkyl, monoalkylamino-alkyl, dialkylamino-alkyl, acylamino-alkyl, phthalimido-alkyl, alkoxycarbonylamino-alkyl, aryloxycarbonyl-amino-alkyl, aralkoxycarbonyl-amino-alkyl, alkylaminocarbonylamino-alkyl, arylaminocarbonylamino-alkyl, aralkylaminocarbonylamino-alkyl, alkylsulphonylamino-alkyl or arylsulphonylamino-alkyl group, $R^{2r}$ represents a carboxyl, alkoxycarbonyl or aralkoxycarbonyl group or a group of the formula

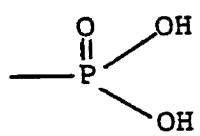 or 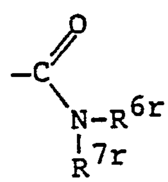

$R^{3r}$ represents a carboxyl, alkoxycarbonyl or aralkoxycarbonyl group, $R^{4r}$ and $R^{5r}$ each represent a hydrogen atom or $R^{4r}$ and $R^{5r}$ together represent an oxo group, $R^{6r}$ and $R^{7r}$ each represent a hydrogen atom or an alkyl or aralkyl group or $R^{6r}$ and $R^{7r}$ together with the nitrogen atom to which they are attached represent a saturated 5 membered or 6-membered heteromonocyclic ring which may contain a further nitrogen atom or an oxygen or sulphur atom, and $n^r$ stands for zero, 1 or 2, and pharmaceutically acceptable salts thereof.

(s) U.S. Patent 4,410,520 to Watthey discloses 3-amino-[1]benzazepin-2-one-1-alkanoic acids which are angiotensin converting enzyme inhibitors and have the formula

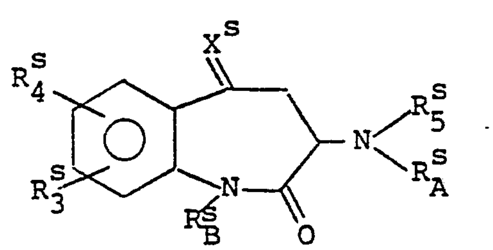

wherein

$R_A{}^s$ and $R_B{}^s$ are radicals of the formula

0254032

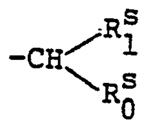

and

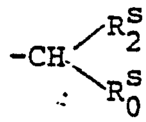

respectively in which

$R_0^s$ is carboxy or a functionally modified carboxy;

$R_1^s$ is hydrogen, lower alkyl, amino(lower) alkyl, aryl, aryl (lower) alkyl, cycloalkyl or cycloalkyl (lower) alkyl;

$R_2^s$ is hydrogen or lower alkyl;

$R_3^s$ and $R_4^s$, each independently, represent hydrogen, lower alkyl, lower alkoxy, lower alkanoyloxy, hydroxy, halogen, trifluoromethyl, or

$R_3^s$ and $R_4^s$ taken together represent lower alkylenedioxy;

$R_5^s$ is hydrogen or lower alkyl, and

$X^s$ represents oxo, two hydrogens, or one hydroxy together with one hydrogen; and wherein the carbocyclic ring may also be hexahydro or 6, 7, 8, 9-tetrahydro, and salts and complexes thereof.

(t) U.S. Patent 4,374,847 to Gruenfeld discloses 1-carboxy-(alkanoyl or aralkanoyl)-indolene-2-carboxylic acids which are angiotensin converting enzyme inhibitors and have the formula

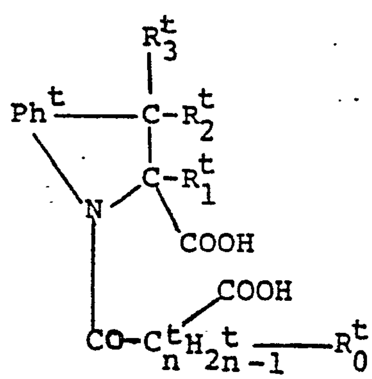

wherein $Ph^t$ is unsubstitued 1,2-phenylene, or 1,2-phenylene substituted by one to three identical or different members selected from lower alkyl, lower alkoxy, lower alkylenedioxy, hydroxy, halogeno and trifluoromethyl; $R_0{}^t$ is hydrogen or $HPh^t$; each of $R_1{}^t$, $R_2{}^t$ and $R_3{}^t$ is hydrogen or lower alkyl; and $n^t$ is an integer from 1 to 10; the amides, mono- or di-lower alkylamides, lower alkyl esters, (amino, mono- or di-lower alkylamino, carboxy or lower carbalkoxy)-lower alkyl esters, or pharmaceutically acceptable salts thereof.

The following Table 1 lists ACE inhibitors preferred for use in the invention.

TABLE I

PREFERRED ACE INHIBITORS

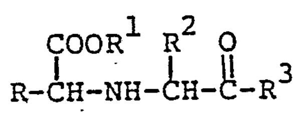

| | R | $R_1$ | $R_2$ | $R_3$ |
|---|---|---|---|---|
| spirapril | $C_6H_5CH_2CH_2-$ | Et | $CH_3$ | 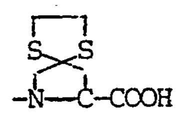 |
| enalapril | $C_6H_5CH_2CH_2-$ | Et | $CH_3$ | prolyl |
| ramipril | $C_6H_5CH_2CH_2-$ | Et | $CH_3$ | 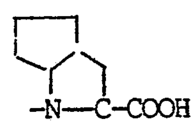 |
| perindopril | $CH_3CH_2CH_2$ | Et | $CH_3$ | 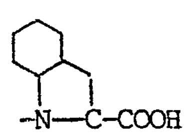 |

| | | | | |
|---|---|---|---|---|
| indolapril | $C_6H_5CH_2CH_2-$ | Et | $CH_3$ | 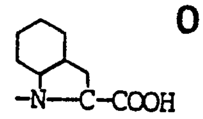 |
| lysinopril | $C_6H_5CH_2CH_2-$ | H | $NH_2(CH_2)_4-$ | prolyl |
| CI-925 | $C_6H_5CH_2CH_2-$ | Et | $CH_3$ | 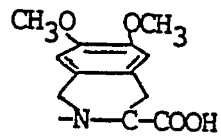 |
| pentopril (NH = $CH_2$) | $CH_3$ | Et | $CH_3$ | 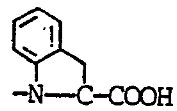 |
| cilazapril | $C_6H_5CH_2CH_2-$ | H | 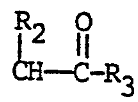 = | 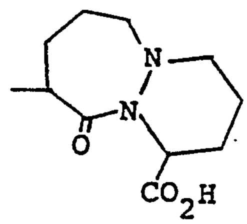 |

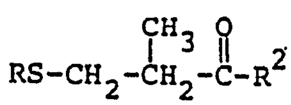

| | R | $R_2$ |
|---|---|---|
| captopril | H | prolyl |
| zofenopril | $C_6H_5CO-$ | 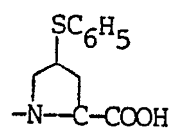 |
| pivalopril | H | 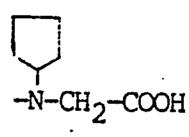 |

As indicated by Needleman et al., a number of atrial peptides have been isolated so far, all having the same core sequence of 17 amino acids within a cysteine disulfide bridge, but having different N-termini lengths. These peptides represent N-terminal truncated fragments (21-48 amino acids) of a common preprohormone (151 and 152 amino acids for man and rats, respectively). Human, porcine and bovine carboxy-terminal 28-amino acid peptides are identical and differ from similar peptides in rats and mice in that the former contain a methionine group at position 12 while the latter contain isoleucine. Various synthetic analogs of naturally occurring atrial peptides also have been found to have comparable biological activity. Examples of atrial peptides contemplated for use in this invention are α human AP 21 (atriopeptin I), α human AP 28, α human AP 23 (atriopeptin II or APII), α human AP 24, α human AP 25, α human AP 26, α human AP 33, and the corresponding rat sequence of each of the above wherein met 12 is ile. See Table 2 for a comparison of the peptides.

TABLE 2

| PEPTIDE | |
|---|---|
| AP 33 | Leu Ala Gly Pro Arg Ser Leu Arg Arg Ser Ser Cys-S Phe Gly Gly Arg Met Asp Arg Ile Gly Ala Gln Ser Gly Leu Gly Cys-S Asn Ser Phe Arg Tyr |
| AP 28 | Ser ——— Tyr |
| AP 26 | Arg ——— Tyr |
| AP 25 | Arg ——— Tyr |
| AP 24 | Ser ——— Tyr |
| AP 23 | Ser ——— Arg |
| AP 21 | Ser ——— Ser |

The antihypertensive effect of NMEP inhibitors, alone and in combination with ACE inhibitors was determined according to the following procedure:

Male Sprague Dawley rats weighing 100-150 g were anesthetized with ether and the right kidney was removed. Three pellets containing Doc acetate (desoxycorticosterone acetate, DOCA, 25 mg/pellet) were implanted subcutaneously. Animals recovered from surgery, were maintained on normal rat chow and were allowed free access to a fluid of 1% NaCl and 0.2% KCl instead of tap water for a period of 17-30 days. This procedure results in a sustained elevation in blood pressure and is a slight modification of published procedures (e.g. Brock et al., 1982) that have been used to produce DOCA salt hypertension in the rat.

On the day of study, animals were again anesthetized with ether and the caudal or carotid artery was cannulated for blood pressure measurement. Patency of the caudal artery cannula was maintained with a continuous infusion of dextrose in water at a rate of 0.2 ml/hr. Animals were placed into restraining cages where they recovered consciousness. Blood pressure was measured from caudal artery catheter using a Statham pressure transducer attached to a Beckman oscillographic recorder. In addition, a cardiovascular monitoring device (Buxco Electronics, Inc.) and a digital computer were used to calculate average blood pressures.

After an equilibration period of at least 1.5 hr., animals were dosed subcutaneously (1 ml/kg) with vehicle (methylcellulose, hereinafter MC), NMEP inhibitor, ACE inhibitor, or the combination of NMEP inhibitor and ACE inhibitor and blood pressure was monitored for the next 4 hours. The doses of NMEP inhibitor and ACE inhibitor are chosen based on amounts previously determined to be effective for inhibition of NMEP and ACE, respectively.

The antihypertensive effect of NMEP inhibitors in combination with atrial peptides was determined according to the following procedures:

Male spontaneously hypertensive rats (SHR), 16-18 weeks old, 270-350 g, were anesthetized with ether and the abdominal aorta was cannulated through the tail artery. The animals were then placed into restrainers to recover from anesthesia (in less than 10 min.) and remained inside throughout the experiments. Through a pressure transducer (Gould P23 series) analog blood pressure signals were registered on a Beckman 612 recorder. A Buxco digital computer was used to obtain mean arterial pressures. Patency of the arterial cannula was maintained with a continuous infusion of 5% dextrose at 0.2 ml/hr. Animals were allowed a 90-min equilibration period. The animals first underwent a challenge with an atrial peptide such as AP23 or AP28 30 µg/kg iv and at the end of 60 min. were treated with MC vehicle or various enzyme inhibitors subcutaneously. A second atrial peptide challenge was administered 15 min. later and blood pressure was monitored for the next 90 min.

The antihypertensive effect in SHR of NMEP inhibitors and ACE inhibitors, alone and in combination, was determined as follows:

Animals were prepared for blood pressure measurement as described above. After stabilization, animals were dosed subcutaneously or orally with test drugs or placebo and blood pressure was monitored for the next 4 hr.

The daily antihypertensive dose of the compound or combinations of this invention is as follows: for NMEP inhibitor alone the typical dosage is 1 to 100 mg/kg of mammalian weight per day administered in single or divided dosages; for the combination of NMEP inhibitors and ACE inhibitors, the typical dosage is 1 to 100 mg of NMEP inhibitor/kg of mammalian weight per day, in single or divided dosages, plus 0.1 to 30 mg ACE inhibitor/kg of mammalian weight per day in single or divided dosages; for

the combination of NMEP inhibitor and atrial peptide, the typical dosage is 1 to 100 mg of NEMP inhibitor/kg mammalian weight per day in single or divided dosages plus 0.001 to 0.1 mg atrial peptide/kg of mammalian weight per day, in single or divided doses. The exact dose of any component or combination to be administered is determined by the attending clinician and is dependent on the potency of the compound administered, the age, weight, condition and response of the patient.

The following tables show the antihypertensive activity of NMEP inhibitors alone (Table 3), in combination with an ACE inhibitor (Table 4), and in combination with an atrial peptide (Table 5).

TABLE 3

POTENTIATION OF ANF AND REDUCTION IN DOCA SALT-INDUCED HYPERTENSION WITH NMEP INHIBITORS

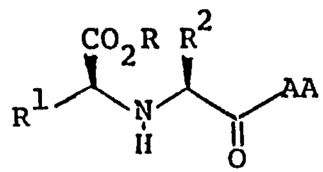

| AA | R | $R^1$ | $R^2$ | Decrease in Blood Pressure by ANF potentiation at 30 mg NMEP inhibitor/kg in SHR (mmHg) | Decrease in Blood Pressure of DOCA Salt Hypertensive Rats at 30 mg NMEP inhibitor/kg (mmHg) |
|---|---|---|---|---|---|
| $NH(CH_2)_2CO_2H$ | H | benzyl | benzyl | 20 | — |
| $NH(CH_2)_2CO_2H$ | H | phenethyl | benzyl | 17 | — |
| $NH(CH_2)_2CO_2H$ | glycerol ketal | benzyl | benzyl | 23 | — |
| $NHCH_2CH(OH)CO_2H$ | H | benzyl | benzyl | 17 | — |
| Trp | H | phenethyl | $CH_3$ | 11 | — |
| Pro | H | phenethyl | benzyl | 18 | 41 |
| OH<br>$NHCH_2CHCO_2H$ | H | phenethyl | benzyl | 23 | 33 |
| $NHCH(CH_2OH)CO_2H$ | H | phenethyl | benzyl | 15 | — |
| $NHCH(CH_2OCH_2C_6H_5)CO_2H$ | H | phenethyl | benzyl | 18 | 25 |
| OH<br>$NHCH_2CHCO_2H$ | $-CH_2CH_3$ | phenethyl | benzyl | 27 | — |

0254032

TABLE 3, cont'd

| AA | R | $R^1$ | $R^2$ | Decrease in Blood Pressure by ANF potentiation at 30 mg NMEP inhibitor/kg in SHR (mmHg) | Decrease in Blood Pressure of DOCA Salt Hypertensive Rats at 30 mg NMEP inhibitor/kg (mmHg) |
|---|---|---|---|---|---|
| OH<br>$NHCH_2CHCO_2H$ | H | phenethyl | benzyl | 28 | 31 |
| $NH_2$<br>$NHCH_2CHCO_2H$ | H | phenethyl | benzyl | 15 | — |
| OH<br>$NHCH_2CHCO_2H$ | $C_6H_5O(CH_2)_2$ | phenethyl | benzyl | 16 | 27 at 10mg/kg |

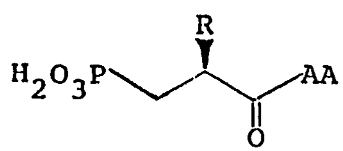

| AA | R | | | | |
|---|---|---|---|---|---|
| $NH(CH_2)_2CO_2H$ | benzyl | | | 27 at 90 mg/kg | — |
| $NH(CH_2)_2CO_2CH_2CH_3$ | benzyl | | | 13 | — |
| $NHCH(CH_2CO_2H)CO_2H$ | benzyl | | | 14 | — |
| $NHCH_2C_6H_4CO_2H$ | benzyl | | | 20 | — |
| Lys | benzyl | | | 15 | — |

TABLE 3, cont'd

| AA | R | | |
|---|---|---|---|
| Arg | benzyl | 12 | — |
| Arg-Tyr | benzyl | 17 | — |
| $NH(CH_2)_2CO_2H$ | $(CH_2)_4NH_3$ | 15 | — |
| $NHCH_2CH(CH_2C_6H_4)CO_2H$ | benzyl | 27 | — |
| Lys-OMe | benzyl | 17 | — |
| $NHCH(CH_2CH_2SO_2CH_3)CO_2H$ | benzyl | 17 | — |

| | | |
|---|---|---|
| N-[2-(S-acetylthiomethyl)-3-phenylpropionyl]-(S)-methionine amide | — | 47 at 3mg/kg |

TABLE 4

EFFECT OF A COMBINATION OF AN NMEP INHIBITOR[1] AND ACE INHIBITOR[2] ON BLOOD PRESSURE IN SHR

| | | CHANGE IN BLOOD PRESSURE (mmHg) | | | |
|---|---|---|---|---|---|
| | | 1 hr | 2 hr | 3 hr | 4 hr |
| A) | methylcellulose vehicle | +7 | +14 | +17 | +17 |
| | NMEP inhibitor (90 mg/kg) | +14 | +11 | +11 | +8 |
| | ACE inhibitor (10 mg/kg) | -11[3] | -7 | -1 | -1 |
| | NMEP inhibitor (90 mg/kg) + ACE inhibitor (10 mg/kg) | -14[3] | -19[3,4] | -19[3,4] | -15[3] |
| B) | methylcellulose vehicle | +6 | +9 | +9 | +8 |
| | NMEP inhibitor (90 mg/kg) | +5 | -2[3] | -9[3] | -11[3] |
| | ACE inhibitor (1 mg/kg) | -10[3] | -17[3] | -11[3] | -13[3] |
| | NMEP inhibitor (90 mg/kg) + ACE inhibitor (1 mg/kg) | 0 | -15[3] | -18[3,4] | -19[3] |
| C) | methylcellulose vehicle | +2 | +5 | +7 | +6 |
| | NMEP inhibitor (30 mg/kg) | -9[3] | -4 | -3 | -4 |
| | ACE inhibitor (30 mg/kg) | -17[3] | -18[3] | -14[3] | -12[3] |
| | NMEP inhibitor (30 mg/kg) + ACE inhibitor (30 mg/kg) | -21[3] | -26[3] | -30[3,4] | -22[3] |

1 A,B = N-[N-[L-1-(2,2-dimethyl-1-oxopropoxy)methoxy]carbonyl]-2-phenylethyl]-L-phenylalanine]-β-alanine

C = N-[2-(S-acetylthiomethyl)-3-phenylpropionyl]-(S)-methionine amide

2 A,C = 1-[(2S)-3-mercapto-2-methyl-1-oxypropyl]-L-proline (captopril)

B = 1-[N-[1-(ethoxycarbonyl-3-phenylpropyl]-L-alanyl]-L-proline (enalapril)

3 P <0.05 vs. vehicle

4 P <0.05 vs. ACE inhibitor alone

Drugs in A and B were administered subcutaneously; drugs in C were administered orally.

TABLE 5

HYPOTENSIVE RESPONSE TO AN ATRIAL PEPTIDE[1] IN SHR PRETREATED WITH AN NMEP INHIBITOR[2]

| | CHANGE IN BLOOD PRESSURE (mmHg) | | | |
|---|---|---|---|---|
| | 5 min. | 15 min. | 30 min. | 60 min. |
| methylcellulose vehicle[3] | -15 | -9 | -6 | -2 |
| NMEP inhibitor[3] | -24 | -29[4] | -29[4] | -21[4] |

1 AP 28 (30 μg/kg iv)

2 N-[1-oxo-3-phenyl-2-(phosphonomethyl)propyl]-β-alanine ethyl ester (90 mg/kg sc)

3 SHR were treated first with vehicle (as a control) or the NMEP inhibitor, then with the atrial peptide.

4 $p < 0.05$ vs. vehicle

Generally, in treating humans having hypertension, the compounds or combinations of this invention may be administered to patients in a dosage range as follows: for treatment with NMEP inhibitors alone, about 10 to about 500 mg per dose given 1 to 4 times a day, giving a total daily dose of about 10 to 2000 mg per day; for the combination of NMEP inhibitor and ACE inhibitor, about 10 to about 500 mg NMEP inhibitor per dose given 1 to 4 times a day and about 5 to about 50 mg ACE inhibitor given 1 to 3 times a day (total daily dosage range of 10 to 2000 mg/day and 5 to 150 mg per day, respectively); for the combination of NMEP inhibitor and atrial peptide, about 10 to about 500 mg NMEP inhibitor per dose given 1 to 4 times a day and about 0.001 to about 1 mg atrial peptide given 1 to 6 times a day (total daily dosage range of 10 to 2000 mg day and .001 to 6 mg/day, respectively). Where the components of a combination are administered separately, the number of doses of each

component given per day may not necessarily be the same, e.g. where one component may have a greater duration of activity, and will therefore need to be administered less frequently.

Typical oral formulations include tablets, capsules, syrups, elixirs and suspensions. Typical injectable formulations include solutions and suspensions. Typical pharmaceutically acceptable carriers may be used in said formulations.

Examples of formulations follow. "NMEP inhibitor" refers to any antihypertensive NMEP inhibitor, especially the preferred compounds named on pages 6, 9 and 11. "ACE inhibitor" refers to any of the ACE inhibitors listed on pages 11-55, especially those listed in the table of preferred ACE inhibitors. "Atrial peptide" refers to any antihypertensive atrial peptide, especially those listed on page 56.

EXAMPLE 1

Tablets

Formula

| No. | Ingredient | mg/tablet | mg/tablet |
|---|---|---|---|
| 1 | NMEP inhibitor | 50 | 400 |
| 2 | Lactose | 122 | 213 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Approximate Tablet Weight | 250 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4") if needed. Dry the damp granules. Screen the dried

granules if needed and mix with Item No. 4 and mix for 10-15 minutes. Add Item No. 5 and mix for 1-3 minutes. Compress the mixture to appropriate size and weight on a suitable tablet machine.

## EXAMPLE 2

NMEP inhibitor Injection: (per vial)

| | g/vial | g/vial |
|---|---|---|
| NMEP inhibitor Sterile Powder | 0.5 | 1.0 |

Add sterile water for injection or bacteriostatic water for injection for reconstitution.

## EXAMPLE 3

NMEP inhibitor Injectable Solution:

| Ingredient | mg/ml | mg/ml |
|---|---|---|
| NMEP inhibitor | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

Dissolve parabens in a portion ( 85% of the final volume) of the water for injection at 65-70°C. Cool to 25-35°C. Charge and dissolve the sodium bisulfite, disodium edetate and sodium sulfate. Charge and dissolve the NMEP inhibitor. Bring the solution to final volume by adding water for injection. Filter the solution through 0.22μ membrane and fill into appropriate containers. Terminally sterilize the units by autoclaving.

## EXAMPLE 4

NMEP inhibitor Injection: (per vial)

| | g/vial |
|---|---|
| NMEP inhibitor | 1.0 |
| Sodium Citrate | 0.05 |

pH is adjusted to 6.2 using 0.1N citric acid solution.

Add sterile water for injection or bacteriostatic water for injection for reconstitution.

## EXAMPLE 5

Tablets

| Formula No. | Ingredient | mg/tablet | mg/tablet |
|---|---|---|---|
| 1 | NMEP inhibitor | 50 | 400 |
| 2 | ACE inhibitor | 25 | 50 |
| 3 | Lactose | 97 | 188 |
| 4 | Corn Starch, Food Grade as a 10% Paste in Purified Water | 30 | 40 |
| 5 | Corn Starch, Food Grade | 45 | 40 |
| 6 | Magnesium Stearate | 3 | 7 |
| | Approximate Tablet Weight | 250 | 700 |

Method of Manufacturing

Prepare the tablets as in Example 1.

## EXAMPLE 6

Tablets

| Formula No. | Ingredient | mg/tablet |
|---|---|---|
| 1 | ACE inhibitor | 50 |
| 2 | Lactose | 122 |
| 3 | Corn Starch, Food Grade, as a 10% past in Purified Water | 30 |
| 4 | Corn Starch, Food Grade | 45 |
| 5 | Magnesium Stearate | 3 |
| | Approximate Tablet Weight | 250 |

Method of Manufacturing

Prepare the tablets as in Example 1.

EXAMPLE 7

Tablets

Formula

| No. | Ingredient | mg/tablet | mg/tablet |
|---|---|---|---|
| 1 | NMEP inhibitor | 50 | 400 |
| 2 | Atrial Peptide | 0.1 | 1 |
| 3 | Lastose | 121.9 | 212 |
| 4 | Corn Starch, Food Grade as a 10% Paste in Purified Water | 30 | 40 |
| 5 | Corn Starch, Food Grade | 45 | 40 |
| 6 | Magnesium Stearate | 3 | 7 |
| | | 250 | 700 |

Method of Manufacturing

Prepare the tablets as in Example 1.

EXAMPLE 8

Tablets

Formula

| No. | Ingredient | mg/tablet | mg/tablet |
|---|---|---|---|
| 1 | Atrial Peptide | 0.1 | 1 |
| 2 | Lactose | 61 | 121 |
| 3 | Corn Starch, as a 10% Paste in Purified Water | 15 | 30 |
| 4 | Corn Starch | 22.4 | 45 |
| 5 | Magnesium Stearate | 1.5 | 3 |
| | | 100 | 200 |

Method of Manufacture

Prepare tablets as in Example 1.

The above descriptions on pages 4-55 inclusive, of suitable classes of NMEP inhibitors and ACE inhibitors for use in the present invention were taken from the noted patents and publications or abstracts thereof. Reference should be made to such patents and publications themselves for their full disclosures of such classes and

specific compounds within such classes, such patents and publications being incorporated herein by reference for such purposes, and as to any typographical errors or the like which may have occurred in transcription. Also, in describing such suitable NMEP inhibitors and ACE inhibitors the superscript letters a-w were included to distinguish among the various classes of compounds and the variable substituent groups thereof.

CLAIMS

1. The use of a neutral metalloendopeptidase (NMEP) inhibitor for the preparation of pharmaceutical compositions useful in the treatment of hypertension wherein the NMEP inhibitor may be used alone or in combination or association with an atrial peptide or an angiotensin converting enzyme (ACE) inhibitor.

2. A pharmaceutical composition according to claim 1 comprising an antihypertensive amount of an NMEP inhibitor.

3. A pharmaceutical composition according to claim 1 comprising an antihypertensive amount of a combination of an NMEP inhibitor and an atrial peptide.

4. A pharmaceutical composition according to claim 1 comprising an antihypertensive amount of a combination of an NMEP inhibitor and an ACE inhibitor.

5. A composition according to claims 1 or 4 wherein the ACE inhibitor is chosen from ACE inhibitors having the formula

(a)

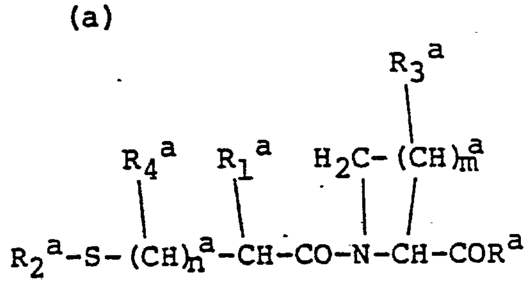

wherein

$R^a$ is hydroxy, $NH_2$ or lower alkoxy;
$R_1^a$ and $R_4^a$ each is hydrogen, lower alkyl, phenyl or phenyllower alkyl;
$R_2^a$ is hydrogen, lower alkyl, phenyl, substituted phenyl wherein the phenyl

substituent is halo, lower alkyl or lower alkoxy, phenyl-lower alkyl, diphenyl-lower alkyl, triphenyl-lower alkyl, lower alkylthiomethyl, phenyl-lower alkylthiomethyl, lower alkanoyl-amidomethyl,

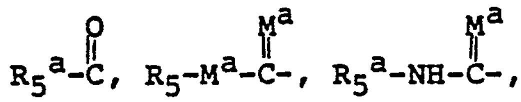

$R_6^a$-S-, or $R_7^a$;
$R_3^a$ is hydrogen, hydroxy or lower alkyl;
$R_5^a$ is lower alkyl, phenyl or phenyl-lower alkyl;
$R_6^a$ is lower alkyl, phenyl, substituted phenyl (wherein the phenyl substituent is halo, lower alkyl or lower alkoxy), hydroxy-lower alkyl or amino (carboxy)-lower alkyl;

$R_7^a$ is 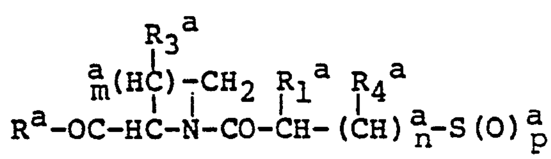

$M^a$ is O or S;
$m^a$ is 1 to 3;
$n^a$ and $p^a$ each is 0 to 2;

(b) having the formula

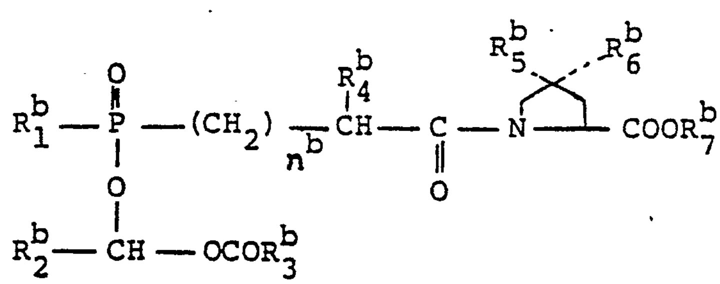

$R_1^b$ is alkyl, aryl, aralkyl, cycloalkyl or cycloalkylalkyl;

$R_2{}^b$ is cycloalkyl, 3-cyclohexenyl or 2-alkyl-3-cyclohexenyl;
$R_3{}^b$ is alkyl, cycloalkyl or phenyl;
$R_4{}^b$ is H or alkyl;
One of $R_5{}^b$ and $R_6{}^b$ is H and the other is alkyl-$X^b$-, phenyl-$X^b$-alkoxy, phenoxy, phenyl, cycloalkyl, alkyl or phenylalkyl; or
$R_5{}^b$ and $R_6{}^b$ together form $-X^bCH_2CH_2X^b-$, $X^b$ is S, SO or $SO_2$;
$R_7{}^b$ is H or $CH(R_2{}^b)OCOR_3{}^b$;
$n^b$ is 0 or 1;
'aryl' is phenyl optionally substituted by halo, alkyl, alkoxy, alkylthio, OH, alkanoyl, $NO_2$, amino, dialkylamino and/or $CF_3$;
alkyl has 1-10C, cycloalkyl 3-7C, alkoxy 1-8C and alkanoyl 2-9C;

(c) having the formula

$$R_1{}^cCO-NH-CHR_2{}^c-CH_2-\overset{\overset{\displaystyle O}{\|}}{\underset{\underset{\displaystyle OR_3{}^c}{|}}{P}}-A^c-CO-X^c$$

$R_1{}^c$ and $R_2{}^c$ are H, 1-7C alkyl, 1-7C haloalkyl, $(CH_2)_m{}^c-D^c$, 1-7C aminoalkyl or a group of formula:

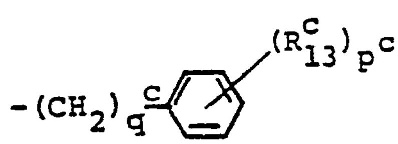

$D^c$ is cycloalkyl, furyl, thienyl or pyridinyl;
$A^c$ is $(CH_2)_n$-$CHR_{21}{}^c$, $NR_{22}{}^c$-$CHR_{23}{}^c$ or O-$CHR_{23}{}^c$;
$n^c$ is 0 or 1;
$q^c$ is 0-7;
$R_{21}{}^c$ is H, 1-7C alkyl, 1-7C haloalkyl, benzyl or phenethyl;
$R_{22}{}^c$ is H or 1-7C alkyl;
$R_{23}{}^c$ is H, 1-7C alkyl, 1-7C haloalkyl or $(CH_2)_r{}^c D_1{}^c$;
$D_1{}^c$ is phenyl, 4-hydroxyphenyl, 3,4-dihydroxyphenyl, 3-indolyl, 4-imidazolyl, $NH_2$, SH, 1-7C alkylthio, guanidino or $CONH_2$;
$X^c$ is a group of formula $(II)^c$-$(V)^c$, or $NR_4{}^c$-$CHR_5{}^c$-$COOR_6{}^c$;

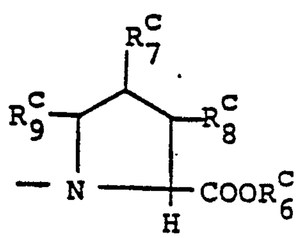

$(II)^c$

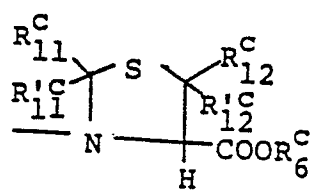

$(III)^c$

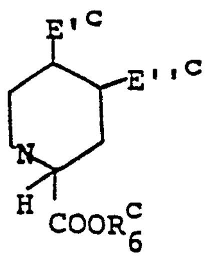

$(IV)^c$

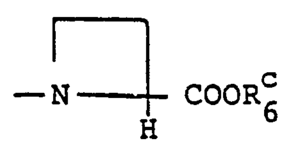

$(V)^c$

where

$R_9{}^c$ and $R_8{}^c$ are H and $R_7{}^c$ is as defined below;
$R_9{}^c$ and $R_7{}^c$ are H and $R_8{}^c$ is as defined below;

$R_7^c$ and $R_8^c$ are H and $R_9^c$ is as defined below;
$R_9^c$ and $R_8^c$ are H and $-CHR_7^c-$ is replaced by $-CR_{10}^cR_{10}^c$;
$R_9^c$ is H and $R_7^c + R_8^c$ form a double bond;
$R_9^c$ is H and $R_7^c + R_8^c$ complete a fused benzene ring; or
$R_8^c$ is H and $R_9^c + R_7^c$ complete a fused benzene ring;
$E'^c$ and $E''^c$ are H or $E'^c + E''^c$ complete a fused benzene ring;
$R_7^c$ is H, 1-7C alkyl, halogen, keto, OH, 2-8C alkanoyl-amino, $N_3$, $NH_2$, $NR_{19}^cR_{20}^c$, $(CH_2)_m^c-D^c$, $O-CONR_{15}^cR_{15}^c$, 1-7C alkoxy, 1-7C alkylthio, or a group of formula $(VI)^c$, $(VII)^c$ or $(VIII)^c$:

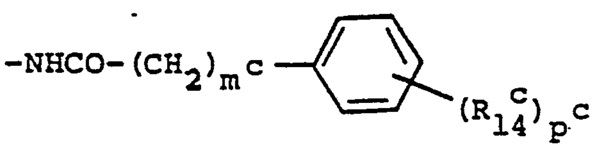

$(VI)^c$

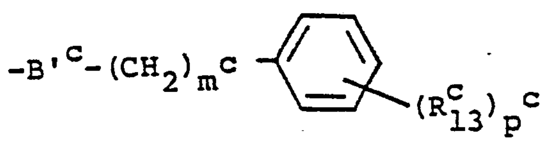

$(VII)^c$

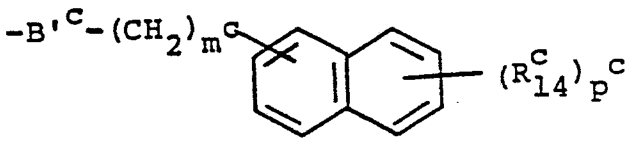

$(VIII)^c$

$B'^c$ is a bond or it is O or S;$R_8^c$ is keto, halogen, $O-CONR_{15}^cR_{15}^c$, 1-7C alkoxy, 1-7C alkylthio, or a group $(VII)^c$ or $(VIII)^c$ in which $B'^c$ is O or S;
$R_9^c$ is keto or a group $(VII)^c$ in which $B'^c$ is a bond;
$R_{10}^c$ is halogen or $Y^cR_{16}^c$;

$R_{11}{}^{c}$, $R'_{11}{}^{c}$, $R_{12}{}^{c}$ and $R'_{12}{}^{c}$ are H or 1-7C alkyl, or $R_{11}{}^{c}$ is a group of formula $(IX)^{c}$ and the other 3 are H;

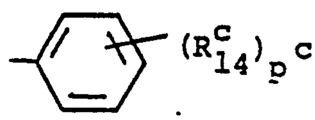

$(IX)^{c}$

$R_{13}{}^{c}$ is H, 1-4C alkyl, 1-4C alkoxy, 1-4C alkylthio, Cl, Br, F, $CF_3$, OH, Ph, PhO, PhS or $PhCH_2$;
$R_{14}{}^{c}$ is H, 1-4C alkyl, 1-4C alkoxy, 1-4C alkylthio, Cl, Br, F, $CF_3$ or OH;
$m^{c}$ is 0-3;
$p^{c}$ is 1-3 but it is 2 or 3 only when $R_{13}{}^{c}$ or $R_{14}{}^{c}$ is H, Me, MeO, Cl or F;
$R_{15}{}^{c}$ is H or 1-4C alkyl;
$Y^{c}$ is O or S;
$R_{16}{}^{c}$ is 1-4C alkyl or group $(VII)^{c}$ in which $B'^{c}$ is a bond; or the two $R_{16}{}^{c}$ groups complete a 5- or 6-membered ring in which the C atoms may each bear 1 or 2 alkyls having 1-4C;
$R_4{}^{c}$ is H, 1-7C alkyl, cycloalkyl or $(CH_2)_r{}^{c}Ph$;
$R_5{}^{c}$ is H, 1-7C alkyl or $(CH_2)_r{}^{c}$-$D_1{}^{c}$;
$r^{c}$ is 1-4;
$R_3{}^{c}$ and $R_6{}^{c}$ are H, 1-7C alkyl, $PhCH_2$, PhCH or $CHR_{17}{}^{c}$-O-$COR_{18}{}^{c}$;
$R_{17}{}^{c}$ is H, 1-7C alkyl or Ph;
$R_{18}{}^{c}$ is H, 1-7C alkyl, 1-7C alkoxy or Ph; or $R_{17}{}^{c}$ + $R_{18}{}^{c}$ form $(CH_2)_2$, $(CH_2)_3$, -CH=CH or o-phenylene;
$R_{19}{}^{c}$ is 1-7C alkyl, benzyl or phenethyl; and
$R_{20}{}^{c}$ is H or chosen as for $R_{19}{}^{c}$;

(d) having the formula

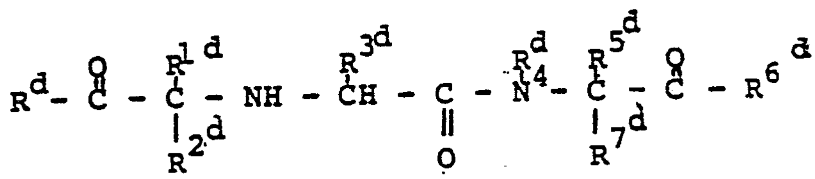

wherein

$R^d$ and $R^{6d}$ are the same or different and are hydroxy, lower alkoxy, lower alkenoxy, dilower alkylamino lower alkoxy (dimethylaminoethoxy), acylamino lower alkoxy (acetylaminoethoxy), acyloxy lower alkoxy (pivaloyloxymethoxy), aryloxy, such as phenoxy, arloweralkoxy, such as benzyloxy, substituted aryloxy or substituted arloweralkoxy wherein the substituent is methyl, halo or methoxy, amino, loweralkylamino, diloweralkylamino, hydroxyamino, arloweralkylamino such as benzylamino;

$R^{1d}$ is hydrogen, alkyl of from 1 to 20 carbon atoms which include branched and cyclic and unsaturated (such as allyl) alkyl groups, substituted loweralkyl wherein the substituent can be halo, hydroxy, lower alkoxy, aryloxy such as phenoxy, amino, diloweralkylamino, acylamino, such acetamido and benzamido, arylamino, guanidino, imidazolyl, indolyl, mercapto, loweralkylthio, arylthio such as phenylthio, carboxy or carboxamido, carboloweralkoxy, aryl such as phenyl or naphthyl, substituted aryl such as phenyl wherein the substituent is lower alkyl, lower alkoxy or halo, arloweralkyl, arloweralkenyl, heteroarlower alkyl or heteroarlower alkenyl such as benzyl, styryl or indolyl ethyl, substituted arloweralkyl, substituted arloweralkenyl, substituted

heteroarlower alkyl, or substituted heteroarlower alkenyl, wherein the substituent(s) is halo, dihalo, lower alkyl, hydroxy, lower alkoxy, amino, aminomethyl, acylamino (acetylamino or benzoylamino) diloweralkylamino, loweralkylamino, carboxyl, haloloweralkyl, cyano or sulfonamido; arloweralkyl or heteroarloweralkyl substituted on the alkyl portion by amino or acylamino (acetylamino or benzoylamino);

$R^{2d}$ and $R^{7d}$ are the same or different and are hydrogen or lower alkyl;

$R^{3d}$ is hydrogen, lower alkyl, phenyl lower alkyl, aminomethyl phenyl lower alkyl, hydroxy phenyl lower alkyl, hydroxy lower alkyl, acylamino lower alkyl (such as benzoylamino lower alkyl, acetylamino lower alkyl), amino lower alkyl, dimethylamino lower alkyl, halo lower alkyl, guanidino lower alkyl, imidazolyl lower alkyl, indolyl lower alkyl, mercapto lower alkyl, lower alkyl thio lower alkyl;

$R^{4d}$ is hydrogen or lower alkyl;

$R^{5d}$ is hydrogen, lower alkyl, phenyl, phenyl lower alkyl, hydroxy phenyl lower alkyl, hydroxy lower alkyl, amino lower alkyl, guanidino lower alkyl, imidazolyl lower alkyl, indolyl lower alkyl, mercapto lower alkyl or lower alkylthio lower alkyl;

$R^{4d}$ and $R^{5d}$ may be connected together to form an alkylene bridge of from 2 to 4 carbon atoms, an alkylene bridge of from 2 to 3 carbon atoms and one sulfur atom, an alkylene bridge of from 3 to 4 carbon atoms containing a double bond or an alkylene bridge as above substituted with hydroxy, loweralkoxy, lower alkyl or dilower

alkyl; and the pharmaceutically acceptable salts thereof;

(e) having the formula

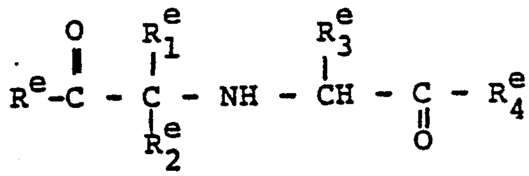

$R_4^e$ is a substituted proline of the formula

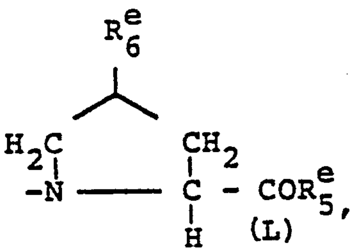

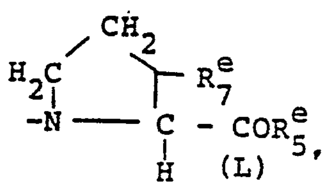

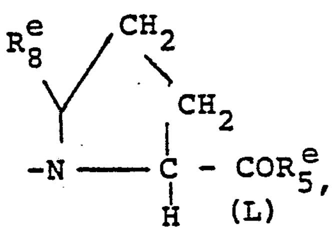

or

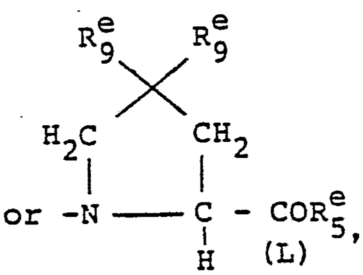

$R_6^e$ is halogen, keto, azido,

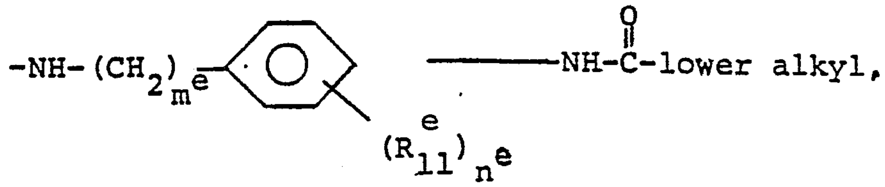

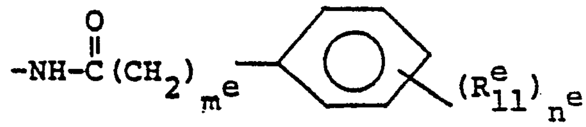

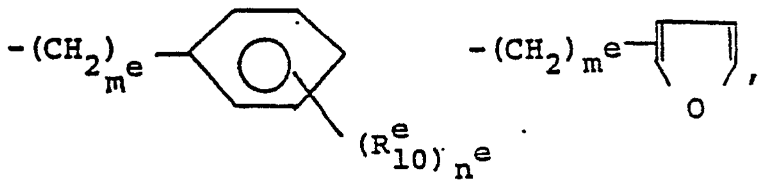,

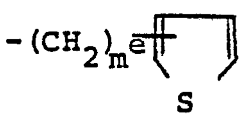,

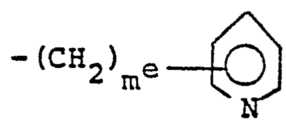,

a 1- or 2-naphthyl of the formula

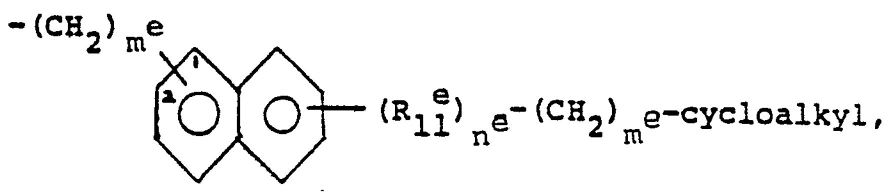

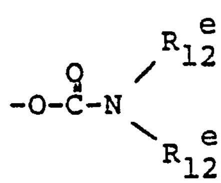

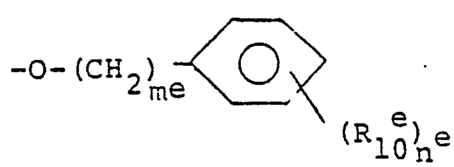

a 1- or 2-naphthyloxy of the formula

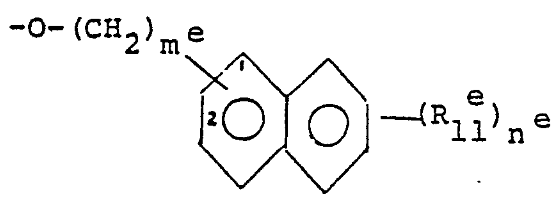

-S-lower alkyl,

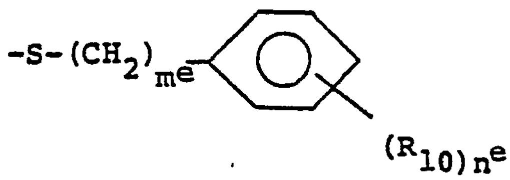

or a 1- or 2-naphthylthio of the formula

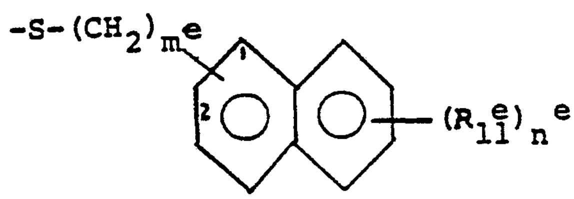

$R_7^e$ is keto, halogen,

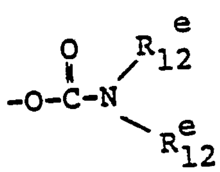

,

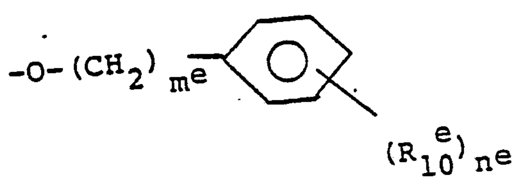

a 1- or 2-naphthloxy of the formula

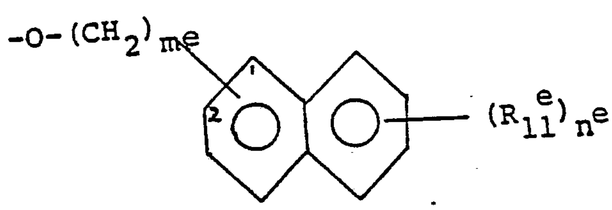

-S-lower alkyl,

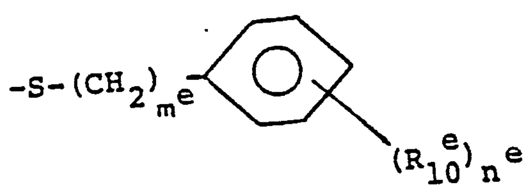

or a 1- or 2-naphthylthio of the formula

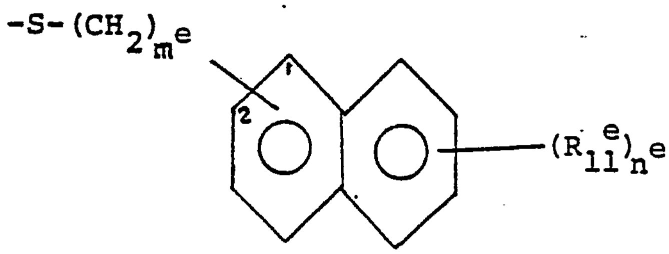

$R_8^e$ is keto or

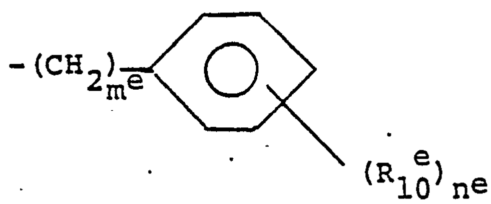

$R_9^e$ is halogen or $-Y^e-R_{13}^e$.

$m^e$ is zero, one, two, or three.

$R_{10}^e$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl.

$R_{11}^e$ is hydrogen, lower alkyl of 1 to 4 carbons, lower alkoxy of 1 to 4 carbons, lower alkylthio of 1 to 4 carbons, bromo, fluoro, trifluoromethyl, or hydroxy.

$n^e$ is one, two or three provided that n is more than one only if $R_{10}^e$ or $R_{11}^e$ is hydrogen, methyl, methoxy, chloro, or fluoro.

$R_{12}^e$ is hydrogen or lower alkyl of 1 to 4 carbons.

$Y^e$ is oxygen or sulfur.

$R_{13}^e$ is lower alkyl of 1 to 4 carbons.

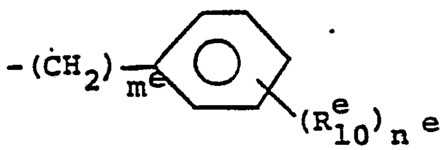

or the $R_{13}^{e}$ group join to complete an unsubstituted 5- or 6-membered ring or said ring in which one or more of the carbons has a lower alkyl of 1 to 4 carbons, or a di(lower alkyl of 1 to 4 carbons) substituent.

$R^{e}$ and $R_{5}^{e}$ are independently selected from hydroxy; lower alkoxy, di(lower alkyl)-amino-lower alkoxy, such as dimethylaminoethoxy, lower alkyl-carbonyl-amino-lower alkoxy, such as acetylaminoethoxy, lower alkyl-carbonyloxy-lower alkoxy, such as pivaloyloxymethoxy.

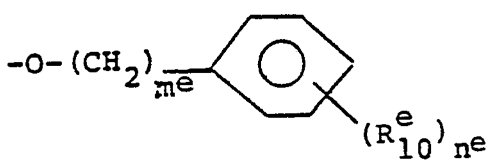

wherein $m^{e}$, $n^{e}$ and $R_{10}^{e}$ are as defined above, amino, lower alkyl-amino, di(lower alkyl)-amino, hydroxyamino, benzylamino, or phenethylamino.

$R_{1}^{e}$ is hydrogen, lower alkyl,

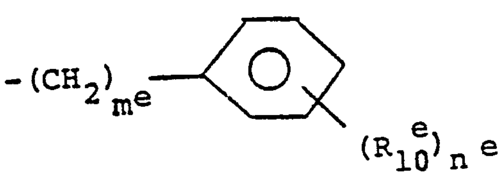

halo substituted lower alkyl, hydroxy substituted lower alkyl, $-(CH_2)_q{}^{e}$-cycloalkyl, $-(CH_2)_q{}^{e}$-carboxy, $-(CH_2)_q{}^{e}$-S-lower alkyl,

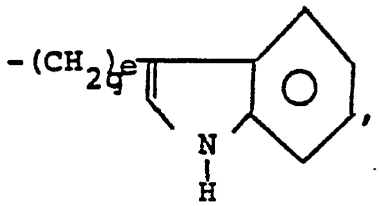 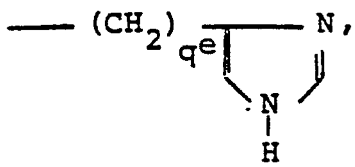

$-(CH_2)_q{}^e$-guanidinyl, $-(CH_2)_q{}^e-NH_2$
$-(CH_2)_q{}^e-N$(lower alkyl)$_2$

$-(CH_2)_q{}^e-NH-\overset{O}{\overset{\|}{C}}-$lower alkyl,

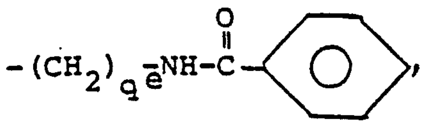, $-(CH_2)_q{}^e-SH$,

$-(CH_2)_{q^e}-$lower alkoxy, 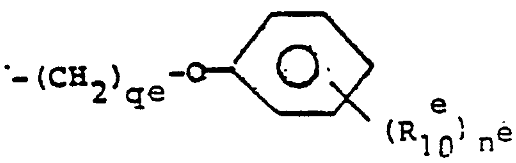

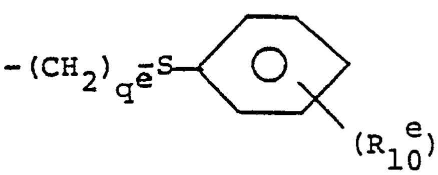 $-(CH_2)_q{}^e-\overset{O}{\overset{\|}{C}}-NH_2$

$-(CH_2)_q{}^e-\overset{O}{\overset{\|}{C}}-$lower alkoxy, or

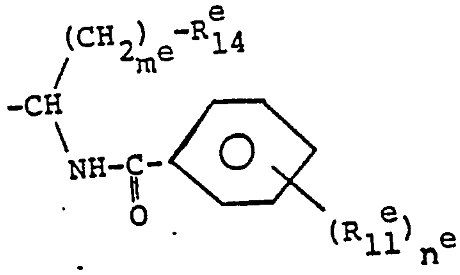

wherein $m^e$, $n^e$, $R_{10}^e$ and $R_{11}^e$ are as defined above, $R_{14}^e$ is lower alkyl, cycloalkyl, or

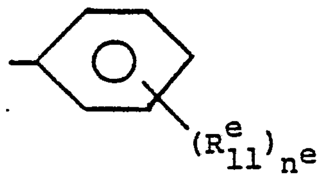

and $q^e$ is an integer from 1 to 4.

$R_2^e$ is hydrogen or lower alkyl.

$R_3^e$ is hydrogen, lower alkyl, halo substituted lower alkyl, hydroxy substituted lower alkyl, $-(CH_2)_{q^e}-NH_2$, $-(CH_2)_{q^e}-N-$(lower alkyl$)_2$, $-(CH_2)_{q^e}-$guanidinyl, $-(CH_2)_{q^e}-SH$, $-(CH_2)_{q^e}-S-$lower alkyl,

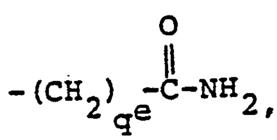, 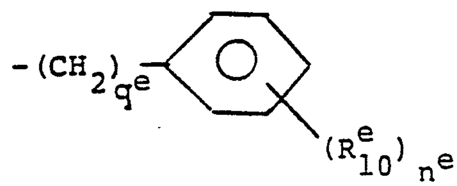,

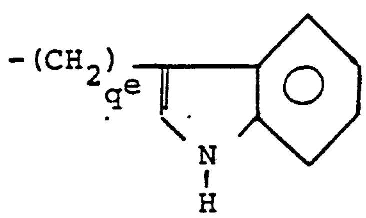, 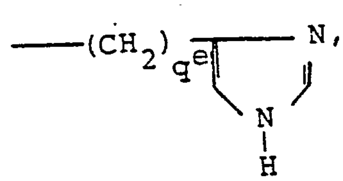,

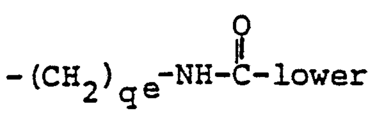lower alkyl, or

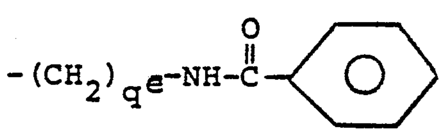

wherein $R_{10}{}^{e}$, $n^{e}$ and $q^{e}$ are as defined above;

(f) having the formula

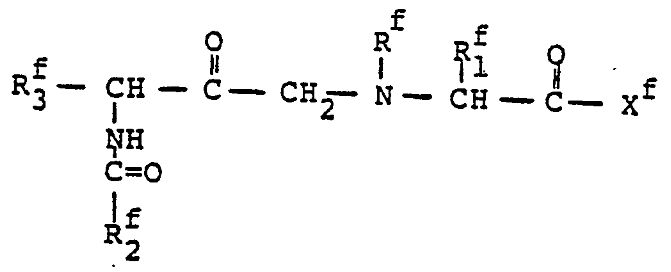

$X^{f}$ is an amino or imino acid of the formula

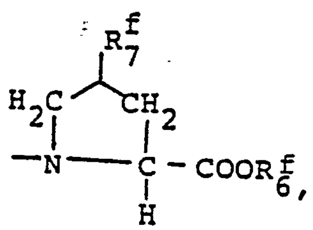

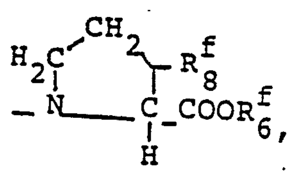

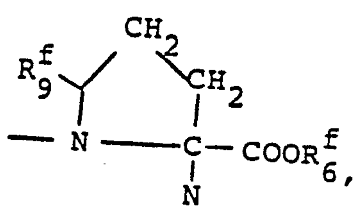

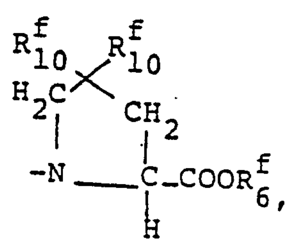

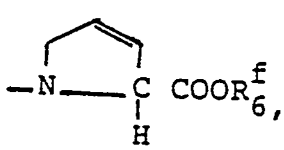

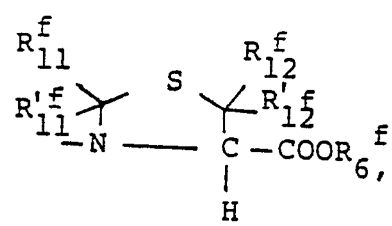

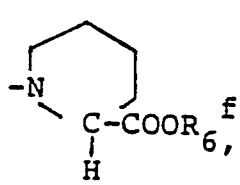

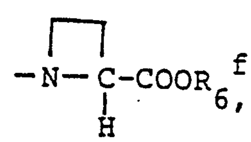

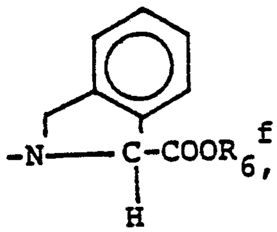

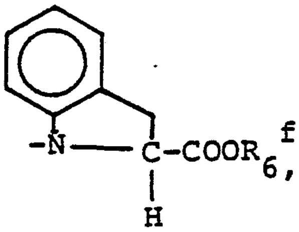

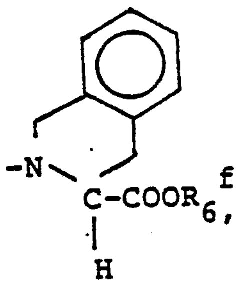

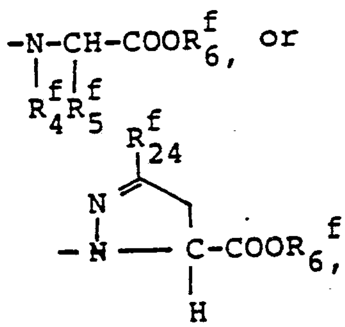

or

$R_7^f$ is hydrogen, lower alkyl, halogen, keto, hydroxy,

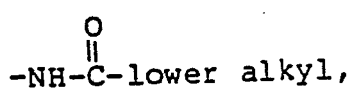

azido, amino,

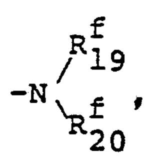

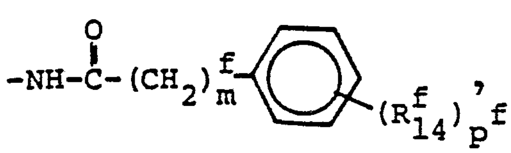

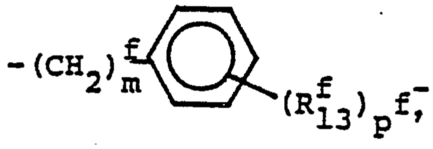, 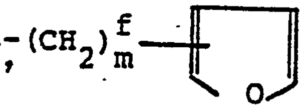,

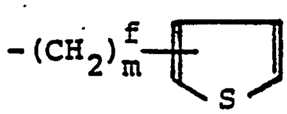, 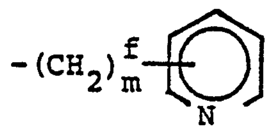,

a 1- or 2-naphthyl of the formula

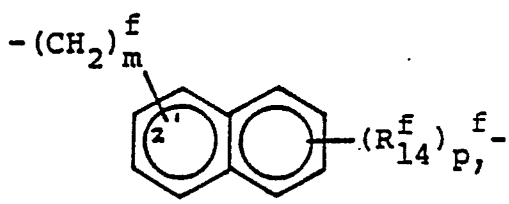 $-(CH_2)_m^f$-cycloalkyl,

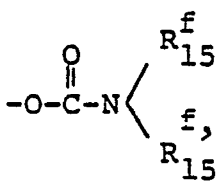, -O-lower alkyl,

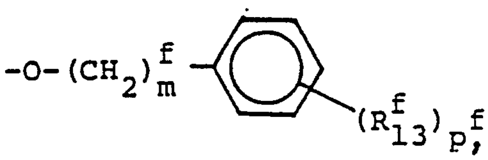

a 1- or 2-naphthyloxy of the formula

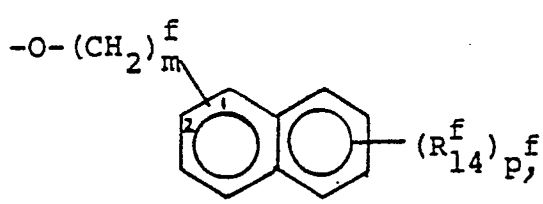

-S-lower alkyl,

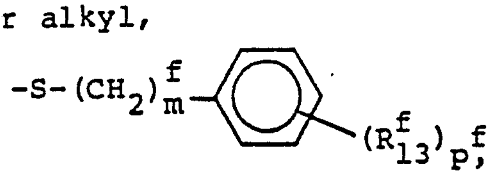

or a 1- or 2-naphthylthio of the formula

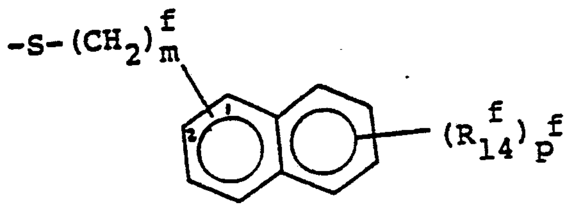

$R_8^f$ is keto, halogen,

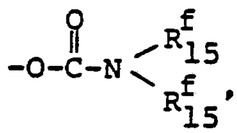

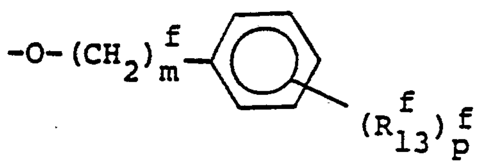

-O-lower alkyl, a 1- or 2-naphthyloxy of the formula

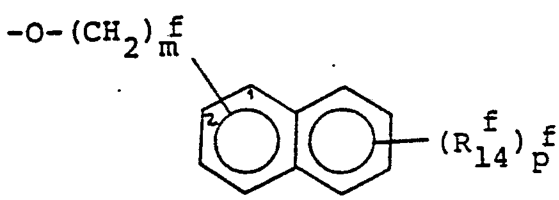

-S-lower alkyl,

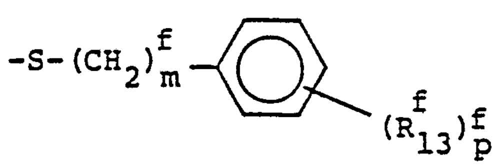

or a 1- or 2-naphthylthio of the formula

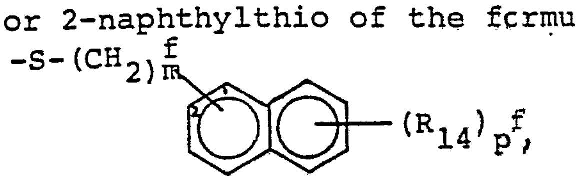

$R_9^f$ is keto or

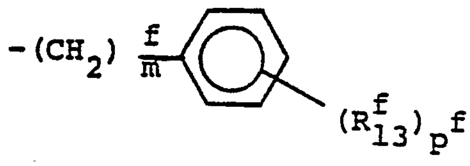

$R_{10}^f$ is halogen or $-Y^f-R_{16}^f$
$R_{11}^f$, $R'_{11}^f$, $R_{12}^f$ and $R'_{12}^f$ are independently selected from hydrogen and lower alkyl or $R'_{11}^f$, $R_{12}^f$ and $R'_{12}^f$ are hydrogen and $R_{11}^f$ is

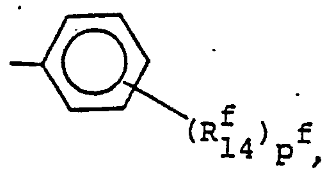

$R_{13}^f$ is hydrogen, lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl.
$R_{14}^f$ is hydrogen, lower alkyl of 1 to 4 carbons, lower alkoxy of 1 to 4 carbons, lower alkylthio of 1 to 4 carbons, chloro, bromo, fluoro, trifluoromethyl, or hydroxy.
$m^f$ is zero, one, two, three, or four.
$p^f$ is one, two or three provided that $p^f$ is more than one only if $R_{13}^f$ or $R_{14}^f$ is hydrogen, methyl, methoxy, chloro, or fluoro.
$R_{15}^f$ is hydrogen or lower alkyl of 1 to 4 carbons.
$Y^f$ is oxygen or sulfur.
$R_{16}^f$ is lower alkyl of 1 to 4 carbons.

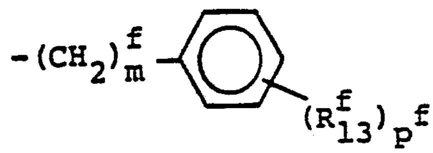 ,

or the $R_{16}{}^f$ groups join to complete an unsubstituted 5- or 6-membered ring or said ring in which one or more of the carbons has a lower alkyl of 1 to 4 carbons or a di(lower alkyl of 1 to 4 carbons) substituent.

$R_4{}^f$ is hydrogen, lower alkyl,

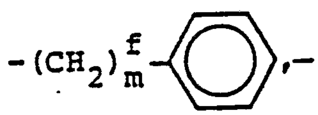, $-(CH_2)_m^f$-cycloalkyl,

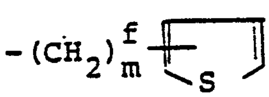, 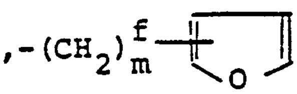,

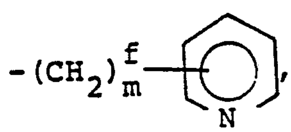, or 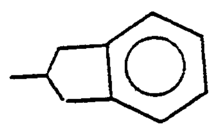

$R_5{}^f$ is hydrogen, lower alkyl,

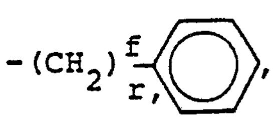, 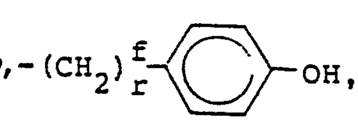,

$-(CH_2)_r^f-OH$, 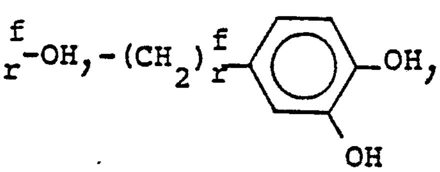,

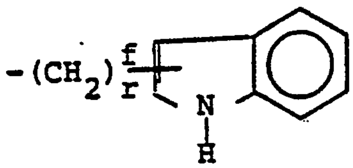, 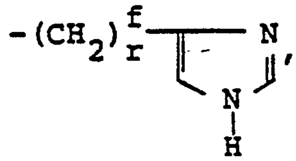,

$-(CH_2)_r^f-NH_2, -(CH_2)_r^f-SH, -(CH_2)_r^f$-S-lower alkyl,

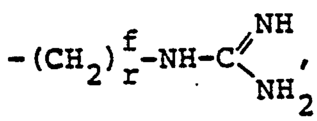, or 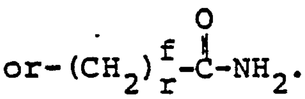.

$r^f$ is an integer from 1 to 4.
$R_{19}{}^f$ is lower alkyl, benzyl, or phenethyl.
$R_{20}{}^f$ is hydrogen, lower alkyl, benzyl or phenethyl.
$R^f$ is hydrogen, lower alkyl, cycloalkyl,

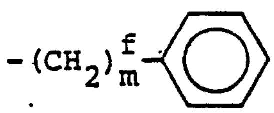,

$-(CH_2)_2-NH_2$, $-(CH_2)_3-NH_2$, $-(CH_2)_4-NH_2$,
$-(CH_2)_2-OH$, $-(CH_2)_3-OH$, $-(CH_2)_4-OH$,
$-(CH_2)_2-SH$, $-(CH_2)_3-SH$, or $-(CH_2)_4-SH$.

$R_1{}^f$ is hydrogen, lower alkyl, halo substituted lower alkyl,

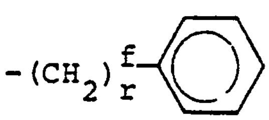, 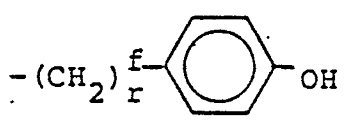,

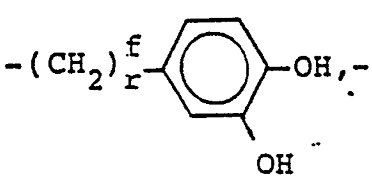, 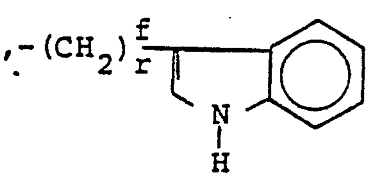,

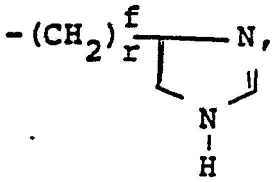, $-(CH_2)_r^f-NH_2$, $-(CH_2)_r^f-SH$ ,

$-(CH_2)_r^f-OH$, $-(CH_2)_r^f-S$-lower alkyl,

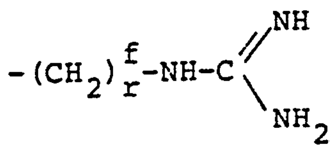 ,or 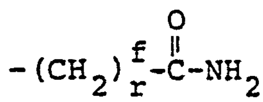

provided that $R_1^f$ is hydrogen only if $R^f$ is other than hydrogen.
$R_2^f$ is

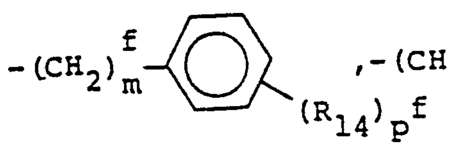 , 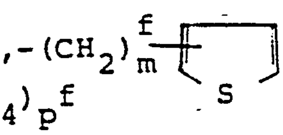 ,

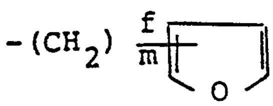 ,or 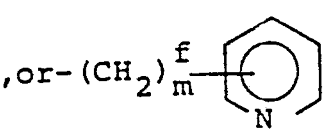

$R_3^f$ is hydrogen, lower alkyl,

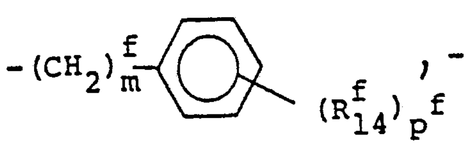 , 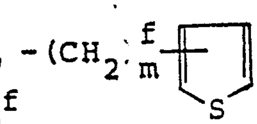 ,

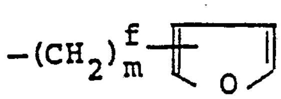 , 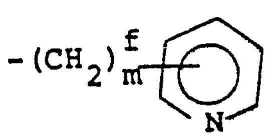 halo substituted

lower alkyl ,$-(CH_2)_m^f$-cycloalkyl ,$-(CH_2)_r^f-$ 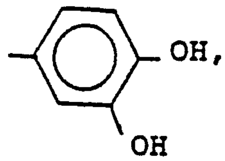

$-(CH_2)_r^f$ 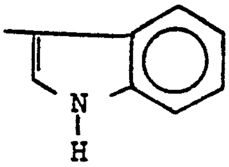, $-(CH_2)_r^f-OH$,

$-(CH_2)_r^f$ 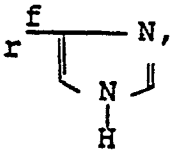,$-(CH_2)_r^f-NH_2$,$-(CH_2)_r^f-SH$,

$-(CH_2)_r^f$-S-lower alkyl,$-(CH_2)_r^f-NH-$ 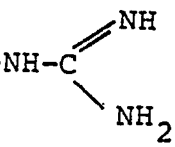 ,or $-(CH_2)_r^f-$ 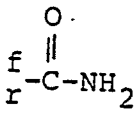

wherein $m^f$, $R_{14}^f$, $p^f$ and $r^f$ are as defined above.

$R_6^f$ is hydrogen, lower alkyl, benzyl, benzhydryl,

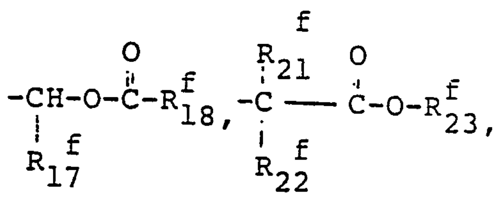, $-CH-(CH_2-OH)_2$,

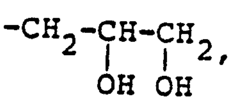, $-(CH_2)_2-N(CH_3)_2$, or $-CH_2-$ 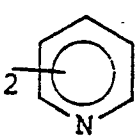

$R_{17}^f$ is hydrogen, lower alkyl, cycloalkyl, or phenyl.

$R_{18}^f$ is hydrogen, lower alkyl, lower alkoxy, or phenyl or $R_{17}^f$ and $R_{18}^f$ taken together are $-(CH_2)_2-$, $-(CH_2)_3-$, $-CH=CH-$, or

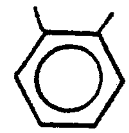,

$R_{21}{}^f$ and $R_{22}{}^f$ are independently selected from hydrogen and lower alkyl.
$R_{23}{}^f$ is lower alkyl.
$R_{24}{}^f$ is hydrogen, lower alkyl.

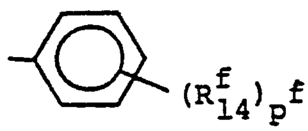 ,

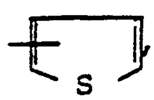, 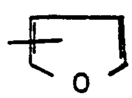, or 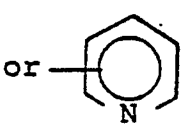

(g) having the formula

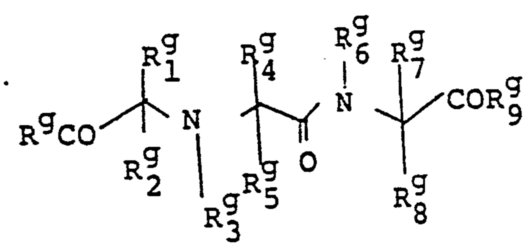

(I)

either

(A) $R^g$ and $R_9{}^g$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1^g$-$R_5^g$, $R_7^g$ and $R_8^g$ are 1-20C alkyl, 2-20C alkenyl, 2-20C alkynyl, aryl, aryl-(1-6C) alkyl having 7-12C or heterocyclyl-(1-6C)alkyl having 7-12C;

$R_6^g$ is cycloalkyl, polycycloalkyl, partly saturated cycloalkyl or polycycloalkyl, cycloalkyl-(1-6C)alkyl having 3-20C, 6-10C aryl, aryl-(1-6C)alkyl, aryl-(2-6C)alkenyl or aryl-(2-6C) alkynyl; or

$R_2^g$ and $R_3^g$ together with the C and N atoms to which they are attached or $R_3^g$ and $R_5^g$ together with the N and C atoms to which they are attached form an N-heterocycle containing 3-5C or 2-4C and a S atom;

all alkyl, alkenyl and alkynyl are optionally substituted by OH, 1-6C alkoxy, thio(sic), 1-6C alkylthio, $NH_2$, mono- or di(1-6C alkyl)amino, halogen or $NO_2$;

all 'cycloalkyl' groups (including poly and partially unsaturated) are optionally substituted by halogen, 1-6C hydroxyalkyl, 1-6C alkoxy, amino-(1-6C alkyl)amino, di-(1-6C alkyl)amino, SH, 1-6C alkylthio, $NO_2$ or $CF_3$; and aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^g$ and $R_9^g$ are H or 1-6C alkoxy;

$R_1^g$ and $R_2^g$ are H, 1-6C alkyl, aryl-(1-6C) alkyl having 7-12C or heterocyclyl-(1-6C) alkyl having 6-12C;

$R_3^g$-$R_5^g$, $R_7^g$ and $R_8^g$ are H or 1-6C alkyl;

$R_6^g$ is cycloalkyl, polycycloalkyl, partly saturated cycloalkyl or polycycloalkyl, cycloalkyl-(1-6C) alkyl having 3-20C, aryl or aryl-(1-6C) alkyl; and

aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino;

(h) having the formula

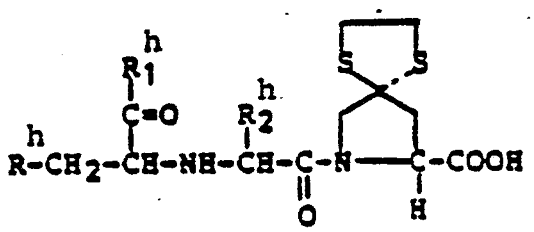

wherein:

$R^h$ is lower alkyl, benzyl, benzylthio, benzyloxy, phenylthio or phenoxy;

$R_1^h$ is hydroxy or lower alkoxy;

$R_2^h$ is hydrogen, lower alkyl or amino lower alkyl; and the pharmaceutcally acceptable salts thereof;

(i) having the formula

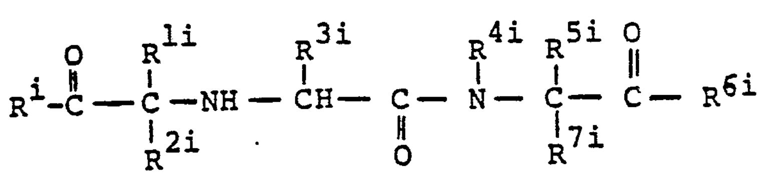

or a pharmaceutically acceptable salt thereof, wherein $R^i$ and $R^{6i}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substituent is methyl, halo or methoxy; $R^{1i}$ is hydrogen, alkyl of from 1 to 10 carbon atoms, substituted lower alkyl wherein the substituent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^{2i}$ and $R^{7i}$ are the same or different and are hydrogen or lower alkyl; $R^{3i}$ is hydrogen, lower alkyl, phenyl lower alkyl, aminomethylphenyl lower alkyl, hydroxyphenyl lower alkyl, hydroxy lower alkyl, acylamino lower alkyl, amino lower alkyl, dimethylamino lower alkyl, guanidino lower alkyl, imidazolyl lower alkyl, indolyl lower alkyl, or lower alkyl thio lower alkyl; $R^{4i}$ and $R^{5i}$ are the same or different and are hydrogen, lower alkyl or $Z^i$, or $R^{4i}$ and $R^{5i}$ taken together form a group represented by $Q^i$, $U^i$, $V^i$, $Y^i$, $D^i$ or $E^i$, wherein;

$Z^i$ is

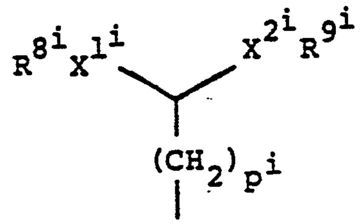

wherein $X^{1i}$ and $X^{2i}$ independent of each other are O, S or $CH_2$, $R^{8i}$ and $R^{9i}$ independent of each other are lower alkyl, lower alkenyl, lower alkynyl, cycloalkyl having 3 to 8 carbon atoms, hydroxy lower alkyl or $-(CH_2)_nAr^i$, wherein $n^i$ is 0, 1, 2 or 3 and $Ar^i$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8i}$ and $R^{9i}$ taken together form a bridge $W^i$, wherein $W^i$ is a single bond or a methylene bridge or a substituted methylene bridge when at least one of $X^{1i}$ and $X^{2i}$ is methylene, or $W^i$ is an alkylene or substituted alkylene bridge having 2 or 3 carbon atoms, said substituted methylene bridge or said substituted alkylene bridge having one or two substituents selected from lower alkyl, aryl and aryl lower alkyl groups, and $p^i$ is 0, 1 or 2; with the proviso that at least one of $R^4$ and $R^5$ is $Z^i$, with the proviso that if $R^4$ is $Z^i$ and $p^i$ is 0 then $X^{1i}$ and $X^{2i}$ must both be methylene, and with the proviso that if $X^{1i}$ and $X^{2i}$ are both methylene then $R^{8i}$ and $R^{9i}$ must form an alkylene bridge $W^i$;

$Q^i$ is

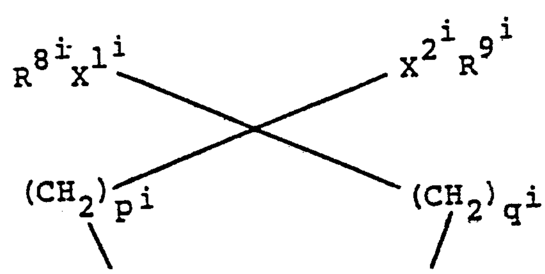

wherein $R^{8i}$, $R^{9i}$, $X^{1i}$ and $X^{2i}$ are as defined above, $p^i$ is 0, 1 or 2, $q^i$ is 0, 1 or 2, with the proviso that the sum of $p^i$ and $q^i$ must be 1, 2 or 3, with the proviso that if $p^i$ is 0 then $X^{1i}$ and $X^{2i}$ must be methylene, and with the proviso that if $X^1$ and $X^{2i}$ are methylene then $R^{8i}$ and $R^{9i}$ taken together form a bridge $W^i$, wherein $W^i$ is as defined above;

$V^i$ is

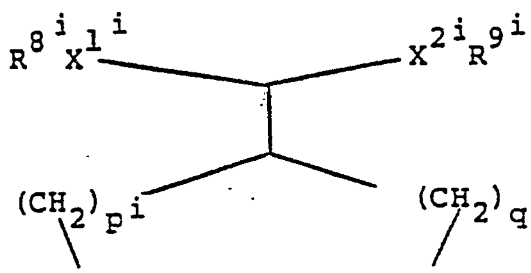

wherein $R^{8i}$, $R^{9i}$, $X^{1i}$ and $X^{2i}$ are as defined above, $p^i$ is 0, 1 or 2 and $q^i$ is 0, 1 or 2, with the proviso that the sum of $p^i$ and $q^i$ is 1, 2 or 3, with the proviso that if $X^{1i}$ and $X^{2i}$ are $CH_2$ then $R^{8i}$ and $R^{9i}$ taken together form a bridge $W^i$, wherein $W^i$ is as defined above;

$U^i$ is

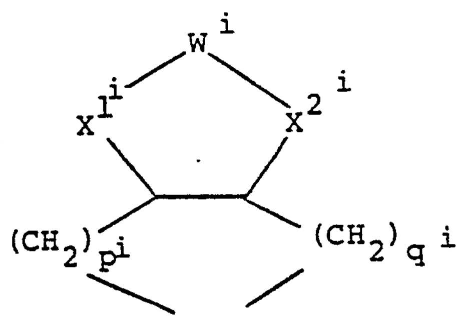

wherein $W^i$ is as defined above (except that $W^i$ may also be a methylene bridge when $X^{1i}$ and $X^{2i}$ are oxygen or sulfur), $X^{1i}$ and $X^{2i}$ are as defined above, $p^i$ is 0, 1 or i is 0, 1 or 2, with the proviso that the sum of $p^i$

and $q^i$ is 1 or 2, and with the proviso that if $p^i$ is 0, $X^{1i}$ must be $CH_2$;

$Y^i$ is

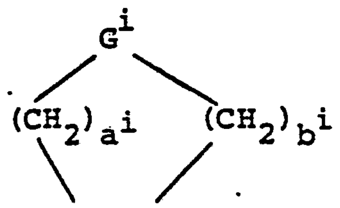

wherein $G^i$ is oxygen, sulfur or $CH_2$, $a^i$ is 2, 3 or 4 and $b^i$ is 1, 2, 3, 4 or 5, with the proviso that the sum of $a^i$ and $b^i$ is 5, 6 or 7 or

$G^i$ is $CH_2$, $a^i$ is 0, 1, 2 or 3, $b^i$ is 0, 1, 2 or 3 with the proviso that the sum of $a^i$ and $b^i$ is 1, 2 or 3, with the proviso that the sum of $a^i$ and $b^i$ may be 1, 2 or 3 only if $R^{1i}$ is lower alkyl substituted with aralkylthio or aralkyloxy;

$D^i$ is

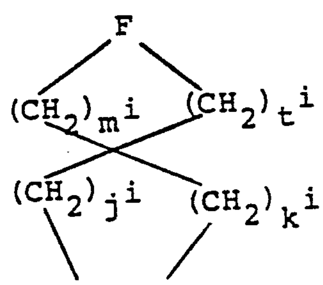

wherein $F^i$ is O or S, $j^i$ is 0, 1 or 2 and $k^i$ is 0, 1 or 2, with the proviso that the sum of $j^i$ and $k^i$ must be 1, 2 or 3, and $m^i$ is 1, 2 or 3 and $t^i$ is 1, 2 or 3, with the proviso that the sum of $m^i$ and $t^i$ must be 2, 3 or 4;

$E^i$ is

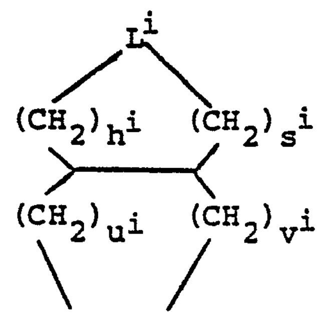

wherein $L^i$ is O or S, $u^i$ is 0, 1 or 2 and $v^i$ is 0, 1 or 2, with the proviso that the sum of $u^i$ and $v^i$ must be 1 or 2, and $h^i$ is 1 or 2 and $s^i$ is 1 or 2, with the proviso that the sum of $h^i$ and $s^i$ must be 2 or 3.

(j) having the formula

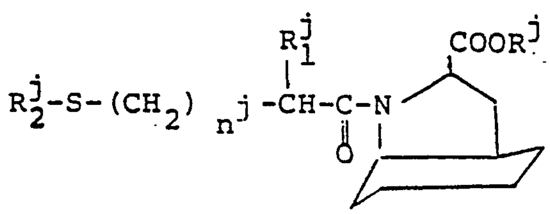

wherein $R^j$ is hydrogen or lower alkyl; $R_1{}^j$ is hydrogen,
lower alkyl, or benzyl; $R_2{}^j$ is hydrogen or $R_3{}^j$-$\overset{O}{\overset{\|}{C}}$-wherein $R_3{}^j$ is lower alkyl, heteroaryl containing 4 to 9 carbon atoms and one or two nitrogen, oxygen or sulfur atoms; phenyl, substituted phenyl having 1 or 2 substituents selected from the group consisting of fluorine, chlorine, bromine, lower alkyl or alkoxy; and $n^j$ is 0 or 1; wherein lower alkyl and lower alkoxy include straight or branched groups containing 1 to 4 carbon atoms, and pharmaceutically acceptable salts of the compounds when

$R^j$ is hydrogen and when $R_3{}^j$ is heteroaryl containing 1 or 2 nitrogen atoms, are disclosed;

(k) having the formula

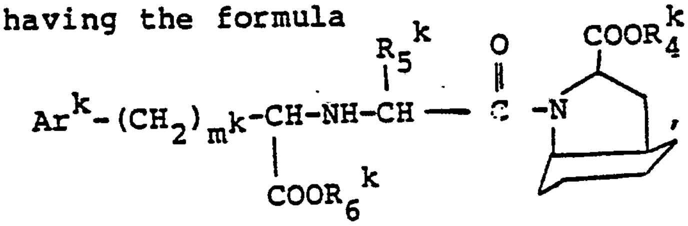

wherein $R_4{}^k$ is hydrogen or lower alkyl; $R_5{}^k$ is hydrogen, lower alkyl or benzyl; $R_6{}^k$ is hydrogen or lower alkyl; $Ar^k$ is phenyl or phenyl substituted with 1 or 2 substituents selected from the group consisting of fluorine, chlorine, bromine, lower alkyl, lower alkoxy, hydroxy or amino; and m is 0 to 3; wherein lower alkyl and lower alkoxy contain 1 to 4 straight or branched carbon atoms; and the pharmaceutically acceptable salts thereof;

(L) having the formula

where

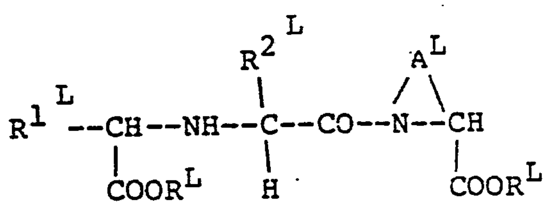

$(I)^L$

and

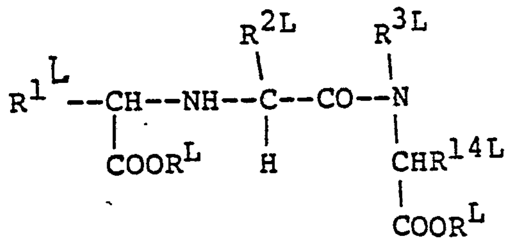

$(Ia)^L$

wherein:

$R^L$ and $R^{2L}$ are independently hydrogen; loweralkyl; aralkyl; or aryl;

$R^{1L}$ is hydrogen; branched or straight chain $C_{1-12}$ alkyl and alkenyl; $C_3$-$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, dihalo, loweralkyl, hydroxy, loweralkoxy, amino, aminoloweralklyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

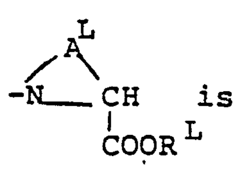 is 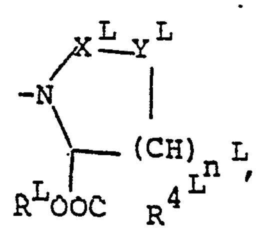, 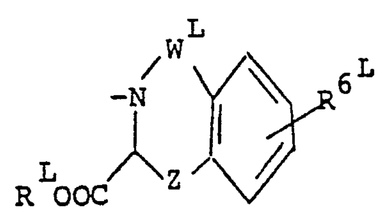

or 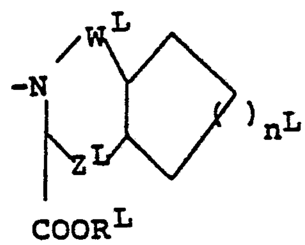

where
$X^L$ and $Y^L$ taken together are $-CH_2-CH_2-$;

$$-\underset{R^{5L}}{\underset{|}{CH}}-S-;\quad -\underset{O}{\underset{\|}{C}}-CH_2-;\quad -CH_2-\overset{O}{\overset{\|}{C}}-;\quad -\overset{O}{\overset{\|}{C}}-O-;\quad -\overset{O}{\overset{\|}{C}}-S-;$$

$$-CH_2-\overset{OR^{4L}}{\overset{|}{CH}}-;\quad -\overset{O}{\overset{\|}{C}}-\overset{R^{4L}}{\overset{|}{N}}-;\ \text{or}\ -CH_2-\overset{R^{4L}}{\overset{|}{C}}-R^{5L};$$

$R^{4L}$ is hydrogen, loweralkyl; aryl; substituted aryl;
$R^{5L}$ is hydrogen; loweralkyl; aryl or substituted aryl;
$n^L$ is 1 to 3;
$w^L$ is absent; $-CH_2$; or $-\overset{O}{\overset{\|}{C}}-$;
$Z^L$ is $-(CH_2)_m{}^L$, where $m^L$ is 0 to 2, provided that $m^L$ may not be 0 and $W^L$ may not be absent at the same time; and
$R^{6L}$ is hydrogen; loweralkyl; halo; or $OR^{4L}$;

$R^{2L}$ is $-(CH_2)_r{}^L - B^L - (CH_2)_s{}^L - NR^{7L}R^{15L}$
where
$r^L$ and $s^L$ are independently 0 to 3;
$B^L$ is absent; -O-; -S-; or $-NR^{8L}$;
where $R^{8L}$ is hydrogen; loweralkyl; alkanoyl; or aroyl; and

$R^{7L}$ is 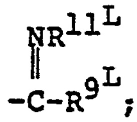 ; 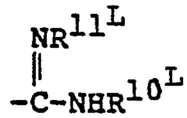 ; or 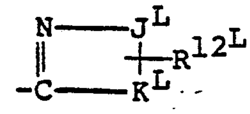

where

$R^{9L}$ is loweralkyl; aralkyl; aryl; heteroaryl; or heteroarloweralkyl and these groups substituted by hydroxy, lower alkoxy or halo; carboxyl; carboxamido; nitromethenyl.

$R^{10L}$ is hydrogen; loweralkyl; aryl; or amidino;

$R^{11L}$ hydrogen; loweralkyl; cyano; amidino; aryl; aroyl; loweralkanoyl; 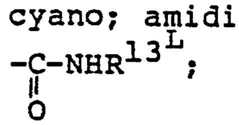 ;

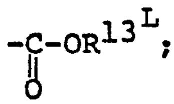 ; $-NO_2$; $-SO_2NH_2$;

or

$SO_2R^{13L}$;

$R^{12L}$ is hydrogen; loweralkyl; halo; aralkyl; amino; cyano; mono- or diloweralkylamino; or $OR^{4L}$;

$R^{13L}$ is hydrogen; loweralkyl; or aryl;

$R^{15L}$ is hydrogen; lower alkyl; aralkyl; or aryl.

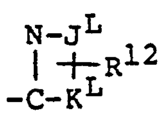

constitute a basic heterocycle of 5 or 6 atoms or benzofused analogs thereof and optionally containing 1-3 N atoms, an oxygen, a sulfur, an S=O, or an $SO_2$ group optionally substituted by amino, lower alkyl amino, diloweralkyl amino, lower alkoxy, or aralkyl groups;

$R^{3L}$ is $C_{3-8}$ cycloalkyl and benzofused $C_{3-8}$ cycloalkyl; perhydrobenzofused $C_{3-8}$ cycloalkyl; aryl; substituted aryl; heteroaryl; substituted heteroaryl;

$R^{14L}$ is hydrogen or loweralkyl; and, a pharmaceutically acceptable salt thereof;

(m) having the formula

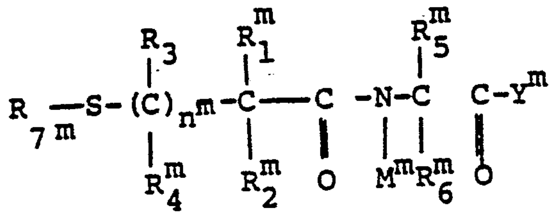

wherein

$R_1^m$, $R_2^m$, $R_3^m$, $R_4^m$, $R_5^m$ and $R_6^m$ are hydrogen, alkyl, alkenyl, alkynyl, phenyl-alkyl, and cycloalkyl, and may be the same or different,

$n^m$ is an integer from 0 to 4 inclusive,

$M^m$ is alkenyl, alkynyl, cycloalkyl, cycloalkyl-alkyl, polycycloalkyl, polycycloalkyl-alkyl, aryl, aralkyl, heteroaryl, heteroaryl-alkyl, hetero-cycloalkyl, hetero-cycloalkyl-alkyl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylamino-alkyl, or dialkylaminoalkyl,

$Y^m$ is hydroxy, alkoxy, amino, or substituted amino, aminoalkanoyl, aryloxy, aminoalkoxy, or hydroxyalkoxy, and

$R_7^m$ is hydrogen, alkanoyl, carboxyalkanoyl, hydroxyalkanoyl, aminoalkanoyl, cyano, amidino, carbalkoxy, $Z^mS$ or

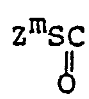

wherein $Z^m$ is hydrogen, alkyl, hydroxyalkyl, or the radical

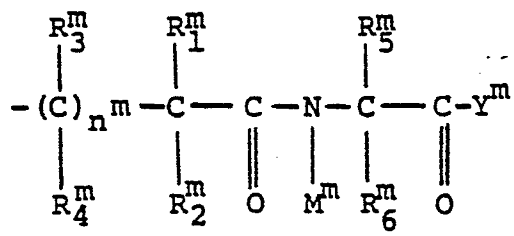

wherein $R_1^m$, $R_2^m$, $R_3^m$, $R_4^m$, $R_5^m$, $R_6^m$ $n^m$, $M^m$ and $Y^m$ are as described above; and where $Y^m$ is hydroxy, their non-toxic, pharmaceutically acceptable alkali metal, alkaline earth metal, and amine salts; or

(n) having the formula

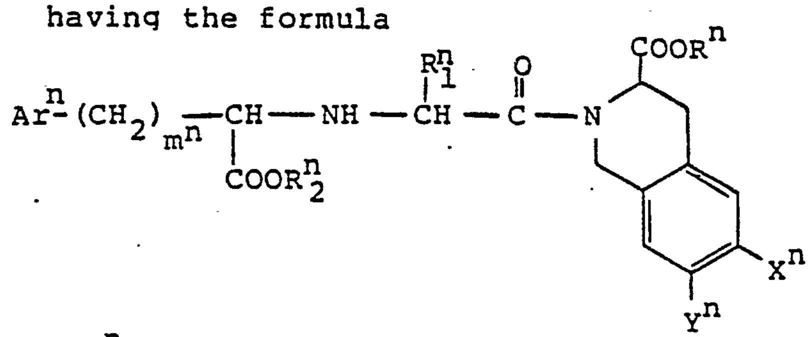

wherein $R^n$ is hydrogen, lower alkyl or aralkyl; $R_1^n$ is hydrogen, lower alkyl, or benzyl; $R_2^n$ is hydrogen or lower alkyl, and Ar is phenyl or phenyl substituted with 1 or 2 substituents selected from the group consisting of fluorine, chlorine, bromine, lower alkyl, lower alkoxy, hydroxy or amino; X and Y are independently hydrogen, lower alkyl, lower alkoxy, lower alkylthio, lower alkylsulfinyl, lower alkylsulfonyl, hydroxy, or X and Y together are methylenedioxy; m is 0 to 3; and the pharmaceutically acceptable acid salts thereof;

(o) having the formula

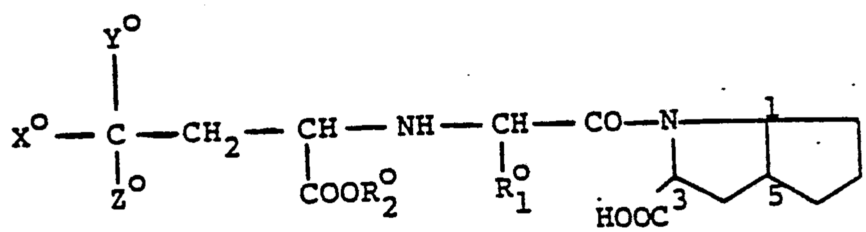

hydrogen atoms at ring positions 1 and 5 are cis to each other and the 3-carboxy group has the endo orientation;

$R_1^o$ is H, allyl, vinyl or the side chain of an optionally protected naturally occurring α-amino acid;

$R_2^o$ is H, 1-6C alkyl, 2-6C alkenyl or aryl(1-4C alkyl);

$Y^o$ is H or OH and $Z^o$ is H, or $Y^o$ and $Z^o$ together oxygen;

$X^o$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C alkyl), or methylenedioxy) or indol-3-yl);

(p) having the formula

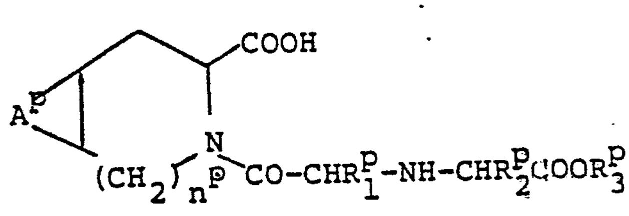

$n^p$ is 0 or 1;

$A^p$ 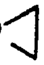 is a benzene or cyclohexane ring:

$R_1{}^p$ and $R_2{}^p$ are each 1-6C alkyl, 2-6C alkenyl, 5-7C cycloalkyl, 5-7C cycloalkenyl, 7-12C cycloalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^p$ and $R_2{}^p$ groups are optionally substituted.

$R_3{}^p$ is H, 1-6C alkyl, 2-6C alkenyl or 7-14C aralkyl;

(q) having the formula

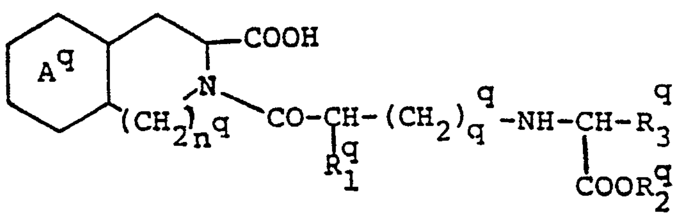

wherein:

the ring $A^q$ is saturated and $n^q$=0 or 1, or the ring $A^q$ is a benzene ring and $n^q$=1,

$R_1{}^q$ represents a lower alkyl group having from 1 to 4 carbon atoms which can carry an amino group,

$R_2{}^q$ represents a hydrogen atom or a alkyl group having from 1 to 4 carbon atoms,

$R_3{}^q$ represents a straight or branched alkyl group, a mono- or dicycloalkylalkyl or phenylalkyl group having no more than a total of 9 carbon atoms, or a substituted alkyl group of the formula:

$$-(CH_2)_p{}^q-Y^q-\underset{R_4{}^q}{CH}-R_5{}^q$$

with $R_4{}^q$=H, a lower alkyl ($C_1$ to $C_4$) or a cycloalkyl ($C_3$ to $C_6$ group,

$R_5{}^q$=H, a lower alkyl ($C_1$ to $C_4$) a cycloalkyl ($C_3$ to $C_6$) or an alkoxycarbonyl group,

$Y^q$=S or >N-$Q^q$ where $Q^q$=H, or an acetyl or benzyloxycarbonyl group, and

$p^q$=1 or 2, and

$q^q$=0 or 1, and the pharmaceutically acceptable salts thereof;

(r) having the formula

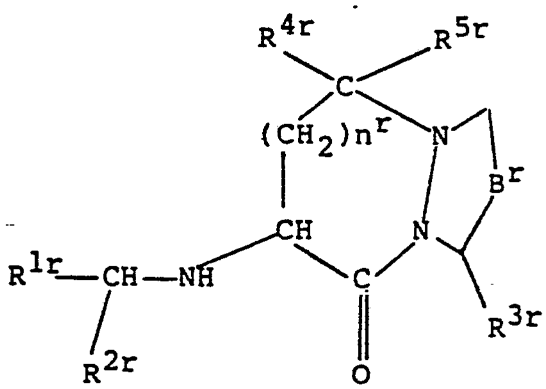

wherein $B^r$ represents a methylene ($-CH_2-$), ethylene ($-CH_2-CH_2-$) or vinylene ($-CH=CH-$) group, $R^{1r}$ represents a hydrogen atom or an alkyl, aralkyl, amino-alkyl, monoalkylamino-alkyl, dialkylamino-alkyl, acylamino-alkyl, phthalimido-alkyl, alkoxycarbonylamino-alkyl, aryloxycarbonyl-amino-alkyl, aralkoxycarbonyl-amino-alkyl, alkylaminocarbonylamino-alkyl, arylaminocarbonylamino-alkyl, aralkylaminocarbonylamino-alkyl, alkylsulphonylamino-alkyl or arylsulphonylamino-alkyl group, $R^{2r}$ represents a carboxyl, alkoxycarbonyl or aralkoxycarbonyl group or a group of the formula

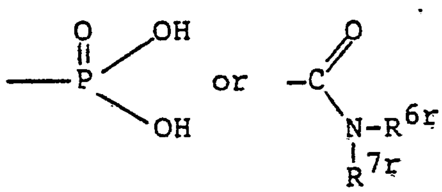

$R^{3r}$ represents a carboxyl, alkoxycarbonyl or aralkoxycarbonyl group, $R^{4r}$ and $R^{5r}$ each represent a hydrogen atom or $R^{4r}$ and $R^{5r}$ together represent an oxo group, $R^{6r}$ and $R^{7r}$ each represent a hydrogen atom or an alkyl or aralkyl group or $R^{6r}$ and $R^{7r}$ together with the nitrogen atom to which they are attached represent a saturated 5 membered or 6-membered heteromonocyclic ring which may contain a further nitrogen atom or an oxygen or sulphur atom, and $n^r$ stands for zero, 1 or 2, and pharmaceutically acceptable salts thereof;

(s) having the formula

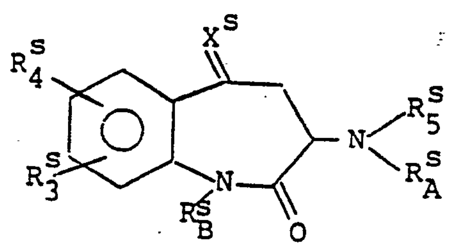

wherein

$R_A{}^s$ and $R_B{}^s$ are radicals of the formula

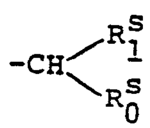

and

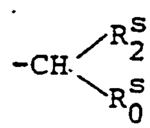

respectively in which

$R_0{}^s$ is carboxy or a functionally modified carboxy;

$R_1{}^s$ is hydrogen, lower alkyl, amino(lower) alkyl, aryl, aryl (lower) alkyl, cycloalkyl or cycloalkyl (lower) alkyl;

$R_2{}^s$ is hydrogen or lower alkyl;

$R_3{}^s$ and $R_4{}^s$, each independently, represent hydrogen, lower alkyl, lower alkoxy, lower alkanoyloxy, hydroxy, halogen, trifluoromethyl, or

$R_3{}^s$ and $R_4{}^s$ taken together represent lower alkylenedioxy;

$R_5{}^s$ is hydrogen or lower alkyl, and

$X^s$ represents oxo, two hydrogens, or one hydroxy together with one hydrogen; and wherein the carbocyclic ring may also be hexahydro or 6, 7, 8, 9-tetrahydro, and salts and complexes thereof;

(t) having the formula

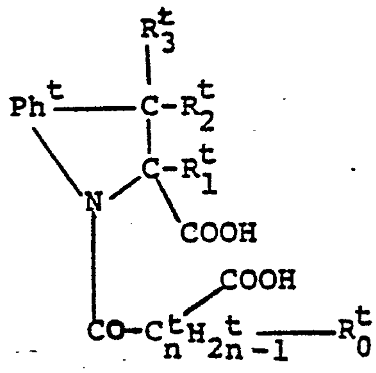

wherein $Ph^t$ is unsubstitued 1,2-phenylene, or 1,2-phenylene substituted by one to three identical or different members selected from lower alkyl, lower alkoxy, lower alkylenedioxy, hydroxy, halogeno and trifluoromethyl; $R_0{}^t$ is hydrogen or $HPh^t$; each of $R_1{}^t$, $R_2{}^t$ and $R_3{}^t$ is hydrogen or lower alkyl; and $n^t$ is an integer from 1 to 10; the amides, mono- or di-lower alkylamides, lower alkyl esters, (amino, mono- or di-lower alkylamino, carboxy or lower carbalkoxy)-lower alkyl esters, or pharmaceutically acceptable salts thereof.

6. A composition according to any one of claims 1,4 or 6 wherein the ACE inhibitor is chosen from spirapril, enalapril, ramipril, perindopril, indolapril, lysinopril, CI-925, pentopril, cilazapril, captopril, zofenopril or pivalopril.

7. A composition according to claim 1 or 3 wherein the atrial peptide is chosen from α human AP 21, α human AP 28, α human AP 23, α human AP 24, α human AP 25, α human AP 26, α human AP 33, and the corresponding atrial peptides wherein the methionine at position 12 is replaced by isoleucine.

8. A kit according to claim 1 comprising in separate containers in a single package pharmaceutical compositions for use in combination to treat hypertension in mammals which comprises in one container a pharmaceutical composition comprising a NMEP inhibitor and in a second container, a pharmaceutical composition comprising an atrial peptide as defined in claims 3 or 7.

9. A kit according to claim 1 comprising in separate containers in a single package pharmaceutical compositions for use in combination to treat hypertension in mammals which comprises in one container a pharmaceutical composition comprising a NMEP inhibitor and in a second container, a pharmaceutical composition comprising an ACE inhibitor as defined in claims 4, 5 or 6.

10. A composition according to any one of claims 1-9 wherein the NMEP inhibitor is chosen from

N-[N-[L-1-(2,2-dimethyl-1-oxopropoxy)methoxy]-carbonyl]-2-phenylethyl]-L-phenylalanyl]-β-alanine;

N-[N-[(L-1-carboxy-2-phenylethyl)]-L-phenylalanyl]-β alanine;

N-[1-oxo-3-phenyl-2-(phosphonomethyl)propyl]-β-alanine;

N-[1-oxo-3-phenyl-2-(phosphonomethyl)propyl]-β-alanine ethyl ester;

N-[N-[1-(S)-carboxyl-3-phenylpropyl]-(S)-phenylalanyl]-(R)-isoserine;

N-[N-[1-(S)-[2-(phenoxy)ethoxycarbonyl]-3-phenylpropyl]-(S)-phenylalanyl]-(S)-isoserine; and

N-[2-(S-acetylthiomethyl)-3-phenylpropionyl]-(S)-methionine amide.

11. A composition according to any one of claims 1-10 wherein the NMEP inhibitor is administered at a dosage level of 1 to 100 mg/kg mammalian weight per day.

12. A composition according to any one of claims 1, 3, 7, 8 or 10 wherein the NMEP inhibitor is administered at a dosage level of 1 to 100 mg/kg mammalian weight per day and the atrial peptide is administered at a dosage level of 0.001 to 0.1 mg/kg mammalian weight per day.

13. A composition according to any one of claims 1, 4, 5, 6, 9 or 10 wherein the NMEP inhibitor is administered at a dosage level of 1 to 100 mg/kg mammalian weight per day and the ACE inhibitor is administered at a dosage level of 0.1 to 30 mg/kg mammalian weight per day.

14. A process for the preparation of a pharmaceutical composition of claim 1 which comprises mixing a NMEP inhibitor, alone or in combination with an atrial peptide or an ACE inhibitor, with a pharmaceutically acceptable carrier.

15. A process of claim 14 wherein the NMEP inhibitor is as defined in claim 10, the atrial peptide is as defined in claim 7 and the ACE inhibitor is as defined in claims 5 or 6.

16. A composition according to claims 1, 3-9, 12 or 13 wherein the NMEP inhibitor is a compound having the formula

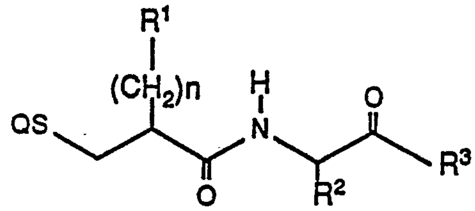

wherein $R^1$ is $Y-C_6H_4-$, $Y-C_6H_4S-$, $Y-C_6H_4O-$,

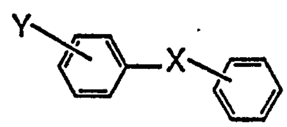

α-naphthyl, β-naphthyl, furyl, thienyl, benzofuryl, benzothienyl, $H_2N(CH_{2m}-$, diphenylmethyl,

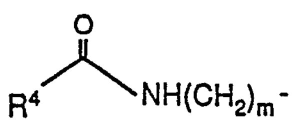

or

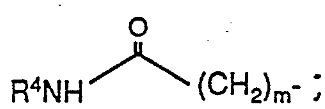

$R^2$ is alkyl, alkyl-$S(O)_{0-2}(CH_2)_q$-, $R^5(CH_2)_kS(O)_{0-2}(CH_2)_q$-, alkyl-$O(CH_2)_q$-, $R^5(CH_2)_k$-$O(CH_2)_q$-, $R^5(CH_2)_q$-, $H_2N(CH_2)_q$-, cycloalkyl$(CH_2)_k$-, $R^{13}CONH(CH_2)_q$-, $R^{13}NHCO(CH_2)_q$- or $R^6OCO(CH_2)_q$-;

$R^3$ is $-OR^7$, 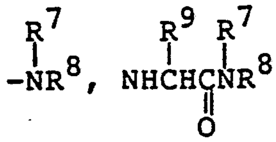 or 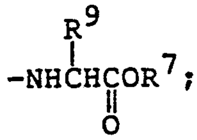;

$R^4$ and $R^{13}$ are independently hydrogen, alkyl or $Y^1-C_6H_4-$;

$R^5$ is $Y^2-C_6H_4-$, $Y^2-C_6H_4S-$, $Y^2-C_6H_4O-$, α-naphthyl, β-naphthyl, furyl, thienyl, benzofuryl, benzothienyl, indolyl or 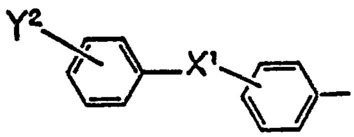,

provided that when $R^5$ is $Y^2-C_6H_4S-$ or $Y^2-C_6H_4O-$, k is 2 or 3;

$R^6$, $R^7$ and $R^8$ are independently H, alkyl, hydroxyalkyl, alkoxyalkyl, aminoalkyl, alkylaminoalkyl, dialkylaminoalkyl or arylalkyl, or $R^7$ and $R^8$ together with the nitrogen to which they are attached complete a 5-7 membered ring, wherein one of the 4-6 ring members comprising $R^7$ and $R^8$ may be a nitrogen atom, an alkyl-substituted nitrogen atom or an oxygen atom, and wherein the ring may be substituted on the ring carbon atoms with substituents chosen from alkyl and hydroxy groups;

$R^9$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

n is 0-2;

m and k are independently 0-3;

q is 1-4;

X and $X^1$ are independently a bond, -O-, -S-, or $-CH_2-$;

Q is hydrogen or $R^{10}CO-$;

$R^{10}$ is alkyl, hydroxyalkyl, alkoxyalkyl, dialkylaminoalkyl, $Y^3$-$C_6H_4$-alkyl, alkoxy, $Y^3$-$C_6H_4$-, naphthyl, furyl, thienyl or pyridyl;

Y, $Y^1$, $Y^2$ and $Y^3$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;

and the pharmaceutically acceptable acid addition salts thereof.

17. A compound having the structural formula

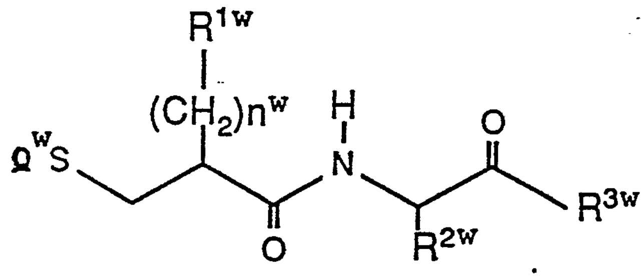

I

wherein $R^{1w}$ is phenyl substituted by one or more substituents independently selected from alkyl, alkoxy, cycloalkyl, cyano and aminomethyl, $Y^w-C_6H_4S-$, $Y^w-C_6H_4O-$,

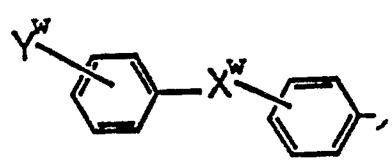

α-naphthyl, β-naphthyl, furyl, benzofuryl, benzothienyl, $H_2N(CH_2)_m{}^w-$, diphenylmethyl,

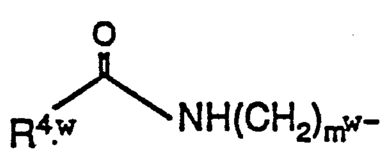

or

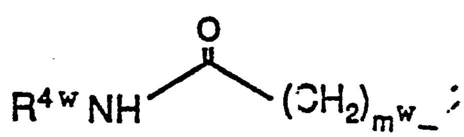

$R^{2w}$ is alkyl, alkyl-$S(O)_{0-2}(CH_2)_q{}^w-$, $R^{5w}(CH_2)_k{}^wS(O)_{0-2}(CH_2)_q{}^w-$, alkyl-$O(CH_2)_q{}^w-$, $R^{5w}(CH_2)_k{}^w-O(CH_2)_q{}^w-$, $R^{5w}(CH_2)_q{}^w-$, $H_2N(CH_2)_q{}^w-$, cycloalkyl$(CH_2)_k{}^w-$, $R^{13w}CONH(CH_2)_q{}^w-$, $R^{13w}NHCO(CH_2)_q{}^w-$ or $R^{6w}OCO(CH_2)_q{}^w-$;

$R^{3w}$ is $-OR^{7w}$, $-NR^{7w}R^{8w}$, 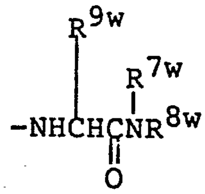 or 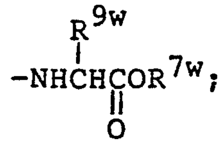;

$R^{4w}$ and $R^{13w}$ are independently hydrogen, alkyl or $Y^{1w}$-$C_6H_4$-;

$R^{5w}$ is $Y^{2w}$-$C_6H_4$-, $Y^{2w}$-$C_6H_4S$-, $Y^{2w}$-$C_6H_4O$-, α-naphthyl, β-naphthyl, furyl, thienyl, benzofuryl, benzothienyl, indolyl or

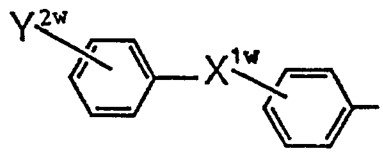

,

provided that when $R^{5w}$ is $Y^{2w}$-$C_6H_4S$- or $Y^{2w}$-$C_6H_4O$-, $k^w$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl;

$n^w$ is 0-2;

$m^w$ and $k^w$ are independently 0-3;

$q^w$ is 1-4;

$X^w$ and $X^{1w}$ are independently a bond, -O-, -S-, or $-CH_2-$;

$Q^w$ is hydrogen or $R^{10w}CO$-;

$R^{10w}$ is alkyl, hydroxyalkyl, alkoxyalkyl, dialkylaminoalkyl, $Y^{3w}$-$C_6H_4$-alkyl, alkoxy, $Y^{3w}$-$C_6H_4$-, naphthyl, furyl, thienyl or pyridyl;

$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;

and the pharmaceutically acceptable addition salts thereof.

18. A compound having the structural formula

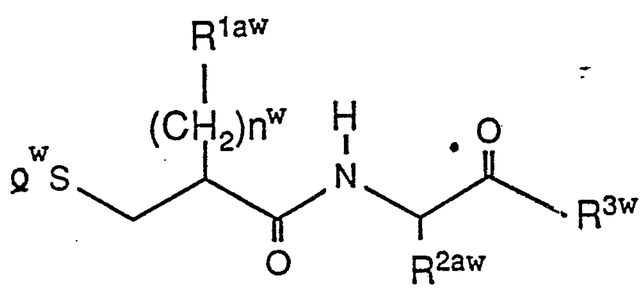

II

wherein $R^{1aw}$ is $Y^w$-$C_6H_4$-, $Y^w$-$C_6H_4S$-, $Y^w$-$C_6H_4O$-,

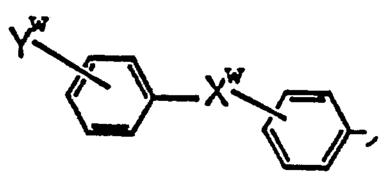

α-naphthyl, β-naphthyl, furyl, thienyl, benzofuryl, benzothienyl, $H_2N(CH_2)_m{}^w$-, diphenylmethyl,

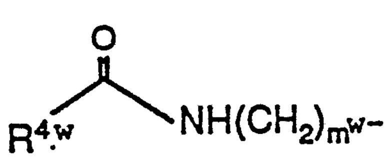

or

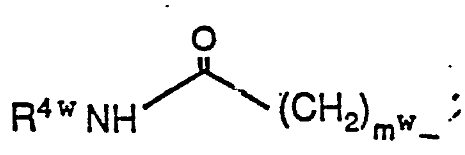

$R^{2aw}$ is $R^{5aw}(CH_2)_k{}^wS(O)_{0-2}(CH_2)_q{}^w$-, $R^{5aw}(CH_2)_k{}^w$-$O(CH_2)_q{}^w$-, $R^{5aw}(CH_2)_q{}^w$-, or cycloalkyl-$(CH_2)_k{}^w$, and when $R^{3w}$ is

$-NR^{7w}R^{8w}$,

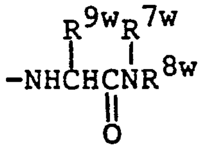 or 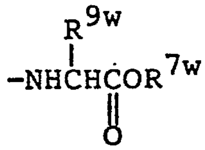, $R^{2aw}$ may also be indolyl-$(CH_2)_q{}^{w}$-,

$R^{13w}CONH(CH_2)_q{}^{w}$-, $R^{13w}NHCO(CH_2)_q{}^{w}$- or $R^{6w}OCO(CH_2)_q{}^{w}$-;

$R^{3w}$ is $-OR^{7w}$, $-NR^{7w}R^{8w}$, 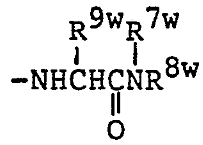 or 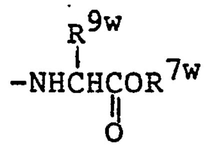;

$R^{4w}$ and $R^{13w}$ are independently hydrogen, alkyl or $Y^{1w}$-$C_6H_4$-;

$R^{5aw}$ is $Y^{2w}$-$C_6H_4$- provided $Y^{2w}$ is not H or OH, $Y^{2w}$-$C_6H_4S$-, $Y^{2w}$-$C_6H_4O$-, α-naphthyl, β-naphthyl, furyl, thienyl, benzofuryl, benzothienyl or

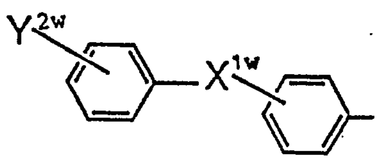 ,

provided that when $R^{5aw}$ is $Y^{2w}$-$C_6H_4S$- or $Y^{2w}C_6H_4O$-, $k^{w}$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and whereim the ring may be substituted on the ring carbon atoms with substituents chosen from alkyl and hydroxy groups;

$R^{9w}$ is hydrogen, alkyl, carboxyalkyl, mercaptoalkyl,

alkylthioalkyl, aminoalkyl, hydroxyalkyl, phenylalkyl, hydroxyphenylalkyl, guanidinoalkyl, imidazolylalkyl, indolylalkyl, or carbamoylalkyl;

$n^w$ is 0-2;

$m^w$ and $k^w$ are independently 0-3;

$q^w$ is 1-4;

$X^w$ and $X^{1w}$ are independently a bond, -O-, -S-, or $-CH_2-$;

$Q^w$ is hydrogen or $R^{10w}CO-$;

$R^{10w}$ is alkyl, hydroxyalkyl, alkoxyalkyl, dialkylaminoalkyl, $Y^{3w}-C_6H_4-$alkyl, alkoxy, $Y^{3w}-C_6H_4-$, naphthyl, furyl, thienyl or pyridyl;

$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;

and the pharmaceutically acceptable addition salts thereof.

19. A compound represented by the structural formula

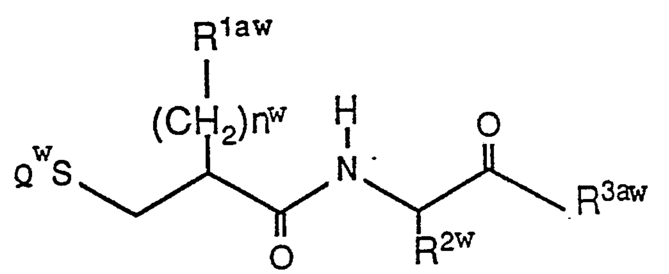

III

wherein $R^{1aw}$ is $Y^w-C_6H_4-$, $Y^w-C_6H_4S-$, $Y^w-C_6H_4O-$,

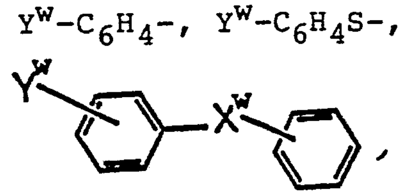,

α-naphthyl, β-naphthyl, furyl, thienyl, benzofuryl, benzothienyl, $H_2N(CH_2)_m{}^w-$, diphenylmethyl,

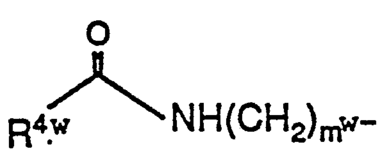

or

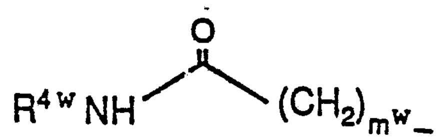;

$R^{2w}$ is alkyl, alkyl-$S(O)_{0-2}(CH_2)_q{}^{w}$-, $R^{5w}(CH_2)_k{}^{w}S(O)_{0-2}(CH_2)_q{}^{w}$-, alkyl-$O(CH_2)_q{}^{w}$-, $R^{5w}(CH_2)_k{}^{w}$-$O(CH_2)_q{}^{w}$-, $R^{5w}(CH_2)_q{}^{w}$-, $H_2N(CH_2)_q{}^{w}$-, cycloalkyl$(CH_2)_k{}^{w}$-, $R^{13w}CONH(CH_2)_q{}^{w}$-, $R^{13w}NHCO(CH_2)_q{}^{w}$- or $R^{6w}OCO(CH_2)_q{}^{w}$-;

$R^{3aw}$ is 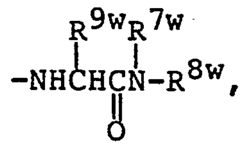, 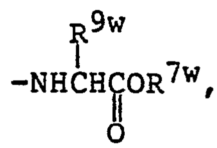, $-OR^{11w}$ or 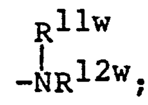;

$R^{4w}$ and $R^{13w}$ are independently hydrogen, alkyl or $Y^{1w}$-$C_6H_4$-;

$R^{5w}$ is $Y^{2w}$-$C_6H_4$-, $Y^{2w}$-$C_6H_4S$-, $Y^{2w}$-$C_6H_4O$-, α-naphthyl, β-naphthyl, furyl, thienyl, benzofuryl, benzothienyl, indolyl or

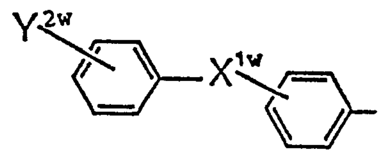,

provided that when $R^{5w}$ is $Y^{2w}$-$C_6H_4S$- or $Y^{2w}$-$C_6H_4O$-, $k^{w}$ is 2 or 3;

$R^{6w}$, $R^{7w}$ and $R^{8w}$ are independently H, alkyl, hydroxyalkyl, alkoxyalkyl, aminoalkyl, alkylaminoalkyl, dialkylaminoalkyl or arylalkyl, or $R^{7w}$ and $R^{8w}$ together with the nitrogen to which they are attached complete a 5-7 membered ring, wherein one of the 4-6 ring members comprising $R^{7w}$ and $R^{8w}$ may be a nitrogen atom, an alkyl-substituted nitrogen atom or an oxygen atom, and wherein the ring may be substituted on the ring carbon atoms with substituents chosen from alkyl and hydroxy groups;

$R^{9w}$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl;

$n^{w}$ is 0-2;

$m^w$ and $k^w$ are independently 0-3;

$q^w$ is 1-4;

$X^w$ and $X^{1w}$ are independently a bond, -O-, -S-, or $-CH_2-$;

$Q^w$ is hydrogen or $R^{10w}CO-$;

$R^{10w}$ is alkyl, hydroxyalkyl, alkoxyalkyl, dialkylaminoalkyl, $Y^{3w}-C_6H_4$-alkyl, alkoxy, $Y^{3w}-C_6H_4-$, naphthyl, furyl, thienyl or pyridyl;

$R^{11w}$ is hydroxyalkyl or substituted phenylalkyl wherein the phenyl group is substituted by one or more groups selected from alkyl, alkoxy, cycloalkyl and cyano; $R^{12w}$ is H or selected from the same group as $R^{11w}$; or $R^{11w}$ and $R^{12w}$ together with the nitrogen to which they are attached complete a 5-7 membered ring wherein one of the 4-6 ring members comprising $R^{11w}$ and $R^{12w}$ may be a nitrogen atom, an alkyl-substituted nitrogen atom or an oxygen atom, and wherein the ring may be substituted on the ring carbon atoms with substituents chosen from alkyl and hydroxy groups;

$Y^w$, $Y^{1w}$, $Y^{2w}$ and $Y^{3w}$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl;

and the pharmaceutically acceptable addition salts thereof.

20. Process for the preparation of a compound of formula I as defined in claim 17, wherein the compound is prepared by an appropriate process selected from the following processes A and B, and wherein $Q^w$, $R^{1w}$, $R^{2w}$, $R^{3w}$ and $n^w$ are as defined in claim 17, including suitable protection:

Process A: condensation of a 3-thiopropionic acid of formula IV, or a reactive derivative thereof, with an amine of formula V

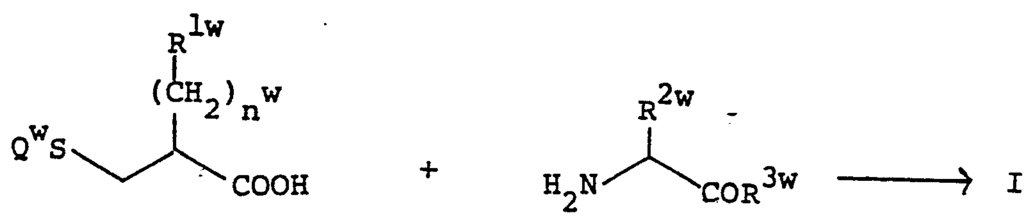

IV V

Process B: for compounds of formula I wherein $R^{3w}$ is $-NR^{7w}R^{8w}$, condensation of a (3-thiopropionyl)amino acid of formula VI, or a reactive derivative thereof, with an amine of formula VII

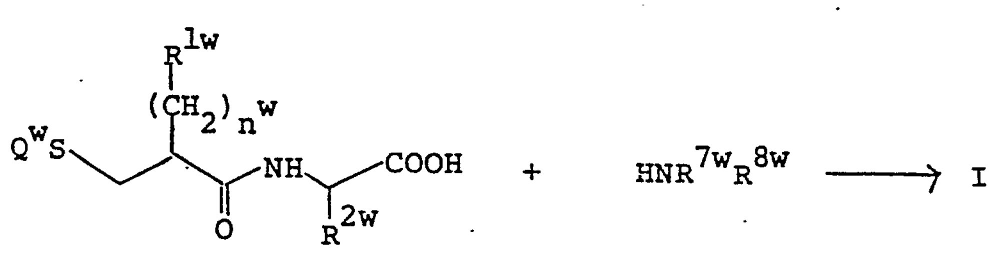

VI VII

wherein both Process A and Process B are followed by isolating

the preferred isomer if desired and removal of the protecting groups, if necessary, to yield the desired product, and if desired, preparing a salt thereof.

21. Process for the preparation of a compound of formula II as defined in claim 18, wherein the compound is prepared by an appropriate process selected from the following processes A and B, and wherein $Q^W$, $R^{1aw}$, $R^{2aw}$, $R^{3w}$ and $n^W$ are as defined in claim 18 including suitable protection:

Process A: condensation of a 3-thiopropionic acid of formula IV, or a reactive derivative thereof, with an amine of formula V

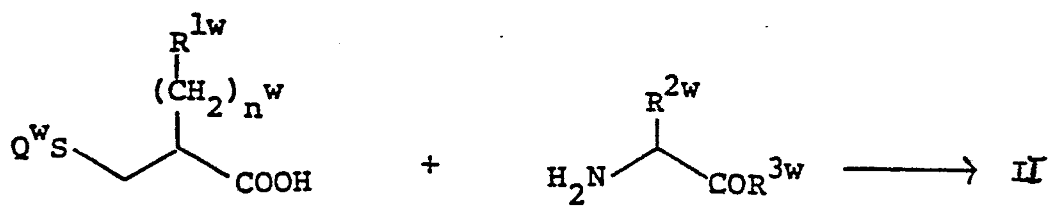

IV V

Process B: for compounds of formula I wherein $R^{3w}$ is $-NR^{7w}R^{8w}$, condensation of a (3-thiopropionyl)amino acid of formula VI, or a reactive derivative thereof, with an amine of formula VII

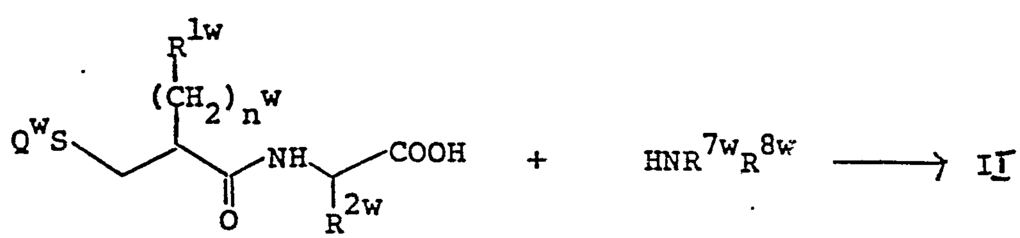

VI VII

wherein both Process A and Process B are followed by isolating

the preferred isomer if desired and removal of the protecting groups, if necessary, to yield the desired product, and if desired, preparing a salt thereof.

22. Process for the preparation of a compound of formula III as defined in claim 19 wherein the compound is prepared by an appropriate process selected from the following processes A and B, and wherein $Q^w$, $R^{1aw}$, $R^{2w}$, $r^{3aw}$ and $n^w$ are as defined in claims 19 including suitable protection:

Process A: condensation of a 3-thiopropionic acid of formula IV, or a reactive derivative thereof, with an amine of formula V

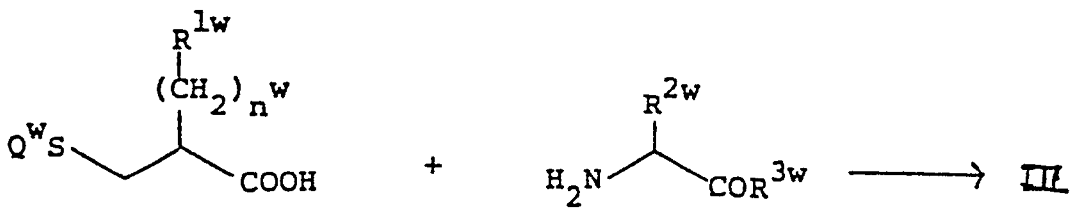

IV V

Process B: for compounds of formula I wherein $R^{3w}$ is $-NR^{7w}R^{8w}$, condensation of a (3-thiopropionyl)amino acid of formula VI, or a reactive derivative thereof, with an amine of formula VII

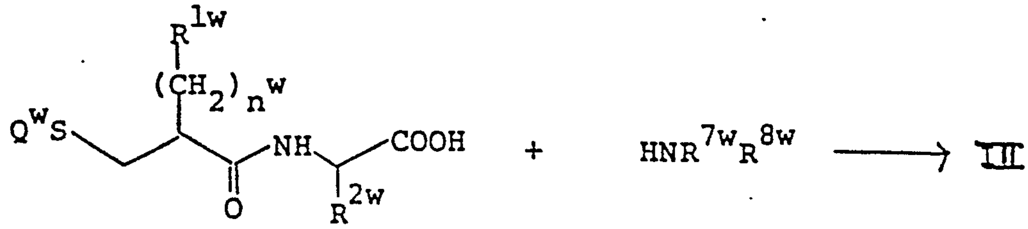

VI VII

wherein both Process A and Process B are followed by isolating

the preferred isomer if desired and removal of the protecting groups, if necessary, to yield the desired product, and if desired, preparing a salt thereof.